# EUROPÄISCHE PATENTSCHRIFT

(11) **EP 1 716 118 B1**
(45) Veröffentlichungstag und Bekanntmachung des Hinweises auf die Patenterteilung: **30.04.2008**
(21) Anmeldenummer: 05701287.4
(22) Anmeldetag: 01.02.2005
(51) Int. Cl.: C07D 223/16, C07D 401/06, A61K 31/55, A61P 9/00

(54) **TETRAHYDROBENZO (D) AZEPIN-2-ON DERIVATE UND IHRE VERWENDUNG ZUR BEHANDLUNG VON KARDIOVASKULÄREN KRANKHEITEN**
TETRAHYDROBENZO[D]AZEPIN-2- ONE DERIVATIVES AND THE USE THEREOF FOR TREATING CARDIOVASCULAR DISEASES
DERIVES DE TETRAHYDROBENZO (D) AZEPINE-2-ONE ET LEUR UTILISATION POUR TRAITER DES MALADIES CARDIO-VASCULAIRES

(30) Priorität: 10.02.2004 DE 102004006325
(43) Veröffentlichungstag der Anmeldung: 02.11.2006
(73) Patentinhaber: Bayer HealthCare AG, 51368 Leverkusen (DE)
(72) Erfinder: GRIEBENOW, Nils, 41541 Dormagen (DE); FLESSNER, Timo, 42113 Wuppertal (DE); HÄRTER, Michael, 51375 Leverkusen (DE); RAABE, Martin, 89081 Ulm (DE); BUCHMÜLLER, Anja, 45239 Essen (DE); BISCHOFF, Hilmar, 42113 Wuppertal (DE); ELLINGHAUS, Peter, 42113 Wuppertal (DE); KOLKHOF, Peter, 42113 Wuppertal (DE)
(86) Internationale Anmeldenummer: PCT/EP2005/000960
(87) Internationale Veröffentlichungsnummer: WO 2005/077907

(56) Entgegenhaltungen:
- WO-A-02/057258
- US-A1- 2003 078 251

## Beschreibung

Die vorliegende Anmeldung betrifft neue Tetrahydrobenzo[d]azepin-2-on-Derivate, Verfahren zu ihrer Herstellung, sowie ihre Verwendung zur Herstellung von Arzneimitteln zur Behandlung und/oder Prophylaxe von Krankheiten, vorzugsweise zur Behandlung und/oder Prävention kardiovaskulärer Erkrankungen, insbesondere von Dyslipidämien, Arteriosklerose, Restenose und Ischämien.

Eine Vielzahl epidemiologischer Studien hat einen ursächlichen Zusammenhang zwischen Dyslipidämien und kardiovaskulären Erkrankungen gezeigt. Isoliert erhöhtes Plasma-Cholesterin ist einer der größten Risikofaktoren für kardiovaskuläre Erkrankungen wie beispielsweise Arteriosklerose. Dies betrifft sdwohl eine isolierte Hypercholesterinämie als auch Hypercholesterinämien kombiniert mit z.B. erhöhten Plasma-Triglyceriden oder niedrigem Plasma-HDL-Cholesterin. Substanzen, welche Cholesterin- oder kombiniert Cholesterin- und Triglycerid-senkend wirken, sollten sich daher zur Behandlung und Prävention kardiovaskulärer Erkrankungen eignen.

Es wurde bereits gezeigt, dass Squalen-Synthase-Inhibitoren im Tiermodell Plasma-Cholesterin und -Triglyceride senken. Squalen-Synthase (EC 2.5.1.21) katalysiert die reduktive Kondensation von Farnesylpyrophosphat zu Squalen. Dies ist ein entscheidender Schritt in der Cholesterin-Biosynthese. Während Farnesylpyrophosphat und Vorläufer auch für andere zelluläre Stoffwechselwege und -Reaktionen von Bedeutung sind, dient Squalen ausschließlich als Vorläufer für Cholesterin. Eine Hemmung der Squalen-Synthase führt somit direkt zur Reduktion der Cholesterin-Biosynthese und damit zur Absenkung der Plasma-Cholesterin-Spiegel. Zusätzlich wurde gezeigt, dass Squalen-Synthase-Inhibitoren auch Plasma-Triglycerid-Spiegel reduzieren. Inhibitoren der Squalen-Synthase könnten somit zur Behandlung und/oder Prävention kardiovaskulärer Erkrankungen, wie beispielsweise Dyslipidämien, Arteriosklerose, Ischämie/Reperfusion, Restenose und arterielle Entzündungen, eingesetzt werden [vgl. z.B. Eur. Heart J. 19 (Suppl. A), A2-A11 (1998); Prog. Med Chem. 33, 331-378 (1996); Europ.J. Pharm. 431, 345-352 (2001)].

Aufgabe der vorliegenden Erfindung war die Bereitstellung neuer Verbindungen, die als Squalen-Synthase-Inhibitoren zur Behandlung und/oder Prävention insbesondere kardiovaskulärer Erkrankungen eingesetzt werden können.

In WO 02/057258 werden Tetrahydrobenzo[d]azepin-2-on-Derivate als Farnesyltransferase-Inhibitoren zur Behandlung von- Krebserkrankungen, Restenose und Neurofibromatose beschrieben.

In US 2003/078 251 - werden Benzoxazepinone als Squalen Synthase Inhibitoren offenbart.

Gegenstand der vorliegenden Erfindung sind Verbindungen der allgemeinen Formel (I) 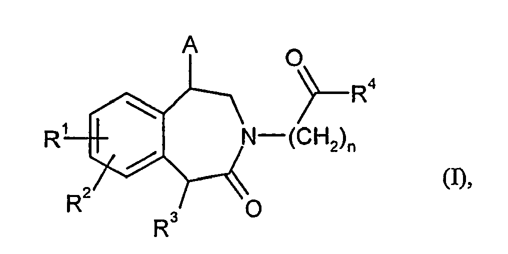 in welcher
- A: für (C₆-C₁₀)-Aryl oder 5- bis 10-gliedriges Heteroaryl, welche jeweils bis zu dreifach, gleich oder verschieden, durch Substituenten ausgewählt aus der Gruppe Halogen, Cyano, Nitro, Trifluormethyl, Trifluormethoxy, (C₁-C₆)-Alkyl, (C₂-C₆)-Alkinyl und (C₁-C₆)-Alkoxy substituiert sein können,
oder
für eine Gruppe der Formel 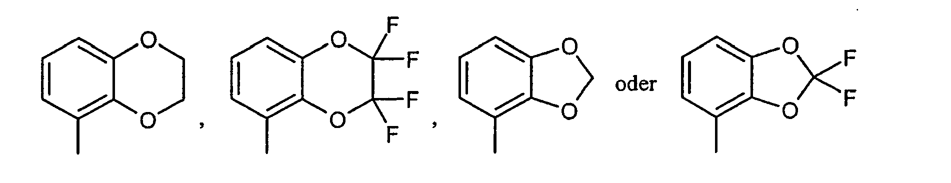 steht,
- n: für die Zahl 1, 2 oder 3 steht,
- R¹ und R²: gleich oder verschieden sind und unabhängig voneinander für Wasserstoff, Halogen, Cyano, Nitro, Trifluormethyl, Trifluormethoxy, (C₁-C₆)-Alkyl oder (C₁-C₆)-Alkoxy stehen,
- R³: für (C₁-C₈)-Alkyl, (C₂-C₈)-Alkenyl oder (C₂-C₈)-Alkinyl, welche jeweils durch Phenyl, (C₃-C₈)-Cycloalkyl, Hydroxy, (C₁-C₆)-Alkoxy, (C₁-C₆)-Acyloxy oder Amino substituiert sein können, steht,
und
- R⁴: für eine Gruppe der Formel -OR⁷ oder NR⁸R⁹ steht, worin
R⁷ Wasserstoff oder (C₁-C₆)-Alkyl bedeutet,
R⁸ und R⁹ gleich oder verschieden sind und unabhängig voneinander Wasserstoff, (C₁-C₆)-Alkyl oder (C₃-C₈)-Cycloalkyl, die durch Substituenten ausgewählt aus der Gruppe Carboxyl, (C₁-C₆)-Alkoxycarbonyl, Aminocarbonyl, Mono- und Di-(C₁-C₆)-alkylaminocarbonyl substituiert sein können, bedeuten
oder
R⁸ und R⁹ gemeinsam mit dem Stickstoffatom, an das sie gebunden sind, einen 4- bis 8-gliedrigen Heterocyclus, der ein weiteres Ring-Heteroatom aus der Reihe N-R¹⁰, O, S, SO oder SO₂ enthalten und durch Substituenten ausgewählt aus der Gruppe Hydroxy, Oxo, Amino, (C₁-C₁₅)-Alkyl, Carboxyl, (C₁-C₆)-Alkoxycarbonyl, Aminocarbonyl, Mono- und Di-(C₁-C₆)-alkylaminocarbonyl substituiert sein kann, bilden, worin
(C₁-C₆)-Alkyl seinerseits durch Substituenten ausgewählt aus der Gruppe Hydroxy, Amino, Carboxyl, (C₁-C₆)-Alkoxycarbonyl, Aminocarbonyl, Mono- und Di-(C₁-C₆)-alkylaminocarbonyl substituiert sein kann
und
R¹⁰ Wasserstoff, (C₁-C₄)-Alkyl, (C₁-C₄)-Acyl oder (C₁-C₄)-Alkoxycarbonyl bedeutet,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfasst Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:

(C₁ -C₈)-Alkyl, (C₁ -C₆)-Alkyl und (C₁-C₄)-Alkyl stehen im Rahmen der Erfindung für einen geradkettigen oder verzweigten Alkylrest mit 1 bis 8, 1 bis 6 bzw. 1 bis 4 Kohlenstoffatomen. Bevorzugt ist ein geradkettiger oder verzweigter Alkylrest mit 1 bis 6 bzw. 1 bis 4 Kohlenstoffatomen. Besonders bevorzugt ist ein geradkettiger oder verzweigter Alkylrest mit 1 bis 4 Kohlenstoffatomen. Beispielhaft und vorzugsweise seien genannt: Methyl, Ethyl, n-Propyl, Isopropyl, n-Butyl, iso-Butyl, sec.-Butyl, tert.-Butyl, 1-Ethylpropyl, n-Pentyl und n-Hexyl.

(C₁-C₈)-Alkenyl und (C₂-C₆)-Alkenyl stehen im Rahmen der Erfindung für einen geradkettigen oder verzweigten Alkenylrest mit 2 bis 8 bzw. 2 bis 6 Kohlenstoffatomen. Bevorzugt ist ein geradkettiger oder verzweigter Alkenylrest mit 2 bis 6, besonders bevorzugt mit 2 bis 4 Kohlenstoffatomen. Beispielhaft und vorzugsweise seien genannt: Vinyl, Allyl, Isopropenyl, n-But-2-en-1-yl und 2-Methyl-2-propen-1-yl.

(C₁-C₈)-Alkinyl steht im Rahmen der Erfindung für einen geradkettigen oder verzweigten Alkinylrest mit 2 bis 8 Kohlenstoffatomen. Bevorzugt ist ein geradkettiger oder verzweigter Alkinylrest mit 2 bis 6, besonders bevorzugt mit 2 bis 4 Kohlenstoffatomen. Beispielhaft und vorzugsweise seien genannt: Ethinyl, n-Prop-2-in-1-yl und n-But-2-in-1-yl.

(C₃-C₈)-Cycloalkyl und (C₃-C₆)-Cycloalkyl stehen im Rahmen der Erfindung für eine monocyclische Cycloalkylgruppe mit 3 bis 8 bzw. 3 bis 6 Kohlenstoffatomen. Bevorzugt ist ein Cycloalkylrest mit 3 bis 6 Kohlenstoffatomen. Beispielhaft und vorzugsweise seien genannt: Cyclopropyl, Cyclobutyl, Cyclopentyl, Cyclohexyl und Cycloheptyl.

(C₆-C₁₀)-Aryl steht im Rahmen der Erfindung für einen aromatischen Rest mit vorzugsweise 6 bis 10 Kohlenstoffatomen. Bevorzugte Arylreste sind Phenyl und Naphthyl.

(C₁-C₆)-Alkoxy und (C₁-C₄ -Alkoxy stehen im Rahmen der Erfindung für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4 Kohlenstoffatomen. Beispielhaft und vorzugsweise seien genannt: Methoxy, Ethoxy, n-Propoxy, Isopropoxy und tert.-Butoxy.

(C₁-C₆)-Alkoxycarbonyl und (C₁-C₄)-Alkoxycarbonyl stehen im Rahmen der Erfindung für einen geradkettigen oder verzweigten Alkoxyrest mit 1 bis 6 bzw. 1 bis 4 Kohlenstoffatomen, der über eine Carbonylgruppe verknüpft ist. Bevorzugt ist ein geradkettiger oder verzweigter Alkoxycarbonylrest mit 1 bis 4 Kohlenstoffatomen in der Alkoxy-Gruppe. Beispielhaft und vorzugsweise seien genannt: Methoxycarbonyl, Ethoxycarbonyl, n-Propoxycarbonyl, Isopropoxycarbonyl und tert.-Butoxycarbonyl.

Mono- oder Di-(C₁-C₆)-alkylaminocarbonyl bzw. Mono- oder Di-(C₁-C₄)-alkylaminocarbonyl stehen im Rahmen der Erfindung für eine Amino-Gruppe, die über eine Carbonylgruppe verknüpft ist und die einen geradkettigen oder verzweigten bzw. zwei gleiche oder verschiedene geradkettige oder verzweigte Alkylsubstituenten mit jeweils 1 bis 6 bzw. 1 bis 4 Kohlenstoffatomen aufweist. Beispielhaft und vorzugsweise seien genannt: Methylaminocarbonyl, Ethylaminocarbonyl, Isopropylaminocarbonyl, tert-Butylaminocarbonyl, *N,N*-Dimethylaminocarbonyl, *N,N*-Diethylaminocarbonyl, *N*-Ethyl-*N*-methylaminocarbonyl und *N*-tert.-Butyl-*N*-methylaminocarbonyl.

(C₁₋C₄)-Acyl [(C₁-C₄)-Alkanoyl] steht im Rahmen der Erfindung für einen geradkettigen oder verzweigten Alkylrest mit 1 bis 4 Kohlenstoffatomen, der in der 1-Position ein doppelt gebundenes Sauerstoffatom trägt und über die 1-Position verknüpft ist. Beispielhaft und vorzugsweise seien genannt: Formyl, Acetyl, Propionyl, n-Butyryl und iso-Butyryl.

(C₁-C₆)-Acyloxy steht im Rahmen der Erfindung für einen geradkettigen oder verzweigten AlkylRest mit 1 bis 6 Kohlenstoffatomen, der in der 1-Position ein doppelt gebundenes Sauerstoffatom trägt und in der 1-Position über ein weiteres Sauerstoffatom verknüpft ist. Bevorzugt ist ein Acyloxy-Rest mit 1 bis 4 Kohlenstoffatomen. Beispielhaft und vorzugsweise seien genannt: Acetoxy, Propionoxy, n-Butyroxy, i-Butyroxy, Pivaloyloxy und n-Hexanoyloxy.

5- bis 10-gliedriges Heteroaryl steht im Rahmen der Erfindung für einen mono- oder gegebenenfalls bicyclischen aromatischen Heterocyclus (Heteroaromaten) mit bis zu drei gleichen oder verschiedenen Heteroatomen aus der Reihe N, O und/oder S, der über ein Ringkohlenstoffatom oder gegebenenfalls über ein Ringstickstoffatom des Heteroaromaten verknüpft ist. Beispielhaft seien genannt: Furanyl, Pyrrolyl, Thienyl, Pyrazolyl, Imidazolyl, Thiazolyl, Oxazolyl, Isoxazolyl, Isothiazolyl, Pyridyl, Pyrimidinyl, Pyridazinyl, Pyrazinyl, Benzofuranyl, Benzothienyl, Benzimidazolyl, Benzoxazolyl, Indolyl, Indazolyl, Chinolinyl, Isochinolinyl, Naphthyridinyl, Chinazolinyl, Chinoxalinyl. Bevorzugt sind 5- bis 6-gliedrige Heteroaryl-Reste mit bis zu zwei Heteroatomen aus der Reihe N, O und/oder S wie beispielsweise Furyl, Thienyl, Thiazolyl, Oxazolyl, Isothiazolyl, Isoxazolyl, Imidazolyl, Pyridyl, Pyrimidinyl, Pyridazinyl, Pyrazinyl.

Ein 4- bis 8-, 5- bis 7- bzw. 5- bis 6-gliedriger Heterocyclus steht im Rahmen der Erfindung für einen gesättigten oder partiell ungesättigten Heterocyclus mit 4 bis 8, 5 bis 7 bzw. 5 bis 6 Ringatomen, der ein Ring-Stickstoffatom enthält, über dieses verknüpft ist und ein weiteres Heteroatom aus der Reihe N, O, S, SO oder SO₂ enthalten kann. Bevorzugt ist ein 5- bis 7-gliedriger gesättigter, N-verknüpfter Heterocyclus, der ein weiteres Heteroatom aus der Reihe N, O oder S enthalten kann. Beispielhaft seien genannt: Pyrrolidinyl, Pyrrolinyl, Piperidinyl, Piperazinyl, Morpholinyl, Thiomorpholinyl, Azepinyl, 1,4-Diazepinyl. Besonders bevorzugt sind Piperidinyl, Piperazinyl, Morpholinyl und Pyrrolidinyl.

Halogen schließt im Rahmen der Erfindung Fluor, Chlor, Brom und Iod ein. Bevorzugt sind Chlor oder Fluor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Bevorzugt sind Verbindungen der Formel (I), in welcher
- A: für Phenyl, Naphthyl oder Pyridyl, welche jeweils bis zu zweifach, gleich oder verschieden, durch Substituenten ausgewählt aus der Gruppe Fluor, Chlor, Brom, Cyano, Trifluormethyl, Trifluormethoxy, (C₁-C₄)-Alkyl, (C₂-C₄)-Alkinyl und (C₁-C₄)-Alkoxy substituiert sein können,
oder
für eine Gruppe der Formel 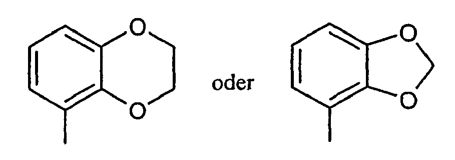 steht,
- n: für die Zahl 1, 2 oder 3 steht,
- R¹: für Wasserstoff, Fluor, Chlor, Cyano, Trifluormethyl, Trifluormethoxy, (C₁-C₄)-Alkyl oder (C₁-C₄)-Alkoxy steht,
- R²: für Wasserstoff steht,
- R³: für (C₁-C₆)-Alkyl oder (C₂-C₆)-Alkenyl, welche jeweils durch Phenyl, (C₃-C₆)-CycJoalkyl oder Hydroxy substituiert sein können, steht,
und
- R⁴: für eine Gruppe der Formel -OR⁷ oder NRsR9 steht, worin
R⁷ Wasserstoff bedeutet,
R⁸ und R⁹ gleich oder verschieden sind und unabhängig voneinander Wasserstoff, (C₁-C₆)-Alkyl oder (C₃-C₆)-Cycloalkyl, die durch Substituenten ausgewählt aus der Gruppe Carboxyl, (C₁-C₄)-Alkoxycarbonyl, Aminocarbonyl, Mono- und Di-(C₁-C₄)-alkylaminocarbonyl substituiert sein können, bedeuten
oder
R⁸ und R⁹ gemeinsam mit dem Stickstoffatom, an das sie gebunden sind, einen 5- bis 7-gliedrigen Heterocyclus, der ein weiteres Ring-Heteroatom aus der Reihe N-R¹⁰ und O enthalten und durch Substituenten ausgewählt aus der Gruppe Hydroxy, Oxo, Amino, (C₁-C₄)-Alkyl, Carboxyl; (C₁-C₄)-Alkoxycarbonyl, Aminocarbonyl, Mono- und Di-(C₁-C₄)-alkylaminocarbonyl substituiert sein kann, bilden, worin
(C₁-C₄)-Alkyl seinerseits durch Substituenten ausgewählt aus der Gruppe Hydroxy, Amino, Carboxyl, (C₁-C₄)-Alkoxycarbonyl, Aminocarbonyl, Mono- und Di-(C₁-C₄)-alkylaminocarbonyl substituiert sein kann
und
R¹⁰ Wasserstoff, (C₁-C₄)-Alkyl, (C₁-C₄)-Acyl oder (C₁-C₄)-Alkoxycarbonyl bedeutet,
sowie ihre Salze, Solvate und Solvate der Salze.

Besonders bevorzugt sind Verbindungen der Formel (I), in welcher
- A: für Phenyl, welches ein- oder zweifach, gleich oder verschieden, durch Fluor, Chlor, Brom, Methyl, Ethyl, Ethinyl oder Methoxy substituiert sein kann, für Naphthyl oder für eine Gruppe der Formel 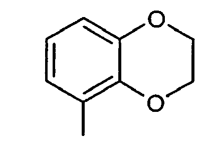 steht,
- n: für die Zahl 1 steht,
- R¹: für Wasserstoff, Chlor, Methyl oder Trifluormethyl steht,
- R²: für Wasserstoff steht,
- R³: für (C₁-C₆)-Alkyl, (C₂-C₆)-Alkenyl oder für Benzyl steht,
und
- R⁴: für eine Gruppe der Formel -OR⁷ oder -NR⁸R⁹ steht, worin
R⁷ Wasserstoff bedeutet,
R⁸ und R⁹ gleich oder verschieden sind und unabhängig voneinander Wasserstoff oder (C₁-C₆)-Alkyl, welches durch Carboxyl oder (C₁-C₄)-Alkoxycarbonyl substituiert sein kann, bedeuten
oder
R⁸ und R⁹ gemeinsam mit dem Stickstoffatom, an das sie gebunden sind, einen 5- bis 6-gliedrigen Heterocyclus, der ein weiteres Ring-Heteroatom aus der Reihe N-R¹⁰ und O enthalten und durch Substituenten ausgewählt aus der Gruppe Hydroxy, Oxo, Amino, (C₁-C₄)-Alkyl, Carboxyl, (C₁-C₄)-Alkoxycarbonyl, Aminocarbonyl, Mono- und Di-(C₁-C₄)-alkylaminocarbonyl substituiert sein kann, bilden, worin
(C₁-C₄)-Alkyl seinerseits durch Substituenten ausgewählt aus der Gruppe Hydroxy, Amino, Carboxyl, (C₁-C₄)-Alkoxycarbonyl, Aminocarbonyl, Mono- und Di-(C₁-C₄)-alkylaminocarbonyl substituiert sein kann
und
R¹⁰ Wasserstoff, (C₁-C₄)-Alkyl oder (C₁-C₄)-Acyl bedeutet,
sowie ihre Salze, Solvate und Solvate der Salze.

Ganz besonders bevorzugt sind Verbindungen der Formel (I-A) 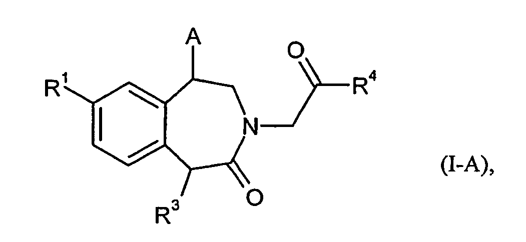 in welcher
- A: für Phenyl, welches ein- oder zweifach, gleich oder verschieden, durch Fluor, Chlor, Brom, Methyl, Ethinyl oder Methoxy substituiert ist, oder für eine Gruppe der Formel 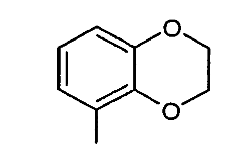 steht,
- R¹: für Chlor, Methyl oder Trifluormethyl steht,
- R³: für (C₁-C₆)-Alkyl oder (C₂-C₆)-Alkenyl steht,
und
- R⁴: für eine Gruppe der Formel -OR⁷ oder -NR⁸R⁹ steht, worin
R⁷ Wasserstoff bedeutet,
R⁸ und R⁹ gleich oder verschieden sind und unabhängig voneinander Wasserstoff oder (C₁-C₆)-Alkyl, welches durch Carboxyl oder (C₁-C₄)-Alkoxycarbonyl substituiert sein kann, bedeuten
oder
R⁸ und R⁹ gemeinsam mit dem Stickstoffatom, an das sie gebunden sind, einen 5- bis 6-gliedrigen Heterocyclus, der ein weiteres Ring-Heteroatom aus der Reihe N-R¹⁰ und O enthalten und durch Substituenten ausgewählt aus der Gruppe Hydroxy, Oxo, Amino, (C₁-C₄)-Alkyl, Carboxyl, (C₁-C₄)-Alkoxycarbonyl, Aminocarbonyl, Mono- und Di-(C₁-C₄)-alkylaminocarbonyl substituiert sein kann, bilden, worin
(C₁-C₄)-Alkyl seinerseits durch Substituenten ausgewählt aus der Gruppe Hydroxy, Amino, Carboxyl, (C₁-C₄)-Alkoxycarbonyl, Aminocarbonyl, Mono- und Di-(C₁-C₄)-alkylaminocarbonyl substituiert sein kann
und
R¹⁰ Wasserstoff, (C₁-C₄)-Alkyl oder (C₁-C₄)-Acyl bedeutet,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen, dadurch gekennzeichnet, dass man Verbindungen der Formel (II) 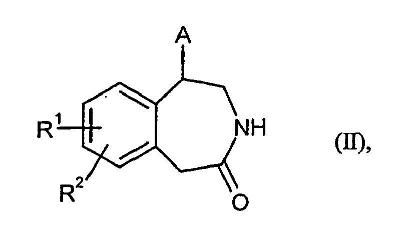 in welcher R¹, R² und A jeweils die oben angegebenen Bedeutungen haben,
zunächst in einem inerten Lösungsmittel in Gegenwart einer Base mit einer Verbindung der Formel (III) 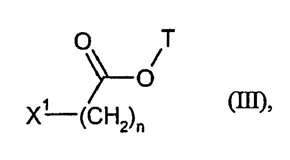 in welcher n die oben angegebenen Bedeutungen hat,
- T: für (C₁-C₄)-Alky] oder Benzyl
und
- X¹: für eine geeignete Fluchtgruppe wie beispielsweise Halogen, Mesylat oder Tosylat steht, zu Verbindungen der Formel (IV) 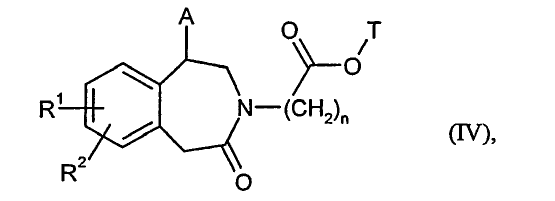 in welcher R¹, R², A, T und n jeweils die oben angegebenen Bedeutungen haben,
umsetzt, anschließend in einem inerten Lösungsmittel in Gegenwart einer geeigneten Base, vorzugsweise einer Phosphazen-Base, mit einer Verbindung der Formel (V)

R³-X² (V),

in welcher R³ die oben angegebenen Bedeutungen hat und
- x²: für eine geeignete Fluchtgruppe wie beispielsweise Halogen, Mesylat oder Tosylat steht, in Verbindungen der Formel (VI) 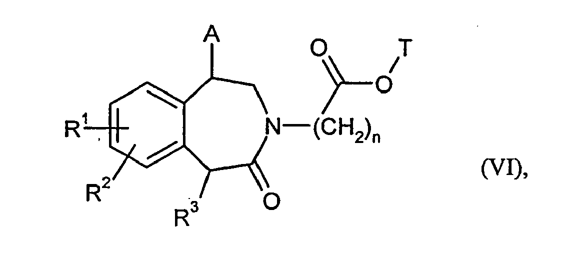 in welcher R¹, R², R³, A, T und n jeweils die oben angegebenen Bedeutungen haben,
überführt, diese durch basische oder saure Hydrolyse oder im Falle, dass T für Benzyl steht, auch hydrogenolytisch zu Carbonsäuren der Formel (VII) 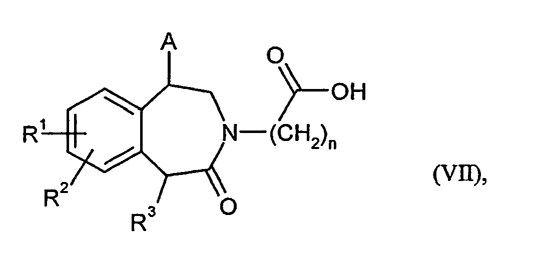 in welcher R¹, R², R³, A und n jeweils die oben angegebenen Bedeutungen haben,
umsetzt und dann nach literaturbekannten Methoden zur Veresterung bzw. Amidierung von Carbonsäuren in die Verbindungen der Formel (I) überführt
und die Verbindungen der Formel (I) gegebenenfalls mit den entsprechenden (i) Lösungsmitteln und/oder (ii) Basen oder Säuren zu ihren Solvaten, Salzen und/oder Solvaten der Salze umsetzt.

Inerte Lösungsmittel für den Verfahrensschritt (II) + (III) → (IV) sind beispielsweise Halogenkohlenwasserstoffe wie Dichlormethan, Trichlormethan, Tetrachlormethan, Trichlorethan, Tetrachlorethan, 1,2-Dichlorethan oder Trichlorethylen, Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Ethylacetat, Aceton, Dimethylformamid, Dimethylsulfoxid, N,N'-Dimethylpropylenharnstoff (DMPU), N-Methylpyrrolidon (NMP), Pyridin oder Acetonitril. Ebenso ist es möglich, Gemische der genannten Lösungsmittel einzusetzen. Bevorzugt ist Dimethylformamid.

Als Basen für den Verfahrensschritt (II) + (III) → (IV) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat, Alkali-Alkoholate wie Natrium- oder Kaliummethanolat, Natrium- oder Kaliumethanolat oder Kalium-tert.-butylat, Alkalihydride wie Natriumhydrid, Amide wie Natriumamid, Lithium- oder Kalium-bis(trimethylsilyl)amid oder Lithiumdiisopropylamid, oder organische bicyclische Amine wie 1,5-Diazabicyclo[4.3.0]non-5-en (DBN), 1,4-Diazabicyclo-[2.2.2]octan (DABCO^{®}) oder 1,8-Diazabicydo[5.4.0]undec-7-en (DBU). Bevorzugt ist Cäsiumcarbonat.

Die Verbindung der Formel (III) sowie die Base werden hierbei jeweils in einer Menge von 1 bis 5 Mol, bevorzugt in einer Menge von 1.5 bis 2.5 Mol, bezogen auf 1 Mol der Verbindung der Formel (II), eingesetzt. Die Reaktion erfolgt im Allgemeinen in einem Temperaturbereich von -20°C bis +100°C, bevorzugt von 0°C bis +40°C. Die Umsetzung kann bei normalem, erhöhtem oder bei erniedrigtem Druck durchgeführt werden (z.B. von 0.5 bis 5 bar). Im Allgemeinen arbeitet man bei Normaldruck.

Inerte Lösungsmittel für den Verfahrensschritt (IV) + (V) → (VI) sind beispielsweise Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder Dimethylformamid. Ebenso ist es möglich, Gemische der genannten Lösungsmittel einzusetzen. Bevorzugt sind Tetrahydrofuran oder Tetrahydrofuran/Hexan-Gemische.

Als Basen für den Verfahrensschritt (IV) + (V) → (VI) eignen sich bevorzugt Phosphazen-Basen (so genannte "Schwesinger-Basen") wie beispielsweise 1-*tert*.-Butyl-2,2,4,4,4-pentakis(dimethylamino)-2λ⁵,4λ⁵-catenadi(phosphazen) oder 3-*tert*.-Butylimino-1,1,1,5,5,5-hexakis(dimethylamino)-3-[tris(dimethylamino)phosphoranyliden]amino-1λ⁵,3λ⁵,5λ⁵-1,4-triphosphazadien [vgl. z.B. R. Schwesinger, H. Schlemper, Angew. Chem. Int. Ed. Engl. 26, 1167 (1987); T. Pietzonka, D. Seebach, Chem. Ber. 124, 1837 (1991)]. Die Base wird hierbei in einer Menge von 1 bis 3 Mol, bevorzugt in einer Menge von 1.1 bis 2 Mol, bezogen auf 1 Mol der Verbindung der Formel (IV), eingesetzt.

Die Verbindung der Formel (V) wird in diesem Verfahrensschritt in einer Menge von 1 bis 5 Mol, bevorzugt in einer Menge von 2 bis 4 Mol, bezogen auf 1 Mol der Verbindung der Formel (IV), eingesetzt. Die Reaktion erfolgt im Allgemeinen in einem Temperaturbereich von -100°C bis 0°C, bevorzugt von -80°C bis -20°C. Die Umsetzung kann bei normalem, erhöhtem oder bei erniedrigtem Druck durchgeführt werden (z.B. von 0.5 bis 5 bar). Im Allgemeinen arbeitet man bei Normaldruck.

Inerte Lösungsmittel für den Verfahrensschritt (VI) → (VII) sind beispielsweise Halogenkohlenwasserstoffe wie Dichlormethan, 1,2-Dichlorethan oder Trichlorethylen, Ether wie Diethylether, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, Kohlenwasserstoffe wie Benzol, Xylol, Toluol, Hexan, Cyclohexan oder Erdölfraktionen, oder andere Lösungsmittel wie Aceton, Dimethylformamid, Dimethylsulfoxid, Acetonitril, N-Methylpyrrolidinon oder auch Wasser. Ebenso ist es möglich, Gemische der genannten Lösungsmittel zu verwenden. Bevorzugt werden Dioxan/Wasser-, Tetrahydrofuran/Wasser-, Methanol/Wasser- oder Tetrahydrofuran/Methanol/Wasser-Gemische eingesetzt.

Als Basen für den Verfahrensschritt (VI) → (VII) eignen sich die üblichen anorganischen Basen. Hierzu gehören bevorzugt Alkalihydroxide wie beispielsweise Lithium-, Natrium- oder Kaliumhydroxid, oder Alkali- oder Erdalkalicarbonate wie Natrium-, Kalium- oder Calciumcarbonat. Besonders bevorzugt ist Natriumhydroxid. Die Base wird hierbei in einer Menge von 1 bis 5, bevorzugt von 1.5 bis 3 Mol, bezogen auf 1 Mol der Verbindung der Formel (VI) eingesetzt.

Als Säuren für den Verfahrensschritt (VI) → (VII) eignen sich wässrige Lösungen der üblichen anorganischen Säuren wie beispielsweise Salzsäure, Schwefelsäure, Phosphorsäure oder Bromwasserstoffsäure, oder Sulfonsäuren wie Toluolsulfonsäure, Methansulfonsäure oder Trifluormethansulfonsäure, oder Carbonsäuren wie Trifluoressigsäure.

Die Reaktion erfolgt im Allgemeinen in einem Temperaturbereich von -20°C bis +100°C, bevorzugt von 0°C bis +40°C. Die Umsetzung kann bei normalem, erhöhtem oder bei erniedrigtem Druck durchgeführt werden (z.B. von 0.5 bis 5 bar). Im Allgemeinen arbeitet man bei Normaldruck.

Der Verfahrensschritt (VII) → (I) wird nach literaturbekannten Methoden zur Veresterung bzw. Amidierung (Amid-Bildung) von Carbonsäuren durchgeführt.

Inerte Lösungsmittel für eine Amidierung im Verfahrensschritt (VII) → (I) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Ethylacetat, Pyridin, Dimethylsulfoxid, Dimethylformamid, N,N'-Dimethylpropylenharnstoff (DMPU), N-Methylpyrrolidon (NMP), Acetonitril oder Aceton. Ebenso ist es möglich, Gemische der genannten Lösungsmittel zu verwenden. Bevorzugt sind Dichlormethan, Tetrahydrofuran, Dimethylformamid oder Gemische dieser Lösungsmittel.

Als Kondensationsmittel für eine Amidbildung im Verfahrensschritt (VII) → (I) eignen sich beispielsweise Carbodiimide wie N,N'-Diethyl-, N,N'-Dipropyl-, N,N'-DÜsopropyl-, N,N'-Dicyclohexylcarbodümid (DCC), N-(3-Dimethylaminoisopropyl)-N'-ethylcarbodiimid-Hydrochlorid (EDC), oder Phosgen-Derivate wie N,N'-Carbonyldümidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Isobutylchlorformiat, Propanphosphonsäureanhydrid, Cyanophosphonsäurediethylester, Bis-(2-oxo-3-oxazolidinyl)-phosphorylchlorid, Benzotriazol-1-yloxy-tris(dimethylamino)phosphonium-hexafluorophosphat, Benzotriazol-1-yloxy-tris(pyrrolidino)phosphonium-hexafluorophosphat (PyBOP), O-(Benzotriazol-1-yl)-N,N,N',N'-tetramethyluronium-hexafluorophosphat (HBTU), 2-(2-Oxo-1-(2H)-pyridyl)-1,1,3,3-tetramethyluronium-tetrafluoroborat (TPTU) oder O-(7-Azabenzotriazol-1-yl)-N,N,N',N'-tetramethyluronium-hexafluorophosphat (HATU), gegebenenfalls in Kombination mit weiteren Hilfsstoffen wie 1-Hydroxybenzotriazol (HOBt) oder N-Hydroxysuccinimid (HOSu), sowie als Basen Alkalicarbonate, z.B. Natrium- oder Kaliumcarbonat oder -hydrogencarbonat, oder organische Basen wie Trialkylamine, z.B. Triethylamin, N-Methylmorpholin, N-Methylpiperidin oder N,N-Diisopropylethylamin. Bevorzugt wird EDC in Kombination mit HOBt und N,N-Diisopropylethylamin, HATU in Kombination mit N,N-Diisopropylethylamin oder auch Cyanophosphonsäurediethylester in Kombination mit Triethylamin verwendet.

Eine Amidbildung im Verfahrensschritt (VII) → (I) wird im Allgemeinen in einem Temperaturbereich von 0°C bis +100°C, bevorzugt von 0°C bis +40°C, durchgeführt. Die Umsetzung kann bei normalem, erhöhtem oder bei erniedrigtem Druck durchgeführt werden (z.B. von 0.5 bis 5 bar). Im Allgemeinen arbeitet man bei Normaldruck.

Die Verbindungen der Formel (II) können in Analogie zu literaturbekannten Verfahren beispielsweise durch Schmidt-Reaktion mit Trimethylsilylazid/Schwefelsäure [vgl. z.B. F. Pozgan, S. Polanc, M. Kocevar, Heterocycles 56, 379 (2002)] aus Tetralon-Derivaten der Formel (VIII) 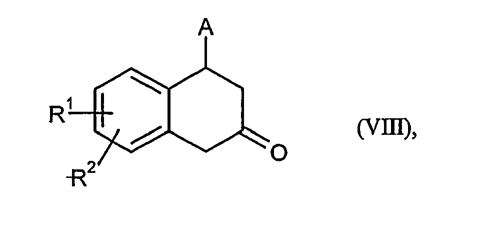 in welcher R¹, R² und A jeweils die oben angegebenen Bedeutungen haben,
hergestellt werden. Die Verbindungen der Formel (VIII) sind literaturbekannt oder können in Analogie zu literaturbekannten Verfahren hergestellt werden [vgl. z.B. *a)*: I. Fleming, A. Pearce, J. Chem. Soc. Perkin I 1980, 2485; R.S. Prasad, R.M. Roberts, Synth. Commun. 21, 3385 (1991); *b)*: G. Bertolini, V. Vecchietti, M. Mabilia, G. Norcini, A. Restelli, F. Santangelo, A.M. Villa, C. Casagrande, Eur. J. Med. Chem. 27, 663-672 (1992); c): S.D. Wyrick, R.G. Booth, A.M. Myers, C.E. Owens, N.S. Kula, R.J. Baldessarini, A.T. McPhail, R.B. Mailman, J. Med. Chem. 36, 2542 (1993); E.C. Bucholtz, R.L. Brown, A. Tropsha, R.G. Booth, S.D. Wyrick, J. Med. Chem. 42, 3041 (1999)].

Die Verbindungen der Formel (II) können auch dadurch hergestellt werden, dass man Isochromanon-Derivate der Formel (IX) 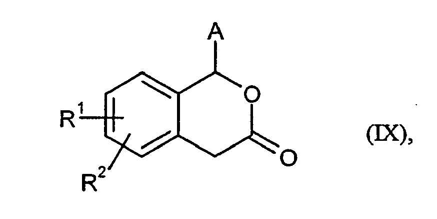 in welcher R¹, R² und A jeweils die oben angegebenen Bedeutungen haben,
zunächst mit Trimethylsilylcyanid unter Iod- und/oder Trimethylsilyliodid-Katalyse in Verbindungen der Formel (X) 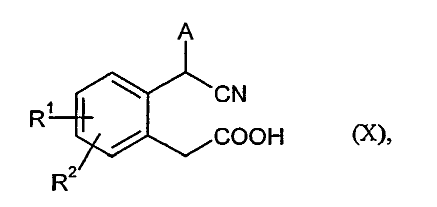 in welcher R¹, R² und A jeweils die oben angegebenen Bedeutungen haben,
überführt, diese mit Trimethylsilylchlorid in Methanol, vorzugsweise in einer Eintopf-Reaktion, zu Verbindungen der Formel (XI) 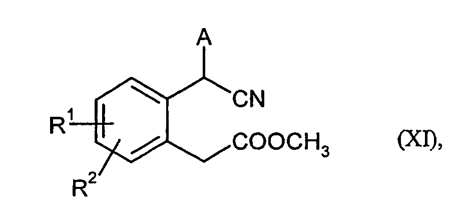 in welcher R¹, R² und A jeweils die oben angegebenen Bedeutungen haben,
verestert, anschließend durch Hydrierung in Gegenwart eines Raney-Nickel-Katalysators oder durch Reduktion mit Natriumborhydrid in Gegenwart von Nickel- oder Kobalt(II)chlorid zu Verbindungen der Formel (XII) 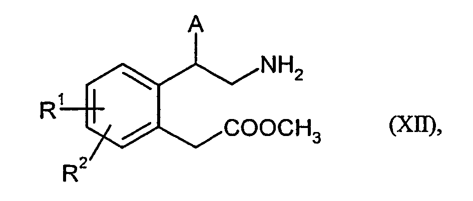 in welcher R¹, R² und A jeweils die oben angegebenen Bedeutungen haben,
umsetzt und diese dann durch Erhitzen in einem inerten Lösungsmittel wie beispielsweise Toluol, vorzugsweise in einer Eintopf-Reaktion, zu den Verbindungen der Formel (II) cyclisiert [vgl. auch Busacca et al., Tetrahedron Lett. 33 (2), 169 (1998)].

Die Verbindungen der Formel (IX) sind bekannt oder können in Analogie zu literaturbekannten Verfahren hergestellt werden [vgl. z.B. G. Brancaccio et al., J. Med. Chem. 24, 998-1000 (1981); M. Shindo et al., J. Org. Chem. 66, 7818-7824 (2001); siehe auch Schema 2].

Die Verbindungen der Formeln (III) und (V) sind kommerziell erhältlich, literaturbekannt oder können in Analogie zu literaturbekannten Verfahren hergestellt werden.

Die Herstellung der erfindungsgemäßen Verbindungen kann durch die folgenden Syntheseschemata veranschaulicht werden: 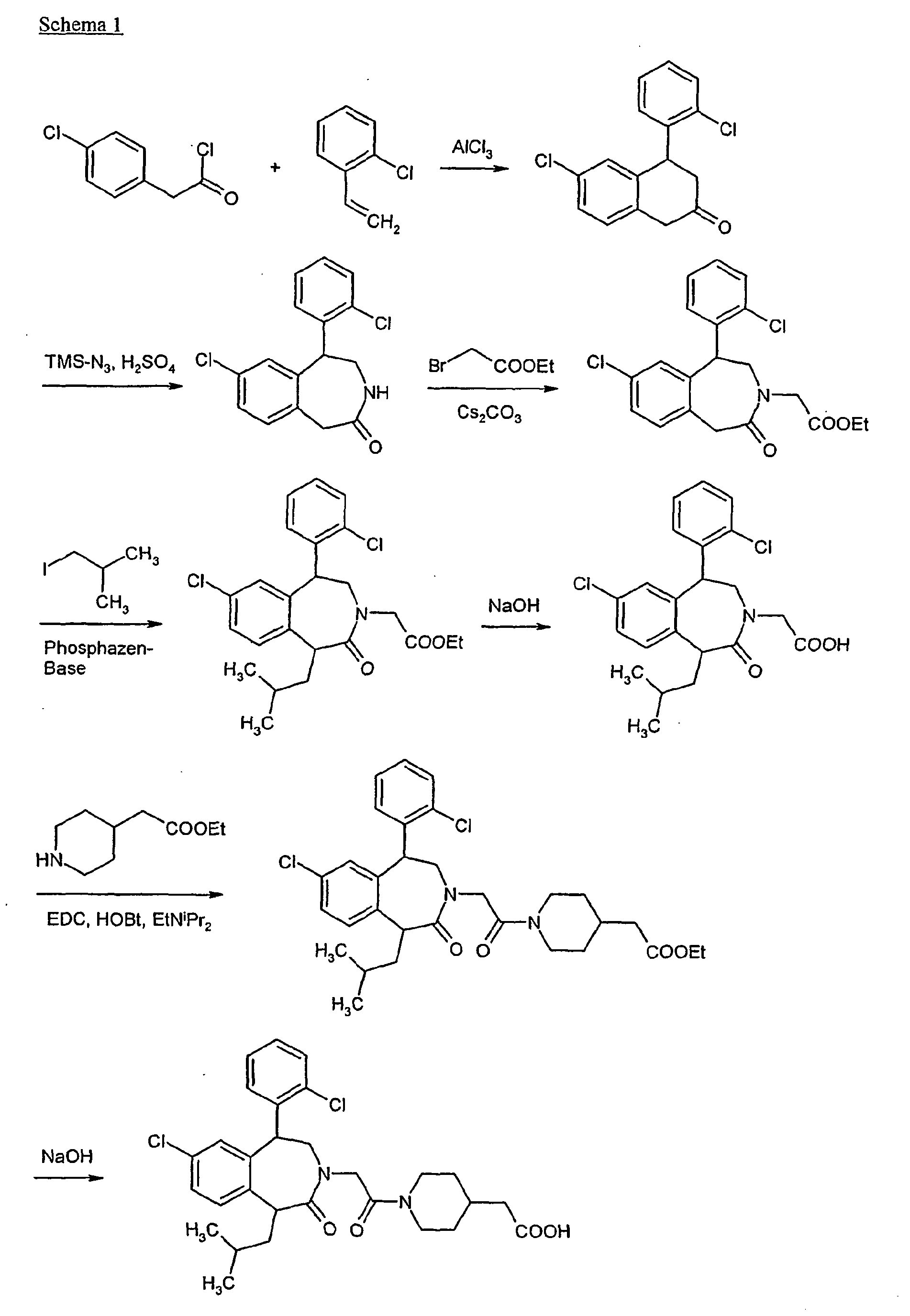 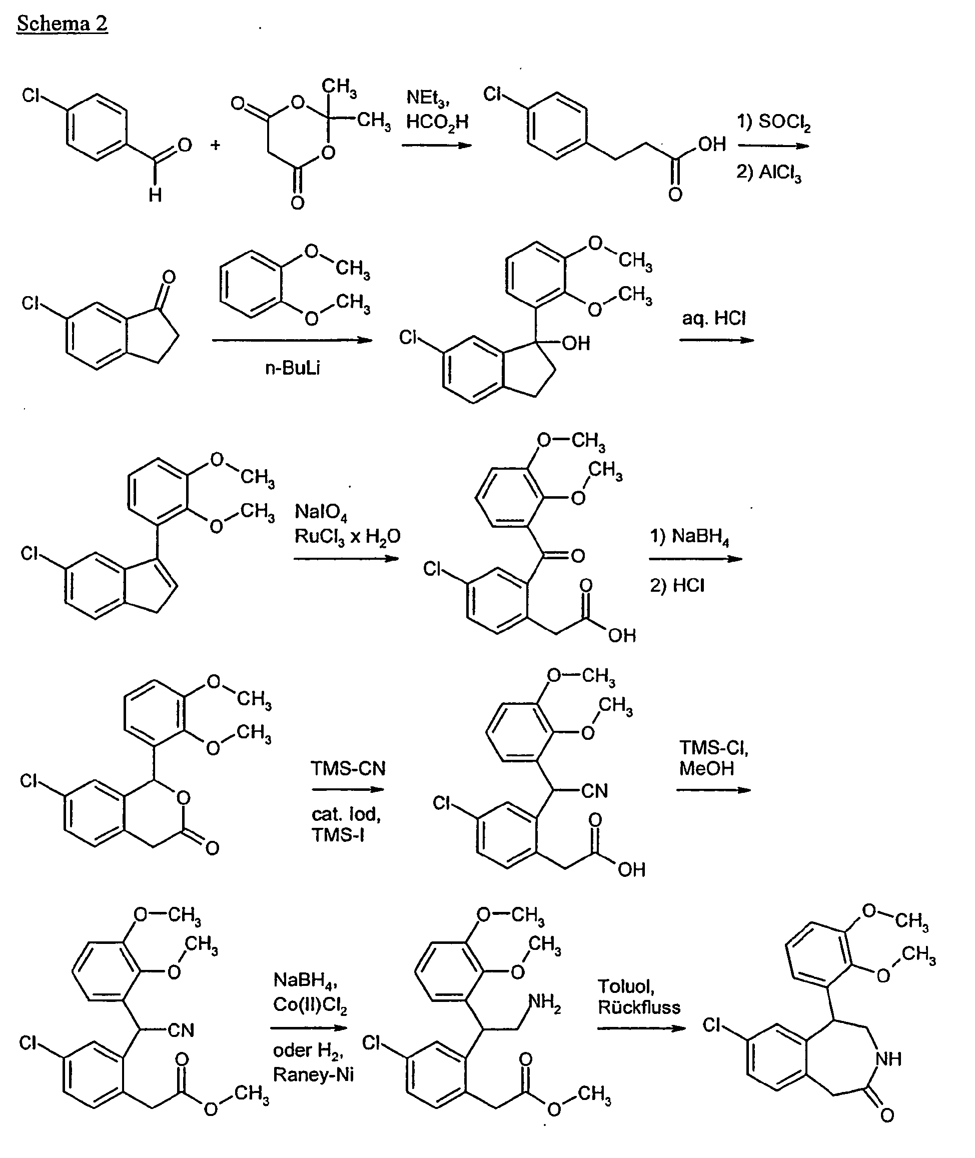 [Abkürzungen: aq. = wässrig; Bu = Butyl; cat. = katalytisch; EDC = *N'*-(3-Dimethylaminopropyl)-*N*-ethylcarbodiimid-Hydrochlorid; Et = Ethyl; HOBt = 1-Hydroxy-1*H*-benzotriazol-Hydrat; Me = Methyl; ⁱPr = Isopropyl; TMS = Trimethylsilyl].

Die erfindungsgemäßen Verbindungen besitzen wertvolle pharmakologische Eigenschaften und können zur Vorbeugung und Behandlung von Erkrankungen bei Menschen und Tieren verwendet werden. Insbesondere sind die erfindungsgemäßen Verbindungen hochwirksame Inhibitoren der Squalen-Synthase und inhibieren die Cholesterin-Biosynthese. Die erfindungsgemäßen Verbindungen bewirken eine Senkung des Cholesterin-Spiegels und des Triglycerid-Spiegels im Blut. Sie können deshalb zur Behandlung und Prävention kardiovaskulärer Erkrankungen, insbesondere von Hypolipoproteinämie, Dyslipidämien, Hyperlipidämien oder Arteriosklerose eingesetzt werden. Die erfindungsgemäßen Verbindungen können darüber hinaus auch zur Behandlung und Prävention von Fettsucht und Fettleibigkeit (obesity) verwendet werden. Die erfindungsgemäßen Verbindungen eignen sich weiterhin zur Behandlung und Prävention von Schlaganfällen (stroke) und der Alzheimer'schen Krankheit.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt: Cholesterin-senkende Statine, Cholesterin-Absorptionshemmer, HDL-erhöhende bzw. Triglycerid-senkende und/oder Apolipoprotein B-senkende Substanzen, Oxidationshemmer oder anti-entzündlich wirkende Verbindungen. Kombinationen mit diesen Wirkstoffen eignen sich bevorzugt zur Behandlung von Dyslipidämien, kombinierten Hyperlipidämien, Hypercholesterolämien oder Hypertriglyceridämien.

Die genannten Kombinationen sind auch zur primären oder sekundären Prävention koronarer Herzerkrankungen (z.B. Myokardinfarkt) einsetzbar sowie bei peripheren arteriellen Erkrankungen.

Statine im Rahmen der Erfindung sind beispielsweise Lovastatin, Simvastatin, Pravastatin, Fluvastatin, Atorvastatin, Rosuvastatin und Pitavastatin. Cholesterin-Absorptionshemmer sind z.B. Cholestyramine oder Ezetimibe; HDL-erhöhende bzw. Triglycerid-senkende oder Apolipoprotein B-senkende Substanzen sind z.B. Fibrate, Niacin, PPAR-Agonisten, IBAT-, MTP- und CETP-Inhibitoren. Anti-entzündlich wirkende Verbindungen sind z.B. Aspirin.

Weiterer Gegenstand der vorliegenden Erfindung ist außerdem die Kombination der erfindungsgemäßen Verbindungen mit einem Glucosidase- und/oder Amylasehemmer zur Behandlung von familiärer Hyperlipidämie, der Fettsucht (Adipositas) und des Diabetes mellitus.

Glucosidase- und/oder Amylasehemmer im Rahmen der Erfindung sind beispielsweise Acarbose, Adiposine, Voglibose, Miglitol, Emiglitate, MDL-25637, Camiglibose (MDL-73945), Tendamistate, AI-3688, Trestatin, Pradimicin-Q und Salbostatin. Bevorzugt ist die Kombination von Acarbose, Miglitol, Emiglitate oder Voglibose mit einer der erfindungsgemäßen Verbindungen.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhälfiisse, Verdünnungsverhältnisse und Konzentrationsangaben von flüssig/flüssig-Lösungen beziehen sich jeweils auf das Volumen.

### A. Beispiele

### Abkürzungen:

- CI: chemische Ionisation (bei MS)
- DMF: *N,N*-Dimethylformamid
- DMSO: Dimethylsulfoxid
- d. Th.: der Theorie (bei Ausbeute)
- ESI: Elektrospray-Ionisation (bei MS)
- GC/MS: Gaschromatographie-gekoppelte Massenspektroskopie
- h: Stunde(n)
- HPLC: Hochdruck-, Hochleistungsflüssigchromatographie
- LC/MS: Flüssigchromatographie-gekoppelte Massenspektroskopie
- min.: Minute(n)
- MS: Massenspektroskopie
- NMR: Kernresonanzspektroskopie
- RT: Raumtemperatur
- Rₜ: Retentionszeit (bei HPLC)
- THF: Tetrahydrofuran

### LC/MS-, GC/MS- und HPLC-Methoden:

### Methode 1:

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 2:

Instrument: Micromass Quattro LCZ mit HPLC Agilent Serie 1100; Säule: Phenomenex Synergi 2µ Hydro-RP Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 3:

Gerätetyp MS: Micromass ZQ; Gerätetyp HPLC: HP 1100 Series; UV DAD; Säule: Phenomenex Synergi 2µ Hydro-RP Mercury 20 mm × 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 4:

Instrument: Micromass GCT, GC 6890; Säule: Restek RTX-35MS, 30 m x 250 µm x 0.25 µm; konstanter Fluss mit Helium: 0.88 ml/min; Ofen: 60°C; Inlet: 250°C; Gradient: 60°C (0.30 min halten), 50°C/min → 120°C, 16°C/min → 250°C, 30°C/min → 300°C (1.7 min halten).

### Methode 5:

Instrument: HP 1100 mit DAD-Detektion; Säule: Kromasil RP-18, 60 mm x 2 mm, 3.5 µm; Eluent A: 5 ml HClO₄/l Wasser, Eluent B: Acetonitril; Gradient: 0 min 2% B → 0.5 min 2% B → 4.5 min 90% B → 6.5 min 90% B; Fluss: 0.75 ml/min; Ofen: 30°C; UV-Detektion: 210 nm.

### Methode 6:

Instrument: HP 1100 mit DAD-Detektion; Säule: Kromasil RP-18, 60 mm x 2 mm, 3.5 µm; Eluent A: 5 ml HClO₄/l Wasser, Eluent B: Acetonitril; Gradient: 0 min 2% B → 0.5 min 2% B → 4.5 min 90% B → 9 min 90% B; Fluss: 0.75 ml/min; Ofen: 30°C; UV-Detektion: 210 nm.

### Ausgangsverbindungen und Intermediate:

### Beispiel 1A

### 6-Chlor-4-(2-chlorphenyl)-3,4-dihydro-1H-naphtbalin-2-on

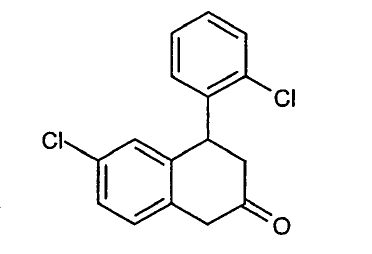

Unter Argonatmosphäre werden 1.59 g Aluminiumtrichlorid (7.93 mmol) in 80 ml Dichlormethan suspendiert und bei 0°C mit einer Lösung von 1.50 g 4-Chlorphenyl-acetylchlorid (11.90 mmol) in 40 ml Dichlormethan versetzt. Bei 0°C wird innerhalb von 30 min eine Lösung von 1.65 g 2-Chlorstyrol (11.90 mmol) in 100 ml Dichlormethan zugetropft. Die Reaktionsmischung wird auf 300 ml Eiswasser gegeben und dreimal mit Dichlormethan extrahiert. Die vereinigten organischen Phasen werden über Natriumsulfat getrocknet und am Rotationsverdampfer eingeengt. Der Rückstand wird durch Chromatographie an Kieselgel (Laufmittel: Cyclohexan/Essigsäureethylester 7:1) gereinigt. Es werden 0.795 g (31% d. Th.) der Titelverbindung erhalten.

¹H-NMR (400 MHz, CDCl₃): δ = 2.84-2.94 (m, 2H), 3.65 (d, *J* = 20.4, 1H), 3.73 (d, *J* = 20.4, 1H), 4.93 (t, *J* = 6.8, 1H), 6.88-6.91 (m, 2H), 7.15-7.17 (m, 1H), 7.20-7.27 (m, 3H), 7.45-7.47 (m, 1H).

LC/MS (Methode 1): Rₜ = 2.64 min.; MS (ESIpos): m/z = 291 [M+H]⁺.

### Beispiel 2A

### 7-Chlor-5-(2-chlorphenyl)-1,3,4,5-tetrahydrobenzo[d]azepin-2-on

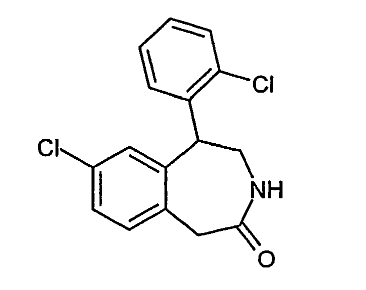

2.16 g der Verbindung aus Beispiel 1A (7.40 mmol) werden in 90 ml Dichlormethan gelöst und mit 7.4 ml konzentrierter Schwefelsäure versetzt. Unter Eiskühlung wird eine Lösung von 1.28 g Trimethylsilylazid (11.11 mmol) in 15 ml Dichlormethan zugetropft. Es wird 1 h bei Raumtemperatur gerührt und die Reaktionsmischung dann auf 300 ml Eiswasser gegeben. Durch portionsweise Zugabe von Natriumhydrogencarbonat wird die wässrige Phase schwach basisch gestellt (pH 8). Die organische Phase wird abgetrennt und die wässrige Phase dreimal mit Dichlormethan extrahiert. Die vereinigten organischen Phasen werden zweimal mit gesättigter Natriumhydrogencarbonat-Lösung gewaschen, über Natriumsulfat getrocknet und am Rotationsverdampfer eingeengt. Der Rückstand wird durch Chromatographie an Kieselgel (Laufmittel: Cyclohexan/Essigsäureethylester 2:3 → 1:2) gereinigt. Es werden 0.65 g (29% d. Th.) der Titelverbindung erhalten.

¹H-NMR (400 MHz, CDCl₃): δ = 3.63-3.71 (m, 1H), 3.83-3.90 (m, 1H), 3.94 (s, 1H), 4.91 (dd, *J* = 6.9 und 4.0, 1H), 5.74-5.81 (m, 1H), 6.74-6.76 (m, 1H), 6.87 (s, 1H), 7.13-7.22 (m, 4H), 7.41-7.43 (m, 1H).

LC/MS (Methode 2): Rₜ = 2.44 min.; MS (ES1pos): m/z = 306 [M+H]⁺.

### Beispiel 3A

### [8-Chlor-1-(2-chlorphenyl)-4-oxo-1,2,4,5-tetrahydrobenzo[d]azepin-3-yl]-essigsäureethylester

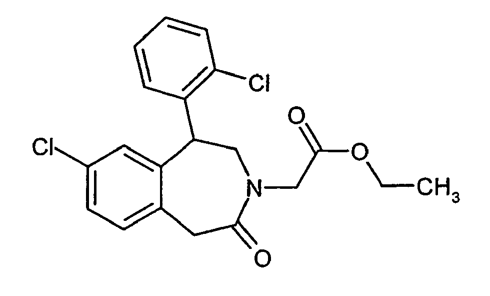

300 mg der Verbindung aus Beispiel 2A (0.98 mmol) werden in 6 ml Dimethylformamid gelöst und mit 638 mg Cäsiumcarbonat (1.96 mmol) versetzt. Es werden 220 µl Bromessigsäureethylester (327 mg, 1.96 mmol) zugetropft und die Suspension bei Raumtemperatur über Nacht gerührt. Es werden 9 ml Wasser hinzugefügt und die Mischung dreimal mit Dichlormethan extrahiert. Die vereinigten organischen Phasen werden über Natriumsulfat getrocknet und am Rotationsverdampfer eingeengt. Der Rückstand wird mittels präparativer HPLC (Eluent: Acetonitril/Wasser mit 0.1% Ameisensäure, Gradient 20:80 → 95:5) gereinigt. Es werden 132 mg (34% d. Th.) der Titelverbindung erhalten.

¹H-NMR (400 MHz, CDCl₃): δ = 1.22 (t, *J* = 7.2, 3H), 3.33-3.39 (m, 1H), 3.92-3.97 (m, 3H), 4.11-4.28 (m, 4H), 5.03 (t, *J* = 5.8, 1H), 6.72-6.74 (m, 1H), 6.88 (s, 1H), 7.15-7.22 (m, 4H), 7.41-7.43 (m, 1H).

LC/MS (Methode 3): Rₜ = 2.70 min.; MS (ESIpos): m/z = 392 [M+H]⁺.

### Beispiel 4A

### 4-Piperidylessigsäureethylester

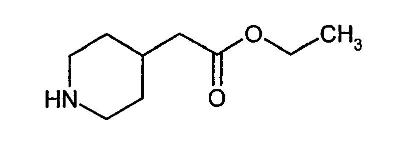

2.0 g 4-Pyridylessigsäureethylester in 20 ml Ethanol werden mit 400 mg Palladium-Schwarz (20 Gew.-%) versetzt, mit 1 N Salzsäure auf pH 2 eingestellt und bei 3 bar über 2 Tage bei Raumtemperatur hydriert. Feststoffe werden über Kieselgur abgesaugt, und das Lösungsmittel des Filtrats wird bei vermindertem Druck entfernt. Der Rückstand wird in 50 ml Essigsäureethylester und 50 ml Wasser aufgenommen. Die wässrige Phase wird mit 1 N Natronlauge auf pH 13 eingestellt und zweimal mit je 50 ml Essigsäureethylester extrahiert. Die vereinigten organischen Phasen werden über Magnesiumsulfat getrocknet, und das Lösungsmittel wird bei vermindertem Druck entfernt. Man erhält 1.21 g (58% d. Th.) des Produkts.

GC/MS (Methode 4): Rₜ = 5.93 min., m/z = 172 [M+H]⁺.

### Beispiel 5A

### [7-Chlor-5-(2-chlorphenyl)-1-(2-methylallyl)-2-oxo-1,2,4,5-tetrahydrobenzo[d]azepin-3-yl]-essigsäureethylester

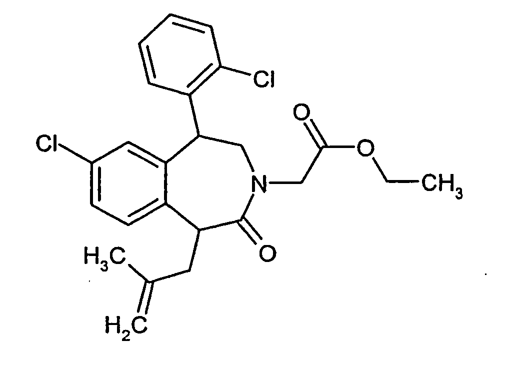

39 mg der Verbindung aus Beispiel 3A (0.10 mmol) und 30 µl 3-Brom-2-methylpropen (41 mg, 0.30 mmol) werden in 550 µl THF gelöst. Bei -78°C werden 75 µl (0.15 mmol) einer 2 M Lösung von 1-*tert*.-Butyl-2,2,4,4,4-pentakis(dimethylamino)-2λ⁵-4λ⁵-catenadi(phosphazen) in THF zugetropft und die Reaktionsmischung 4.5 h bei -78°C gerührt. Die Reaktionslösung wird mit 1 ml 1 M Salzsäure und Wasser versetzt und dreimal mit Dichlormethan extrahiert. Die vereinigten organischen Phasen werden zweimal mit gesättigter Natriumhydrogencarbonat-Lösung gewaschen, über Natriumsulfat getrocknet und am Rotationsverdampfer eingeengt. Der Rückstand wird mittels präparativer HPLC (Eluent: Acetonitril/Wasser mit 0.1% Ameisensäure, Gradient 20:80 → 95:5) gereinigt. Es werden 27 mg (60% d. Th.) der Titelverbindung erhalten.

¹H-NMR (400 MHz, GDCl₃): δ = 1.20 (t, *J* = 7.2, 3H), 1.87 (s, 3H), 2.71 (dd, *J* = 15.7 und 6.1, 1H), 2.97 (dd, *J* = 15.7 und 8.0, 1H), 3.78 (dd, *J* = 15.7 und 5.6, 1H), 3.88-4.01 (m, 1H), 4.10-4.27 (m, 4H), 4.67-4.69 (m, 1H), 4.72 (s, 1H), 4.87 (s, 1H), 5.11 (dd, *J* = 10.9 und 6.5, 1H), 6.85-6.88 (m, 1H), 6.91-6.94 (m, 1H), 7.14-7.24 (m, 4H), 7.40-7.44 (m, 1H).

LC/MS (Methode 2): Rₜ = 3.12 min.; MS (ESIpos): m/z = 445 [M+H]⁺.

### Beispiel 6A

### [7-Chlor-5-(2-chlorphenyl)-1-isobutyl-2-oxo-1,2,4,5-tetrahydrobenzo[d]azepin-3-yl]-essigsäureethylester

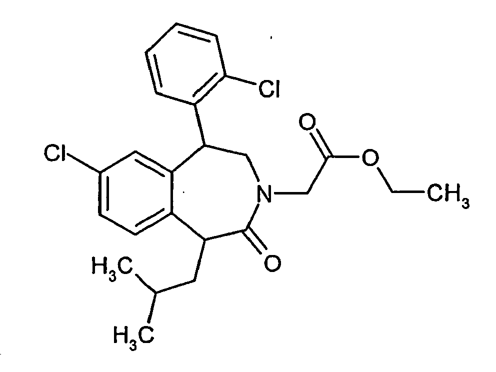

505 mg der Verbindung aus Beispiel 3A (1.29 mmol) werden in 5 ml THF gelöst. Bei -78°C werden 1.93 ml einer 1 M Lösung von 3-*tert*.-Butylimino-1,1,1,5,5,5-hexakis(dimethylamino)-3-[tris(dimethylamino)phosphoranyliden]amino-1λ⁵,3λ⁵,5λ⁵-1,4-triphosphazadien in THF zugetropft und die Reaktionslösung für 30 min. bei -78°C gerührt. Es werden 0.45 ml 1-Iod-2-methylpropan (711 mg, 3.86 mmol) zugetropft und weitere 2 h bei -78°C gerührt. Die Reaktionslösung wird mit 5 ml 1 N Salzsäure und Wasser versetzt und dreimal mit Dichlormethan extrahiert. Die vereinigten organischen Phasen werden zweimal mit gesättigter Natriumhydrogencarbonat-Lösung gewaschen, über Natriumsulfat getrocknet und am Rotationsverdampfer eingeengt. Der Rückstand wird mittels präparativer HPLC (Eluent: Acetonitril/Wasser mit 0.1% Ameisensäure, Gradient 20:80 → 95:5) gereinigt. Es werden 149 mg (26% d. Th.) der Titelverbindung erhalten.

¹H-NMR (400 MHz, CDCl₃): δ = 1.01 (d, *J* = 7.3, 3H), 1.02 (d, *J* = 6.9, 3H), 1.20 (t, *J* = 7.1, 3H), 1.67-1.84 (m, 2H), 2.21-2.28 (m, 1H), 3.55-4.28 (m, 6H), 4.39-4.50 (m, 1H), 5.04-5.08 (m, 1H), 6.79-6.81 (m, 1H), 6.92 (s, 1H), 7.14-7.27 (m, 4H), 7.41-7.43 (m, 1H).

LC/MS (Methode 1): Rₜ = 3.07 min.; MS (ESIpos): m/z = 448 [M+H]⁺.

### Beispiel 7A

### 4-(2-Chlorphenyl)-6-methyl-3,4-dihydro-1H-naphthalin-2-on

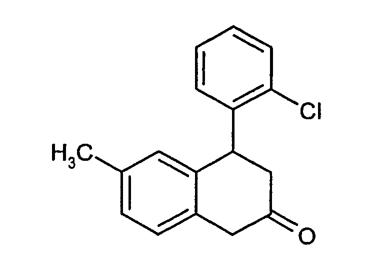

Unter Argonatmosphäre werden 5.93 g Aluminiumtrichlorid (44.48 mmol) in 250 ml Dichlormethan suspendiert und bei -20°C mit einer Lösung von 5.00 g *p*-Tolyl-acetylchlorid (29.65 mmol) in 150 ml Dichlormethan versetzt. Bei -20°C wird innerhalb von 30 min eine Lösung von 6.16 g 2-Chlorstyrol (44.48 mmol) in 350 ml Dichlormethan zugetropft. Die Reaktionsmischung wird auf 1000 ml Eiswasser gegeben und dreimal mit Dichlormethan extrahiert. Die vereinigten organischen Phasen werden über Natriumsulfat getrocknet und am Rotationsverdampfer eingeengt. Der Rückstand wird durch Chromatographie an Kieselgel (Laufinittel: Cyclohexan/Essigsäureethylester 15:1) gereinigt. Es werden 4.80 g (60% d. Th.) der Titelverbindung erhalten.

¹H-NMR (400 MHz, CDCl₃): δ = 2.26 (s, 3H), 2.83-2.96 (m, 2H), 3.62 (d, *J* = 20.4, 1H), 3.72 (d, *J* = 20.4,1 H), 4.93 (t, *J* = 6.5, 1H), 6.57 (s, 1H), 6.84-6.87 (m, 1H), 7.06-7.23 (m, 4H), 7.42-7.45 (m, 1H).

HPLC (Methode 5): Rₜ = 5.15 min.; MS (CI): m/z = 288 [M+NH₄]⁺.

### Beispiel 8A

### 4-(2-Bromphenyl)-6-chlor-3,4-dihydro-1H-naphthalin-2-on

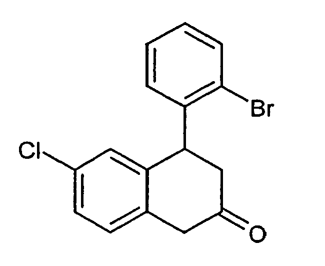

Unter Argonatmosphäre werden bei 0°C 5.01 g Aluminiumtrichlorid (38.24 mmol) in 400 ml Dichlormethan suspendiert und bei gleicher Temperatur mit einer Lösung von 4.82 g 4-Chlorphenyl-acetylchlorid (25.49 mmol) in 200 ml Dichlormethan versetzt. Bei 0°C wird innerhalb von 30 min eine Lösung von 7.00 g 2-Bromstyrol (38.24 mmol) in 500 ml Dichlormethan zugetropft. Nach 10 min Rühren bei 0°C wird die Reaktionsmischung auf 1000 ml Eiswasser gegeben und dreimal mit Dichlormethan extrahiert. Die vereinigten organischen Phasen werden über Natriumsulfat getrocknet und am Rotationsverdampfer eingeengt. Der Rückstand wird durch Chromatographie an Kieselgel (Laufmittel: Cyclohexan/Essigsäureethylester 25:1 → 15:1) gereinigt. Es werden 4.46 g (51 % d. Th.) der Titelverbindung erhalten.

¹H-NMR (400 MHz, CDCl₃): δ = 2.88 (d, *J* = 7.0, 2H), 3.65 (d, *J* = 20.4, 1H), 3.74 (d, *J* = 20.4, 1H), 4.93 (t, *J* = 7.0, 1H), 6.88-6.91 (m, 2H), 7.15-7.29 (m, 2H), 7.42-7.29 (m, 2H), 7.65 (dd, *J* = 7.9, *J* = 1.2, 1H).

HPLC (Methode 5): Rₜ = 5.22 min.; MS (CI): m/z = 352 [M+NH₄]⁺.

### Beispiel 9A

### 5-(2-Chlorphenyl)-7-methyl-1,3,4,5-tetrahydrobenzo[d]azepin-2-on

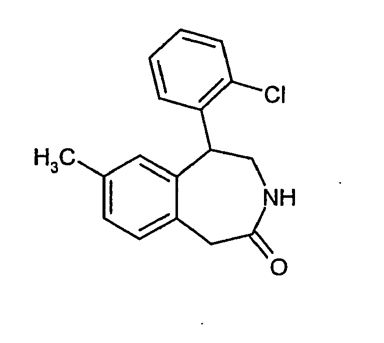

4.80 g der Verbindung aus Beispiel 7A (17.73 mmol) werden in 250 ml Dichlormethan gelöst und mit 20.0 ml konzentrierter Schwefelsäure versetzt. Unter Eiskühlung wird eine Lösung von 3.06 g Trimethylsilylazid (3.53 ml, 26.59 mmol) in 55 ml Dichlormethan zugetropft. Es wird 1 h bei Raumtemperatur gerührt und die Reaktionsmischung dann auf 1500 ml Eiswasser gegeben. Durch portionsweise Zugabe von Natriumhydrogencarbonat wird die wässrige Phase schwach basisch gestellt (pH 8). Die organische Phase wird abgetrennt und die wässrige Phase dreimal mit Dichlormethan extrahiert. Die vereinigten organischen Phasen werden zweimal mit gesättigter Natriumhydrogencarbonat-Lösung gewaschen, über Natriumsulfat getrocknet und am Rotationsverdampfer eingeengt. Der Rückstand wird durch Chromatographie an Kieselgel (Laufmittel: Cyclohexan /Essigsäureethylester 2:3 → 1:2) gereinigt. Es werden 1.48 g (29% d. Th.) der Titelverbindung erhalten.

¹H-NMR (400 MHz, CDCl₃): δ = 2.18 (s, 3H), 3.58-3.68 (m, 1H), 3.85-3.88 (m, 1H), 3.88 (d, *J* = 14.5, 1H), 3.97 (d, *J* = 14.5, 1H, 4.90-4.94 (m, 1H), 5.54-5.64 (m, 1H), 6.67 (s, 1H), 6.73-6.76 (m, 1H), 6.97-7.00 (m, 1H), 7.07-7.20 (m, 3H), 7.39-7.42 (m, 1H).

HPLC (Methode 5): Rₜ = 4.51 min.; MS (CI): m/z = 286 [M+H]⁺.

### Beispiel 10A

### 5-(2-Bromphenyl)-7-chlor-1,3,4,5-tetrahydrobenzo[d]azepin-2-on

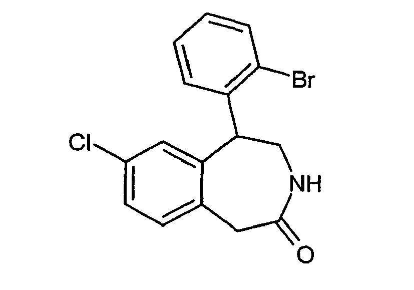

5.80 g der Verbindung aus Beispiel 8A (17.28 mmol) werden in 250 ml Dichlormethan gelöst und mit 20.0 ml konzentrierter Schwefelsäure versetzt. Unter Eiskühlung wird eine Lösung von 2.99 g Trimethylsilylazid (3.44 ml, 25.92 mmol) in 55 ml Dichlormethan zugetropft. Es wird 1 h bei Raumtemperatur gerührt und die Reaktionsmischung dann auf 1500 ml Eiswasser gegeben. Durch portionsweise Zugabe von Natriumhydrogencarbonat wird die wässrige Phase schwach basisch gestellt (pH 8). Die organische Phase wird abgetrennt und die wässrige Phase dreimal mit Dichlormethan extrahiert. Die vereinigten organischen Phasen werden zweimal mit gesättigter Natriumhydrogencarbonat-Lösung gewaschen, über Natriumsulfat getrocknet und am Rotationsverdampfer eingeengt. Der Rückstand wird durch Chromatographie an Kieselgel (Laufmittel: Cyclohexan /Essigsäureethylester 1:1 → 1:3) gereinigt. Es werden 1.59 g (24% d. Th.) der Titelverbindung erhalten.

¹H-NMR (400 MHz, CDCl₃): δ = 3.62-3.69 (m, 1H), 3.84-3.91 (m, 1H), 3.94 (s, 2H), 4.91 (dd, *J* = 6.7, *J* = 3.9,1H), 5.63-5.67 (m, 1H), 6.72-6.75 (m, 1H), 6.86 (s, 1H), 7.10-7.22 (m, 4H), 7.61 (dd, *J* = 7.9, *J* = 1.3, 1 H).

HPLC (Methode 6): Rₜ = 4.63 min.; MS (CI): m/z = 367 [M+NH₄]⁺.

### Beispiel 11A

### [1-(2-Chlorphenyl)-8-methyl-4-oxo-1,2,4,5-tetrahydrobenzo[d]azepin-3-yl]-essigsäureethylester

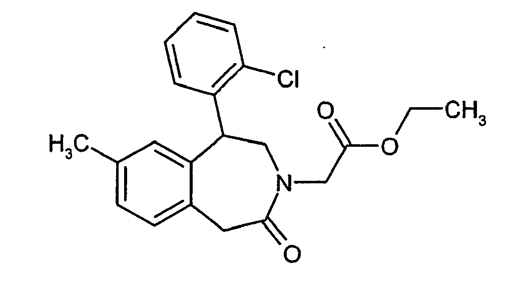

1400 mg der Verbindung aus Beispiel 9A (4.90 mmol) werden in 30 ml Dimethylformamid gelöst und mit 3192 mg Cäsiumcarbonat (9.80 mmol) versetzt. Es werden 1.09 ml Bromessigsäureethylester (1636 mg, 9.80 mmol) zugetropft und die Suspension bei Raumtemperatur über Nacht gerührt. Es wird Wasser hinzugefügt und die Mischung dreimal mit Dichlormethan extrahiert. Die vereinigten organischen Phasen werden über Natriumsulfat getrocknet und am Rotationsverdampfer eingeengt. Der Rückstand wird mittels präparativer HPLC (Eluent: Acetonitril/Wasser mit 0.1% Ameisensäure, Gradient 20:80 → 95:5) gereinigt. Es werden 670 mg (37% d. Th.) der Titelverbindung erhalten.

¹H-NMR (400 MHz, CDCl₃): δ = 1.22 (t, *J* = 7.2, 3H), 2.18 (s, 3H), 3.27 (d, *J* = 17.5, 1H), 3.85-4.18 (m, 6H), 4.22 (d, *J* = 17.5, 1H), 4.99-5.02 (m, 1H), 6.68-6.72 (m, 2H), 6.97-6.99 (m, 1H), 7.08-7.21 (m, 3H), 7.40-7.42 (m, 1H).

LC/MS (Methode 2): Rₜ = 2.67 min.; MS (ESIpos): m/z = 372 [M+H]⁺.

### Beispiel 12A

### [1-(2-Bromphenyl)-8-chlor-4-oxo-1,2,4,5-tetrahydrobenzo[d]azepin-3-yl]-essigsäureethylester

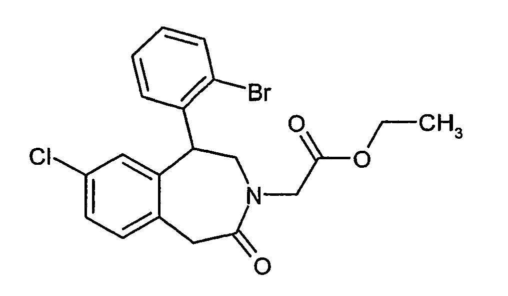

1500 mg der Verbindung aus Beispiel 10A (4.28 mmol) werden in 30 ml Dimethylformamid gelöst und mit 2788 mg Cäsiumcarbonat (8.56 mmol) versetzt. Es werden 0.95 ml Bromessigsäureethylester (1429 mg, 8.56 mmol) zugetropft und die Suspension bei Raumtemperatur über Nacht gerührt. Es wird Wasser hinzugefügt und die Mischung dreimal mit Dichlormethan extrahiert. Die vereinigten organischen Phasen werden über Natriumsulfat getrocknet und am Rotationsverdampfer eingeengt. Der Rückstand wird mittels präparativer HPLC (Eluent: Acetonitril/Wasser mit 0.1% Ameisensäure, Gradient 20:80 → 95:5) gereinigt. Es werden 450 mg (24% d. Th.) der Titelverbindung erhalten.

HPLC (Methode 6): Rₜ = 5.08 min.; MS (CI): m/z = 453 [M+NH₄]⁺.

### Beispiel 13A

### [5-(2-Chlorphenyl)-1-isobutyl-7-methyl-2-oxo-1,2,4,5-tetrahydrobenzo[d]azepin-3-yl]-essigsäureethylester

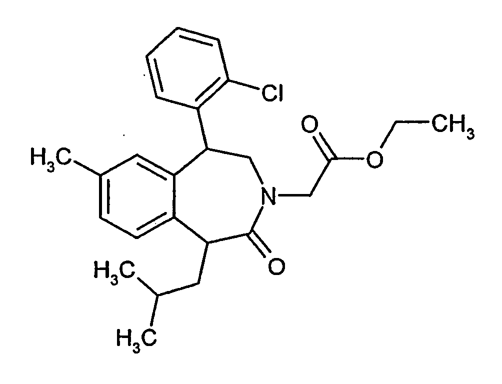

660 mg der Verbindung aus Beispiel 11A (1.77 mmol) werden in 9.2 ml THF gelöst. Bei -78°C werden 2.66 ml einer 1 M Lösung von 3-*tert*.-Butylimino-1,1,1,5,5,5-hexakis(dimethylamino)-3-[tris(dimethylamino)phosphoranyliden]amino-1λ⁵,3λ⁵,5λ⁵-1,4-triphosphazadien in *n*-Hexan zugetropft und die Reaktionslösung für 45 min. bei -78°C gerührt. Es werden 490 mg 1-Iod-2-methylpropan (2.66 mmol) zugetropft und weitere 3 h bei -78°C gerührt. Die Reaktionslösung wird mit 25 ml 1 N Salzsäure und Wasser versetzt und dreimal mit Dichlormethan extrahiert. Die vereinigten organischen Phasen werden zweimal mit gesättigter Natriumhydrogencarbonat-Lösung gewaschen, über Natriumsulfat getrocknet und am Rotationsverdampfer eingeengt. Der Rückstand wird mittels präparativer HPLC (Eluent: Acetonitril/Wasser mit 0.1% Ameisensäure, Gradient 20:80 → 95:5) gereinigt. Es werden 410 mg (54% d. Th.) der Titelverbindung erhalten.

¹H-NMR (400 MHz, CDCl₃): δ = 1.01 (d, *J* = 6.2, 3H), 1.02 (d, *J* = 6.4, 3H), 1.20 (t, *J* = 7.2, 3H), 1.68-1.88 (m, 2H), 2.15-2.28 (m, 1H), 2.18 (s, 3H), 3.95-4.22 (m, 3H), 4.13 (q, *J* = 7.2, 2H), 4.38-4.43 (m, 1H), 5.04 (t, *J* = 7.4, 1H), 6.72 (s, 1H), 6.78-6.81 (m, 1H), 6.99-7.03 (m, 1H), 7.10-7.20 (m, 3H), 7.39-7.42 (m, 1H).

LC/MS (Methode 2): Rₜ = 3.21 min.; MS (ESIpos): m/z = 428 [M+H]⁺.

### Beispiel 14A

### [5-(2-Bromphenyl)-7-chlor-1-isobutyl-2-oxo-1,2,4,5-tetrahydrobenzo[d]azepin-3-yl]-essigsäureethylester

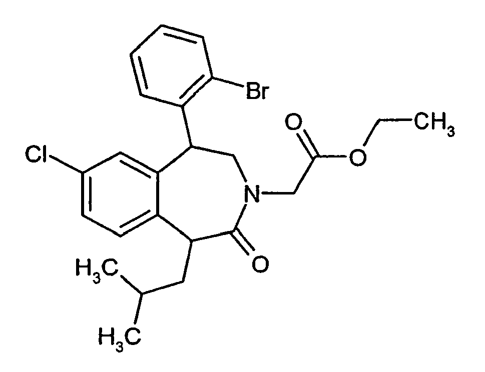

440 mg der Verbindung aus Beispiel 12A (1.01 mmol) werden in 5.2 ml THF gelöst. Bei -78°C werden 1.51 ml einer 1 M Lösung von 3-*tert*.-Butylimino-1,1,1,5,5,5-hexakis(dimethylamino)-3-[tris(dimethylamino)phosphoranyliden]amino-1λ⁵,3λ⁵,5λ⁵-1,4-triphosphazadien in *n*-Hexan (1.51 mmol) zugetropft und die Reaktionslösung für 30 min. bei -78°C gerührt. Es werden 278 mg 1-Iod-2-methylpropan (1.51 mmol) zugetropft und weitere 3 h bei -78°C gerührt. Die Reaktionslösung wird mit 14 ml 1 N Salzsäure und Wasser versetzt und dreimal mit Dichlormethan extrahiert. Die vereinigten organischen Phasen werden zweimal mit gesättigter Natriumhydrogencarbonat-Lösung gewaschen, über Natriumsulfat getrocknet und am Rotationsverdampfer eingeengt. Der Rückstand wird mittels präparativer HPLC (Eluent: Acetonitril/Wasser mit 0.1% Ameisensäure, Gradient 20:80 → 95:5) gereinigt. Es werden 200 mg (40% d. Th.) der Titelverbindung erhalten.

¹H-NMR (400 MHz, CDCl₃): δ = 1.01 (d, J= 6.2, 3H), 1.02 (d, *J* = 6.4, 3H), 1.20 (t, *J* = 7.2, 3H), 1.66-1.87 (m, 2H), 2.20-2.30 (m, 1H), 3.83-4.19 (m, 2H), 4.14 (q, *J* = 7.2, 2H), 4.23 (d, *J* = 17.4, 1H), 4.41-4.48 (m, 1H), 5.05 (t, *J* = 7.6, 1H), 6.78-6.81 (m, 1H), 6.92 (s, 1H), 7.09-7.24 (m, 4H), 7.61 (dd, J = 7.8, J = 1.4,1H).

LC/MS (Methode 3): Rₜ = 3.24 min.; MS (ESIpos): m/z = 492 [M+H]⁺.

### Beispiel 15A

### 3-(4-Chlorphenyl)propionsäure

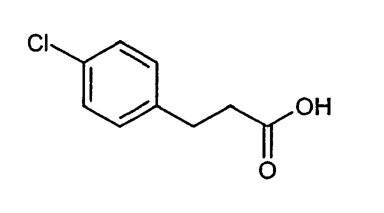

Zu 55.2 g Ameisensäure (45.3 ml, 1.20 mol) werden unter Kühlen und Rühren 48.2 g Triethylamin (67.2 ml, 0.48 mol) zugetropft. Zu diesem Gemisch werden bei Raumtemperatur 28.1 g 4-Chlorbenzaldehyd (0.20 mol), 28.8 g Meldrumsäure (0.20 mmol) und 100 ml DMF gegeben. Das Gemisch wird unter Rühren langsam auf 95°C erwärmt und für weitere 2 h bei dieser Temperatur gerührt. Nach dem Abkühlen wird das Reaktionsgemisch mit 6 N Salzsäure auf einen pH-Wert von 1 angesäuert und für 16 h bei 5°C gelagert. Die auskristallisierte Zielverbindung wird abfiltriert und im Hochvakuum getrocknet. Es werden 32.4 g (88% d. Th.) der Titelverbindung erhalten.

¹H-NMR (400 MHz, DMSO-d₆): δ = 2.53 (t, *J* = 7.6, 3H), 2.80 (d, *J* = 7.6, 3H), 7.24-7.27 (m, 2H), 7.32-7.34 (m, 2H), 12.14 (br. s, 1H).

LC/MS (Methode 1): Rₜ, = 1.75 min.; MS (ESIneg): m/z = 183 [M-H]⁻.

### Beispiel 16A

### 6-Chlorindan-1-on

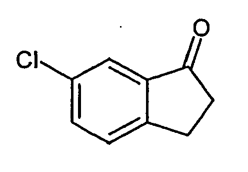

55.0 g der Verbindung aus Beispiel 15A (297.8 mmol) werden in 212.7 g Thionylchlorid (130 ml, 1788.5 mmol) suspendiert und das Gemisch 1 h unter Rückfluss erhitzt. Das überschüssige Thionylchlorid wird abdestilliert, der Rückstand in Dichlormethan aufgenommen und das Lösungsmittel am Rotationsverdampfer entfernt. Das entstandene rohe Säurechlorid wird ohne weitere Reinigung weiter umgesetzt. Hierfür werden 60.5 g des Säurechlorids (297.9 mmol) in 100 ml *n*-Heptan gelöst und portionsweise mit insgesamt 47.7 g Aluminiumtrichlorid (357.5 mmol) versetzt. Die Zugabe erfolgt derart, dass die Temperatur des Gemisches 25°C nicht überschreitet. Es wird 4 h bei Raumtemperatur gerührt und anschließend das Reaktionsgemisch langsam auf 500 ml Eiswasser gegeben. Das Gemisch wird viermal mit Essigsäureethylester extrahiert und die vereinigten organischen Phasen über Natriumsulfat getrocknet. Die Lösung wird bis auf 200 ml Restvolumen eingeengt und für 16 h bei 5°C gelagert. Die auskristallisierte Zielverbindung wird abfiltriert, mit wenig *n*-Pentan gewaschen und im Hochvakuum getrocknet. Es fallen 34.1 g (69% d. Th.) der Titelverbindung an.

¹H-NMR (400 MHz, CDCl₃): δ = 2.71-2.75 (m, 2H), 3.10-3.14 (m, 1H), 7.40-7.43 (m, 1H), 7.53-7.56 (m, 1H), 7.71-7. 72 (m, 1H).

LC/MS (Methode 2): Rₜ = 2.09 min.; MS (ESIpos): m/z = 167 [M+H]⁺.

### Beispiel 17A

### 2-(5-Chlor-1H-inden-3-yl)phenyl-methylether

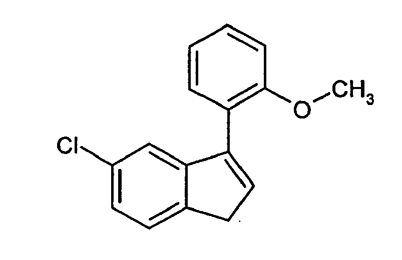

Unter Argonatmosphäre werden 88 ml einer 1 M Lösung von 2-Methoxyplienylmagnesiumbromid in THF (88.0 mmol) vorgelegt und bei 0°C mit einer Lösung von 12.80 g 6-Chlorindan-1-on (73.45 mmol) aus Beispiel 16A in 60 ml THF tropfenweise versetzt. Die Reaktionsmischung wird 1.5 h bei Raumtemperatur gerührt und zur Aufarbeitung mit 200 ml 1 N Salzsäure versetzt. Das Gemisch wird dreimal mit Essigsäureethylester extrahiert, die vereinigten organischen Phasen über Natriumsulfat getrocknet und das Lösungsmittel am Rotationsverdampfer entfernt. Der Rückstand wird in 400 ml Dichlormethan gelöst, mit 0.10 g 4-Toluolsulfonsäure-Monohydrat (0.53 mmol) versetzt und bei Raumtemperatur 16 h lang gerührt. Die Reaktionsmischung wird mit gesättigter Natriumhydrogencarbonat-Lösung und gesättigter Natriumchlorid-Lösung gewaschen und über Natriumsulfat getrocknet. Das Lösungsmittel wird am Rotationsverdampfer entfernt und der Rückstand über Chromatographie an Kieselgel (Laufinittel: Cyclohexan/Essigsäureethylester 150:1) gereinigt. Es werden 12.02 g (61 % d. Th.) der Titelverbindung erhalten.

¹H-NMR (400 MHz, CDCl₃): δ = 3.50 (d, *J* = 2.0, 2H), 3.81 (s, 3H), 6.62 (t, *J* = 2.0, 1H), 6.99-7.06 (m, 2H), 7.15-7.22 (m, 2H), 7.33-7.42 (m, 3H).

LC/MS (Methode 3): Rₜ = 3.10 min.; MS (ESIpos): m/z = 257 [M+H]⁺.

### Beispiel 18A

### [4-Chlor-2-(2-methoxybenzoyl)phenyl]essigsäure

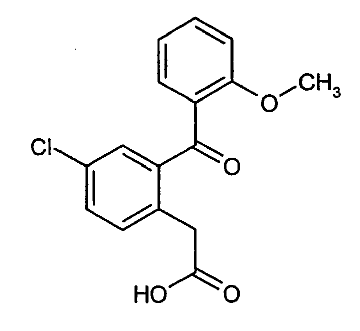

12.02 g der Verbindung aus Beispiel 17A (44.76 mmol) werden mit 120 ml Acetonitril, 120 ml Hexan und 180 ml Wasser versetzt. Es werden 39.25 g Natriumperiodat (183.51 mmol) sowie 0.18 g Ruthenium(III)chlorid-Hydrat (0.81 mmol) hinzugefügt und das Gemisch für 48 h bei Raumtemperatur gerührt. Zur Aufarbeitung wird das Reaktionsgemisch mit einer 5%-igen wässrigen Trifluoressigsäure-Lösung angesäuert und dreimal mit Methylenchlorid extrahiert. Die vereinigten organischen Phasen werden mit gesättigter Natriumchlorid-Lösung gewaschen, über Natriumsulfat getrocknet und das Lösungsmittel am Rotationsverdampfer entfernt. Der Rückstand wird in Essigsäureethylester aufgenommen und die Lösung bei 5°C für 16 h gelagert. Die abgeschiedenen Kristalle werden abfiltriert und im Hochvakuum getrocknet. Es werden 5.12 g (38% d. Th.) der Titelverbindung erhalten.

¹H-NMR (400 MHz, CDCl₃): δ = 3.68 (s, 3H), 3.85 (s, 2H), 6.96 (d, *J* = 8.3, 1H), 7.07 (t, *J* = 7.5, 1H), 7.35-7.48 (m, 3H), 7.53-7.59 (m, 2H).

LC/MS (Methode 1): Rₜ = 1.97 min.; MS (ESIpos): m/z = 305 [M+H]⁺.

### Beispiel 19A

### 7-Chlor-1-(2-methoxyplienyl)-1,4-dihydro-3H-isochroman-3-on

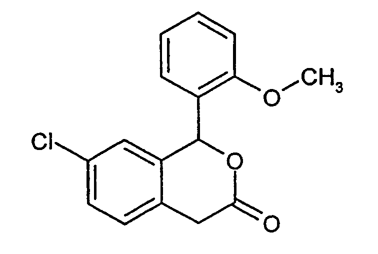

898 mg der Verbindung aus Beispiel 18A (2.95 mmol) werden in 25 ml Ethanol gelöst. Es werden 167 mg Natriumborhydrid (4.42 mmol) zugegeben und die Reaktionsmischung 16 h lang bei Raumtemperatur gerührt. Die Reaktionslösung wird mit 20 ml 20%-iger Salzsäure versetzt, mit Wasser verdünnt, die organische Phase am Rotationsverdampfer entfernt und die verbleibende wässrige Phase dreimal mit Dichlormethan extrahiert. Die vereinigten organischen Phasen werden über Natriumsulfat getrocknet und das Lösungsmittel am Rotationsverdampfer entfernt. Der Rückstand wird durch Chromatographie an Kieselgel (Laufmittel: Cyclohexan/Essigsäureethylester 5:1) gereinigt. Es werden 712 mg (83% d. Th.) der Titelverbindung erhalten.

¹H-NMR (400 MHz, CDCl₃): δ = 3.80 (s, 2H), 3.85 (s, 3H), 6.73 (s, 1H), 6.80 (s, 1H), 6.98-7.05 (m, 2H), 7.17-7.32 (m, 2H), 7.3 8-7.43 (m, 1H).

LC/MS (Methode 1): Rₜ = 2.35 min.; MS (ES1pos): m/z = 389 [M+H]⁺.

### Beispiel 20A

### Methyl {4-chlor-2-[cyano(2-methoxyphenyl)methyl]phenyl}acetat

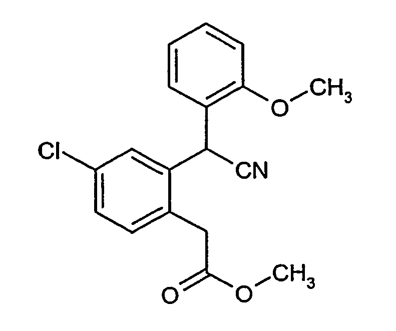

2.462 g der Verbindung aus Beispiel 19A (8.53 mmol) werden in 85 ml Ethanol gelöst und mit 1.015 g Trimethylsilylcyanid (10.23 mmol) sowie 0.108 g Iod (0.43 mmol) versetzt. Die Reaktionsmischung wird bei Raumtemperatur 16 h lang gerührt und dann erneut mit 0.443 mg Trimethylsilylcyanid (4.26 mmol) sowie 0.108 g Iod (0.43 mmol) versetzt. Nach 16 h Rühren bei Raumtemperatur werden 10 ml Wasser sowie 30 ml Acetonitril hinzugefügt, die Mischung 20 min bei 40°C gerührt und dann am Rotationsverdampfer eingeengt. Der Rückstand wird in 60 ml Methanol gelöst, tropfenweise mit 1.947 g Trimethylsilylchlorid (17.92 mmol) versetzt und bei Raumtemperatur für 16 h gerührt. Das Lösungsmittel wird am Rotationsverdampfer entfernt und der Rückstand über Chromatographie an Kieselgel (Laufmittel: Cyclohexan/Essigsäureethylester 5:1) gereinigt. Es werden 1.20 g (43% d. Th.) der Titelverbindung erhalten.

¹H-NMR (400 MHz, CDCl₃): δ = 3.54 (d, *J* = 16.0, 1H), 3.62 (s, 3H), 3.65 (d, *J* = 16.0, 1H), 3.85 (s, 3H), 5.69 (s, 1H), 6.89-6.91 (m, 1H), 6.96-6.99 (m, 1H), 7.18-7.35 (m, 4H), 7.49-7.50 (m, 1H).

LC/MS (Methode 3): Rₜ = 2.66 min.; MS (ESIpos): m/z = 330 [M+H]⁺.

### Beispiel 21A

### 7-Chlor-5-(2-methoxyphenyl)-1,3,4,5-tetrahydro-2H-3-benzazepin-2-on

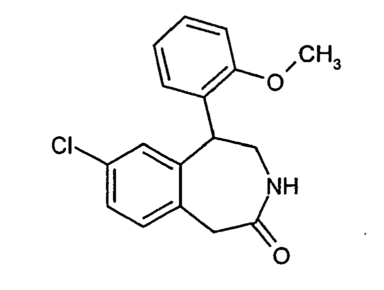

201 mg der Verbindung aus Beispiel 20A (0.61 mmol) werden in 30 ml Ethanol gelöst, in einer Hydrierapparatur vorgelegt und mit wasserfreiem Raney-Nickel versetzt. Der Katalysator wurde zuvor durch mehrmaliges Waschen von 1.0 ml einer 50%-igen wässrigen Raney-Nickel-Suspension mit Ethanol gewonnen. Es wird für 2.5 h bei Raumtemperatur und Normaldruck hydriert. Anschließend wird der Katalysator unter Argonatmosphäre abfiltriert, dreimal mit Ethanol gewaschen und die vereinigten Filtrate eingeengt. Der Rückstand wird in 30 ml Toluol gelöst und für 5 h unter Rückfluss erhitzt. Das Lösungsmittel wird am Rotationsverdampfer entfernt und der Rückstand mittels präparativer HPLC (Eluent: Acetonitril/Wasser, Gradient 20:80 → 95:5) gereinigt. Es werden 55 mg (30% d. Th.) der Titelverbindung erhalten.

¹H-NMR (400 MHz, CDCl₃): δ = 3.72 (t, *J* = 6.3,2H), 3.82 (d, *J*= 14.6, 1H), 3.83 (s, 3H), 4.02 (d, *J* = 14.6,1H), 4.76 (t, *J* = 6.3, 1H), 5.77-5.81 (m, 1H), 6.73 (dd, *J* = 7.4, *J* = 1.1,1H), 6.84-6.91 (m, 3H), 7.10-7.11 (m, 2H), 7.21-7.25 (m, 1H).

LC/MS (Methode 2): Rₜ = 2.29 min.; MS (ESI pos): m/z = 302 [M+H]⁺.

### Beispiel 22A

### Ethyl [8-chlor-1-(2-methoxyphenyl)-4-oxo-1,2,4,5-tetrahydro-3H-3-benzazepin-3-yl]acetat

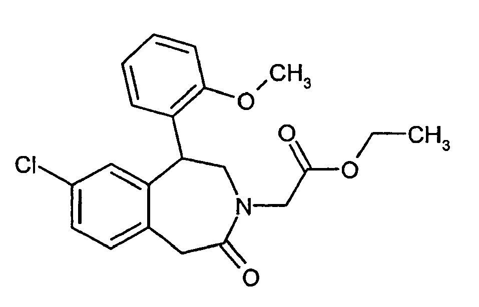

254 mg der Verbindung aus Beispiel 21A (0.84 mmol) werden in 5 ml DMF gelöst und mit 549 mg Cäsiumcarbonat (1.68 mmol) sowie 190 µl Bromessigsäureethylester (281 mg, 1.68 mmol) versetzt. Die Reaktionsmischung wird bei Raumtemperatur über Nacht gerührt. Es werden 6 ml Wasser zugegeben und dreimal mit Dichlormethan extrahiert. Die vereinigten organischen Phasen werden mit gesättigter Natriumchlorid-Lösung gewaschen und über Natriumsulfat getrocknet. Das Lösungsmittel wird am Rotationsverdampfer entfernt und der Rückstand mittels präparativer HPLC (Eluent: Acetonitril/Wasser, Gradient 20:80 → 95:5) gereinigt. Es werden 254 mg (29% d. Th.) der Titelverbindung erhalten.

¹H-NMR (400 MHz, CDCl₃): δ = 1.22 (t, *J* = 7.2, 3H), 3.40-3.46 (m, 1H), 3.79-4.05 (m, 3H), 3.83 (s, 3H), 4.83-4.86 (m, 1H), 6.71-6.73 (m, 1H), 6.84-6.91 (m, 3H), 7.11 (s, 2H), 7.22-7.24 (m, 1H).

LC/MS (Methode 2): Rₜ = 2.59 min.; MS (ESIpos): m/z = 388 [M+H]⁺.

### Beispiel 23A

### Ethyl [7-chlor-1-isobutyl-5-(2-methoxyphenyl)-2-oxo-1,2,4,5-tetrahydro-3H-3-benzazepin-3-yl]-acetat

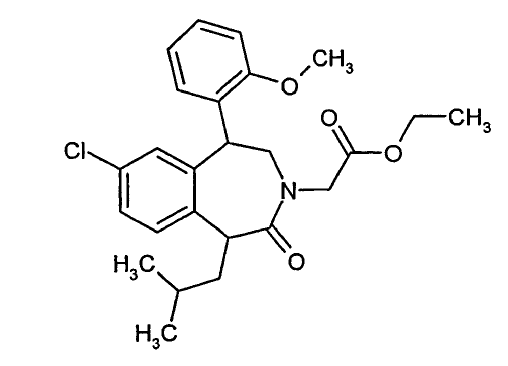

134 mg der Verbindung aus Beispiel 22A (0.35 mmol) werden in 1.35 ml THF gelöst. Bei -78°C werden 0.52 ml einer 1 M Lösung von 3-tert.-Butylimino-1,1,1,5,5,5-hexakis(dimethylamino)-3-(tris(dimethylamino)phosphoranyliden)amino-1λ⁵,3λ⁵,5λ⁵-1,4-triphosphazadien (P₄-*t*-Bu; 328 mg, 0.52 mmol) in *n*-Hexan zugetropft und die Reaktionslösung für 30 min. bei -78°C gerührt. Es werden 191 mg 1-Iod-2-methylpropan (1.04 mmol) zugetropft und weitere 2 h bei -78°C gerührt. Die Reaktionslösung wird mit 10 ml 1 N Salzsäure und Wasser versetzt und dreimal mit Dichlormethan extrahiert. Die vereinigten organischen Phasen werden mit gesättigter Natriumhydrogencarbonat-Lösung gewaschen, über Natriumsulfat getrocknet und am Rotationsverdampfer eingeengt. Der Rückstand wird mittels präparativer HPLC (Eluent: Acetonitril/Wasser, Gradient 20:80 → 95:5) gereinigt. Es werden 87 mg (57% d. Th.) der Titelverbindung erhalten.

¹H-NMR (400 MHz, CDCl₃): δ = 1.00 (d, *J* = 6.8, 3H), 1.03 (d, *J* = 7.2, 3H), 1.19 (t, *J* = 7.2, 3H), 1.61-1.85 (m, 2H), 2.23-2.32 (m, 1H), 3.68-4.28 (m, 4H), 3.81 (s, 3H), 4.12 (q, *J* = 7.2, 2H), 4.52-4.59 (m, 1H), 4.82 (dd, *J* = 10.2, *J* = 6.4, 1H), 6.82-6.95 (m, 4H), 7.10-7.27 (m, 3H).

LC/MS (Methode 2): Rₜ = 3.10 min.; MS (ESIpos): m/z = 444 [M+H]⁺.

### Beispiel 24A

### 5-Chlor-3-(2,3-dimethoxyphenyl)-1H-inden

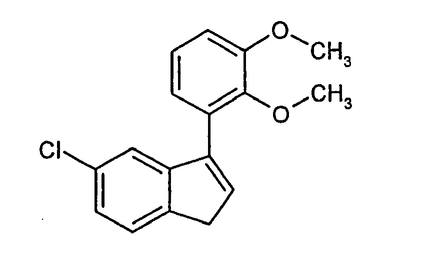

Unter Argonatmosphäre werden 45.9 ml Veratrol (49.78 g, 360.1 mmol) in 240 ml THF gelöst und bei -78°C mit 236.2 ml einer 1.6 M Lösung von *n*-Butyllithium in THF (377.7 mmol) tropfenweise versetzt. Das Reaktionsgemisch wird auf 0°C erwärmt und für 3 h bei dieser Temperatur gerührt. Das Gemisch wird auf -60°C abgekühlt und 30.0 g der Verbindung aus Beispiel 16A (180.1 mmol), gelöst in 150 ml THF, zugetropft. Innerhalb von 3 h wird das Reaktionsgemisch auf Raumtemperatur erwärmt, dann mit 1 N Salzsäure angesäuert und mit Dichlormethan extrahiert. Die vereinigten organischen Phasen werden über Magnesiumsulfat getrocknet und das Lösungsmittel am Rotationsverdampfer entfernt. Der Rückstand wird durch Filtration über Kieselgel (Laufmittel: Cyclohexan/Essigsäureethylester 5:1) aufgereinigt. Das Rohprodukt [6-Chlor-1-(2,3-dimethoxyphenyl)indan-1-ol] wird in 11 Dichlormethan gelöst, mit 75 mg 4-Toluolsulfonsäure-Monohydrat (0.39 mmol) versetzt und für 16 h bei RT gerührt. Die Reaktionslösung wird mit gesättigter Natriumhydrogencarbonat-Lösung sowie mit gesättigter Natriumchlorid-Lösung gewaschen, über Natriumsulfat getrocknet und am Rotationsverdampfer eingeengt. Der Rückstand wird über Chromatographie an Kieselgel (Laufmittel: Cyclohexan/Essigsäureethylester 20:1) gereinigt. Es werden 10.5 g (20% d. Th.) der Titelverbindung erhalten.

¹H-NMR (400 MHz, CDCl₃): δ = 3.51 (d, *J* = 1.8, 2H), 3.63 (s, 3H), 3.93 (s, 3H), 6.66 (t, *J* = 1.8, 1H), 6.95-6.97 (m, 2H), 7.12 (dd, *J* = 8.3, *J* = 7.5, 1H), 7.19 (dd, *J* = 7.9, *J* = 2.0, 1H), 7.34 (d, *J* = 1.8, 1H), 7.41 (d, *J* = 7.9, 1H).

LC/MS (Methode 2): Rₜ = 3.00 min.; MS (ESIpos): m/z = 287 [M+H]⁺.

### Beispiel 25A

### [4-Chlor-2-(2,3-dimethoxybenzyl)phenyl]essigsäure

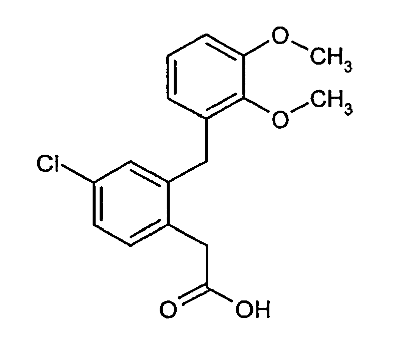

Es werden 701.6 mg Natriumperiodat (3.28 mmol) und 3.6 mg Ruthenium(III)chlorid-Monohydrat (0.02 mmol) in 3 ml Wasser vorgelegt. Es werden 229.4 mg der Verbindung aus Beispiel 24A (0.80 mmol), gelöst in 2 ml Tetrachlorkohlenstoff, zugegeben, 2 ml Acetonitril hinzugefügt und die Reaktionsmischung bei Raumtemperatur für 16 h gerührt. Zur Aufarbeitung wird das Gemisch auf 20 ml einer 5%-igen wässrigen Trifluoressigsäure-Lösung gegeben und dreimal mit Dichlormethan extrahiert. Die vereinigten organischen Phasen werden über Natriumsulfat getrocknet und das Lösungsmittel am Rotationsverdampfer entfernt. Der Rückstand wird mit 5 ml Essigsäureethylester sowie 2 ml *n*-Heptan verrührt und für 3 h bei 5°C gelagert. Das abgeschiedene Produkt wird abfiltriert, mit *n*-Pentan gewaschen und im Hochvakuum getrocknet. Es werden 119.0 mg (44% d. Th.) der Titelverbindung erhalten.

¹H-NMR (400 MHz, CDCl₃): δ = 3.61 (s, 3H), 3.90 (s, 5H), 7.05 (dd, J= 6.4, *J* = 2.8, 1H), 7.11-7.15 (m, 2H), 7.35-7.39 (m, 2H), 7.46 (dd, *J* = 8.3, J= 2.1,1H).

LC/MS (Methode 2): Rₜ = 2.19 min.; MS (ESIpos): m/z = 335 [M+H]⁺.

### Beispiel 26A

### 7-Chlor-1-(2,3-dimethoxyphenyl)-1,4-dihydro-3H-isochroman-3-on

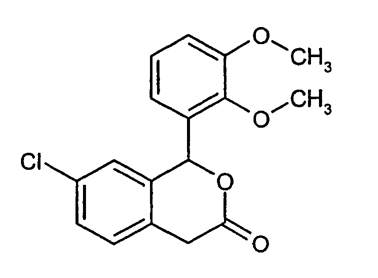

1.607 g der Verbindung aus Beispiel 25A (4.80 mmol) werden in 48 ml Ethanol gelöst. Es werden 0.182 g Natriumborhydrid (4.80 mmol) zugegeben und das Reaktionsgemisch für 1 h unter Rückfluss erhitzt. Nach dem Abkühlen werden 120 ml 10%-iger Salzsäure hinzugefügt und das Gemisch am Rotationsverdampfer eingeengt, bis nur eine wässrige Phase zurückbleibt. Das Produkt scheidet sich dabei ab. Durch Abkühlen des Gemisches auf Raumtemperatur wird die Fällung vervollständigt. Der Niederschlag wird abfiltriert und im Hochvakuum getrocknet. Es werden 1.319 g (86% d. Th.) der Titelverbindung erhalten.

¹H-NMR (400 MHz CDCl₃): δ = 3.81 (s, 2H), 3.87 (s, 3H), 3.93 (s, 3H), 6.71 (s, 1H), 6.78-6.81 (m, 2H), 7.00 (dd, *J* = 8.3, J = 1.5, 1H), 7.11 (t, *J* = 7.9, 1H), 7.19 (d, *J* = 8.1, 1H), 7.31 (dd, *J* = 8.0, *J* = 2.0, 1H).

LC/MS (Methode 1): Rₜ = 2.28 min.; MS (ESIpos): m/z = 319 [M+H]⁺.

### Beispiel 27A

### Methyl {4-chlor-2-[cyano(2,3-dimethoxyphenyl)methyl]phenyl}acetat

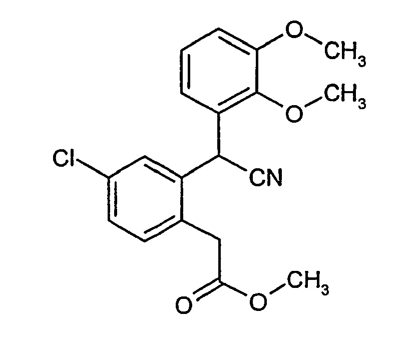

Unter Argonatmosphäre werden 1.478 g mg der Verbindung aus Beispiel 26A (4.64 mmol) in 35 ml Dichlormethan gelöst und bei 0°C mit 552 mg Trimethylsilylcyanid (5.56 mmol) sowie 59 mg Iod (0.23 mmol) versetzt. Bei 0°C werden 46 mg Iodtrimethylsilan (0.23 mmol) zugetropft und das Reaktionsgemisch 16 h bei Raumtemperatur gerührt. Es werden weitere 139 mg Iodtrimethylsilan (0.69 mmol) hinzugefügt und für weitere 6 h bei Raumtemperatur gerührt. Die Reaktionsmischung wird mit 20 ml Methanol versetzt, 15 min. bei Raumtemperatur gerührt und dann am Rotationsverdampfer eingeengt. Der Rückstand wird in 30 ml Methanol gelöst, mit 151 mg Chlortrimethylsilan (1.39 mmol) versetzt und für 16 h bei Raumtemperatur gerührt. Die Reaktionsmischung wird am Rotationsverdampfer eingeengt und der Rückstand über Chromatographie an Kieselgel (Laufmittel: Cyclohexan/Essigsäureethylester 3:1) gereinigt. Es werden 902 mg (54% d. Th.) der Titelverbindung erhalten.

¹H-NMR (400 MHz, CDCl₃): δ = 3.56 (d, *J* = 15.8), 3.65 (s, 3H), 3.68 (d, *J* = 15.8, 1H), 3.73 (s, 3H), 3.87 (s, 3H), 5.76 (s, 1H), 6.87-6.89 (m, 1H), 6.92-6.94 (m, 1H), 7.07 (t, *J* = 8.1, 1H), 7.20 (d, *J* = 8.3, 1H), 7.28 (dd, *J* = 8.2, *J* = 2.1, 1H), 7.47 (d, *J* = 2.2, 1H).

LC/MS (Methode 2): Rₜ = 2.65 min.; MS (ESIpos): m/z = 360 [M+H]⁺.

### Beispiel 28A

### 7-Chlor-5-(2,3-dimethoxyphenyl)-1,3,4,5-tetrahydro-2H-3-benzazepin-2-on

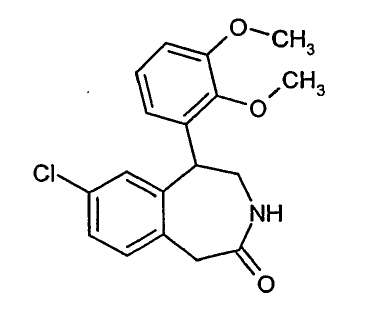

118 mg der Verbindung aus Beispiel 27A (0.33 mmol) werden in 2.3 ml Methanol gelöst und mit 156 mg Cobalt[II]chlorid-Hexahydrat (0.66 mmol) versetzt. Unter Rühren werden bei 0°C innerhalb von 10 min. portionsweise 133 mg Natriumborhydrid (3.51 mmol) zugegeben. Die Reaktionsmischung wird 30 min. bei 0°C sowie 1 h bei Raumtemperatur gerührt. Zur Aufarbeitung werden 7 ml 1 N Salzsäure zugegeben und die Mischung gerührt, bis eine homogene Lösung entsteht. Die Lösung wird mit konzentrierter Ammoniaklösung basisch gestellt und mit Dichlormethan extrahiert. Die vereinigten organischen Phasen werden über Natriumsulfat getrocknet und am Rotationsverdampfer eingeengt. Der Rückstand wird in 10 ml Toluol gelöst und für 16 h unter Rückfluss erhitzt. Das Lösungsmittel wird am Rotationsverdampfer entfernt und der Rückstand mittels präparativer HPLC (Eluent: Acetonitril/Wasser, Gradient 20:80 → 95:5) gereinigt. Es werden 42 mg (40% d. Th.) der Titelverbindung erhalten.

¹H-NMR (400 MHz, CDCl₃): δ = 3.53-3.62 (m, 1H), 3.56 (s, 3H), 3.68 (d, *J* = 14.5, 1H), 3.85-3.94 (m, 1H), 3.88 (s, 3H), 4.20 (d, *J* = 14.5, 1H), 4.55 (dd, *J* = 9.3, J = 4.7, 1H), 5.82-5.86 (m, 1H), 6.50-6.53 (m, 1H), 6.85 (dd, *J* = 8.2, *J* = 1.2, 1H), 6.94 (s, 1H), 6.99 (t, *J* = 7.9, 1H), 7.11-7.12 (m, 2H).

LC/MS (Methode 1): Rₜ = 2.03 min.; MS (ESIpos): m/z = 332 [M+H]⁺.

### Beispiel 29A

### Ethyl [8-chlor-1-(2,3-dimethoxyphenyl)-4-oxo-1,2,4,5-tetrahydro-3H-3-benzazepin-3-yl]acetat

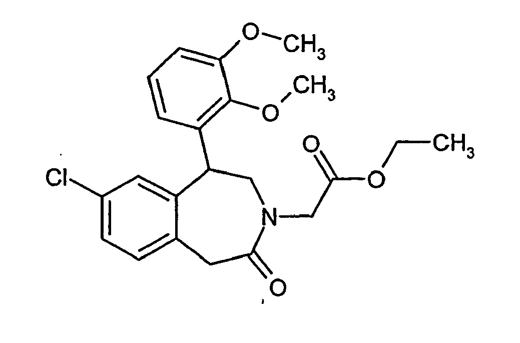

451 mg der Verbindung aus Beispiel 28A (1.36 mmol) werden in 8 ml DMF gelöst und mit 885 mg Cäsiumcarbonat (2.72 mmol) sowie 300 µl Bromessigsäureethylester (454 mg, 2.72 mmol) versetzt. Die Reaktionsmischung wird bei Raumtemperatur über Nacht gerührt. Es werden 10 ml Wasser zugegeben und dreimal mit Dichlormethan extrahiert. Die vereinigten organischen Phasen werden mit gesättigter Natriumchlorid-Lösung gewaschen und über Natriumsulfat getrocknet. Das Lösungsmittel wird am Rotationsverdampfer entfernt und der Rückstand mittels präparativer HPLC (Eluent: Acetonitril/Wasser, Gradient 20:80 → 95:5) gereinigt. Es werden 418 mg (30% d. Th.) der Titelverbindung erhalten.

¹H-NMR (400 MHz, CDCl₃): δ = 1.21 (t, *J* = 7.1, 3H), 3.56-3.80 (m, 6H), 3.88 (s, 3H), 4.10-4.35 (m, 5H), 4.69-4.73 (m, 1H), 6.47-6.50 (m, 1H), 6.85 (dd, *J* = 8.2, *J* = 1.4, 1H), 6.94-7.01 (m, 2H), 7.08-7.14 (m, 2H).

LC/MS (Methode 2): Rₜ = 2.56 min.; MS (ESIpos): m/z = 418 [M+H]⁺.

### Beispiel 30A

### Ethyl [7-chlor-5-(2,3-dimethoxyphenyl)-1-isobutyl-2-oxo-1,2,4,5-tetrahydro-3H-3-benzazepin-3-yl]acetat

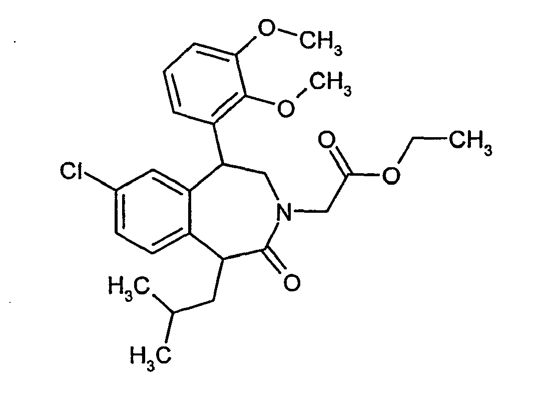

94 mg der Verbindung aus Beispiel 29A (0.22 mmol) werden in 0.90 ml THF gelöst. Bei -78°C werden 0.34 ml einer 1 M Lösung von 3-*tert*.-Butylimino-1,1,1,5,5,5-hexakis(dimethylamino)-3-(tris(dimethylamino)phosphoranyliden)amino-1λ⁵,3λ⁵,5λ⁵-1,4-triphosphazadien (P₄-*t*-Bu; 213 mg, 0.34 mmol) in *n*-Hexan zugetropft und die Reaktionslösung für 30 min. bei -78°C gerührt. Es werden 124 mg 1-Iod-2-methylpropan (0.67 mmol) zugetropft und weitere 2 h bei -78°C gerührt. Die Reaktionslösung wird mit 1 N Salzsäure und Wasser versetzt und dreimal mit Dichlormethan extrahiert. Die vereinigten organischen Phasen werden mit gesättigter Natriumhydrogencarbonat-Lösung gewaschen, über Natriumsulfat getrocknet und am Rotationsverdampfer eingeengt. Der Rückstand wird mittels präparativer HPLC (Eluent: Acetonitril/Wasser, Gradient 20:80 → 95:5) gereinigt. Es werden 53 mg (50% d. Th.) der Titelverbindung erhalten.

¹H-NMR (400 MHz, CDCl₃): δ = 1.00 (d, *J* = 6.6, 3H), 1.04 (d, *J* = 6.4, 3H), 1.18 (t, *J* = 7.1, 3H), 1.61-1.68 (m, 1H), 1.72-1.82 (m, 1H), 2.26-2.33 (m, 1H), 3.45-4.75 (m, 8H), 3.87 (s, 3H), 4.11 (q, J = 7.1, 2H), 4.21 (d, *J* = 17.4, 1H), 6.60-6.65 (m, 1H), 6.86 (d, *J* = 8.1, 1H), 6.97-7.02 (m, 2H), 7.10-7.13 (m, 1H), 7.19 (d, *J* = 8.3,1H).

LC/MS (Methode 3): Rₜ = 3.12 min.; MS (ESIpos): m/z = 474 [M+H]⁺.

### Beispiel 31A

### 1-[(7-Chlor-1-isobutyl-2-oxo-5-{2-[(trimethylsilyl)ethynyl]phenyl}-1,2,4,5-tetrahydro-3H-3-benzazepin-3-yl)acetyl]piperidin-4-carbonsäureethylester

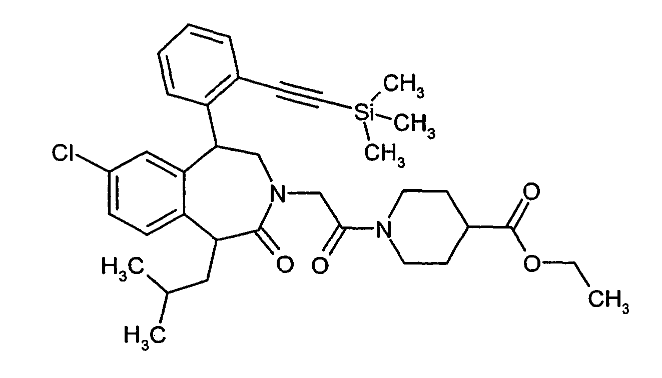

100 mg der Verbindung aus Beispiel 22 (0.166 mmol) werden in 6 ml DMF/Triethylamin (5:1) gelöst. Es werden 12 mg Bis(triphenylphosphin)palladium(II)chlorid (0.017 mmol), 9 mg Kupfer(I)iodid (0.05 mmol) sowie 183 mg Tetra-n-butylammoniumiodid (0.497 mmol) hinzugefügt und die Reaktionsmischung 5 min. bei Raumtemperatur gerührt. Dann werden 94 µl Trimethylsilylacetylen (65 mg, 0.662 mmol) zugegeben und das Gemisch 16 h bei 85°C gerührt. Es werden erneut 12 mg Bis(triphenylphosphin)palladium(II)chlorid (0.017 mmol), 9 mg Kupfer(I)-iodid (0.05 mmol) sowie 188 µl Trimethylsilylacetylen (130 mg, 1.324 mmol) zugegeben und das Gemisch für weitere 16 h bei 85°C gerührt. Weitere 282 µl Trimethylsilylacetylen (195 mg, 1.986 mmol) werden zugegeben und das Gemisch erneut für 16 h bei 85°C gerührt. Zur Aufarbeitung wird das Reaktionsgemisch mit Dichlormethan versetzt, mit 1 N Salzsäure gewaschen, über Celite filtriert und das Filtrat am Rotationsverdampfer eingeengt. Der Rückstand wird mittels präparativer HPLC (Eluent: Acetonitril/Wasser, Gradient 20:80 → 95:5) gereinigt. Es werden 13 mg (12% d. Th.) der Titelverbindung erhalten.

¹H-NMR (400 MHz, CDCl₃): δ = 0.29 (s, 9H), 0.99-1.03 (m, 6H), 1.25 (t, *J* = 7.1, 3H), 1.56-1.93 (m, 6H), 2.21-2.27 (m, 1H), 2.45-2.51 (m, 1H), 2.77-3.06 (m, 2H), 3.54-4.32 (m, 6H), 4.14 (t, *J* = 7.1, 2H), 4.42-4.49 (m, 1H), 5.07-5.13 (m, 1H), 6.75 (br. s, 1H), 6.98 (s, 1H), 7.15-7.23 (m, 4H), 7.50-7.52 (m, 1H).

LC/MS (Methode 1): Rₜ = 3.27 min.; MS (ESIpos): m/z = 621 [M+H]⁺.

### Ausführungsbeispiele:

### Beispiel 1

### [7-Chlor-5-(2-chlorphenyl)-1-(2-methylallyl)-2-oxo-1,2,4,5-tetrahydrobenzo[d]azepin-3-yl]-essigsäure

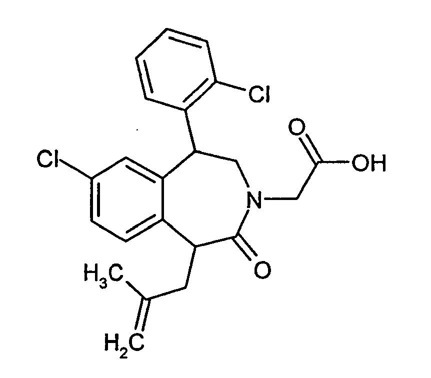

21 mg der Verbindung aus Beispiel 5A (0.05 mmol) werden in 1 ml Dioxan/Wasser (1:1) gelöst und mit 70 µl 1M Natronlauge versetzt. Die Reaktionsmischung wird 24 h bei Raumtemperatur gerührt. Es werden 5 ml 1 M Salzsäure zugegeben und die Mischung dreimal mit Dichlormethan extrahiert. Die vereinigten organischen Phasen werden über Natriumsulfat getrocknet und am Rotationsverdampfer eingeengt. Der Rückstand wird mittels präparativer HPLC (Eluent: Acetonitril/Wasser mit 0.1% Ameisensäure, Gradient 20:80 → 95:5) gereinigt. Es werden 11 mg (54% d. Th.) der Titelverbindung erhalten.

¹H-NMR (400 MHz, CDCl₃): δ = 2.69 (dd, *J* = 16.0 und 6.5, 1H), 2.95 (dd, *J* = 16.0 und 8.2, 1H), 3.78-4.06 (m, 2H), 4.09-4.29 (m, 2H), 4.64 (d, *J* = 7.5, 1H), 4.71 (s, 1H), 4.87 (s, 1H), 5.07 (dd, 10.1 und 6.1, 1H), 6.79-6.85 (m, 1H), 6.93 (s, 1H), 7.14-7.27 (m, 4H), 7.41.-7.46 (m, 1H).

LC/MS (Methode 3): Rₜ = 2.68 min.; MS (ES1pos): m/z = 418 [M+H]⁺.

### Beispiel 2

### [7-Chlor-5-(2-chlorphenyl)-1-isobutyl-2-oxo-1,2,4,5-tetrahydrobenzo[d]azepin-3-yl]-essigsäure

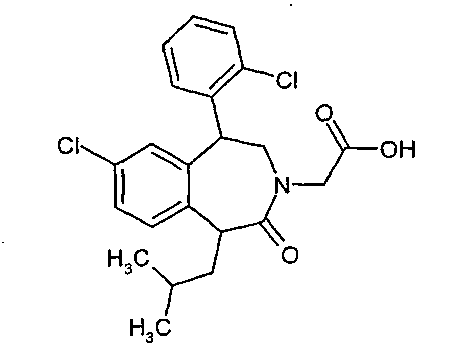

62 mg der Verbindung aus Beispiel 6A (0.14 mmol) werden in 1 ml Dioxan/Wasser (1:1) gelöst und mit 210 µl 1 M Natronlauge versetzt. Die Reaktionsmischung wird über Nacht bei Raumtemperatur gerührt. Es werden 5 ml 1 M Salzsäure zugegeben und die Mischung dreimal mit Dichlormethan extrahiert. Die vereinigten organischen Phasen werden über Natriumsulfat getrocknet und am Rotationsverdampfer eingeengt. Der Rückstand wird mittels präparativer HPLC (Eluent: Acetonitril/Wasser mit 0.1 % Ameisensäure, Gradient 20:80 → 95:5) gereinigt. Es werden 49 mg (83% d. Th.) der Titelverbindung erhalten.

LC/MS (Methode 1): Rₜ = 2.65 min.; MS (ESIpos): m/z = 420 [M+H]⁺.

### Beispiel 3

### (1-{2-[7-Chlor-5-(2-chlorphenyl)-1-isobutyl-2-oxo-1,2,4,5-tetrahydrobenzo[d]azepin-3-yl]-acetyl}-piperidin-4-yl)-essigsäureethylester

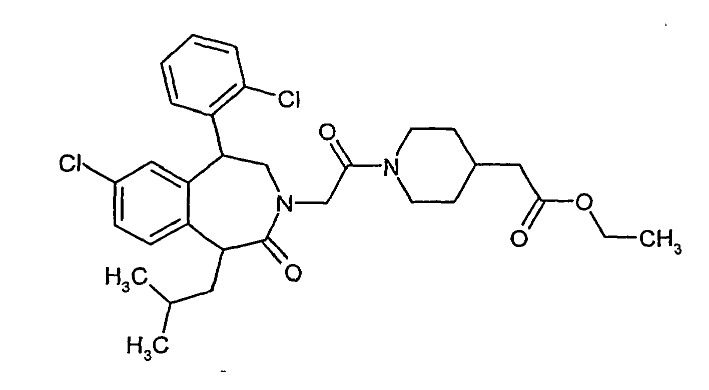

47 mg der Verbindung aus Beispiel 2 (0.11 mmol) werden in 2 ml Dichlormethan gelöst. Es werden 35 mg 4-Piperidylessigsäureethylester-Hydrochlorid (0.17 mmol), 18 mg 1-Hydroxy-1*H-*benzotriazol-Hydrat (0.13 mmol), 26 mg 1-Ethyl-3-(3-dimethylaminopropyl)carbodiimid-Hydrochlorid (0.13 mmol) und 22 mg *N,N-*Düsopropylethylamin (0.17 mmol) hinzugegeben. Die Reaktionsmischung wird bei Raumtemperatur über Nacht gerührt, dann am Rotationsverdampfer eingeengt und der Rückstand mittels präparativer HPLC (Eluent: Acetonitril/Wasser mit 0.1% Ameisensäure, Gradient 20:80 → 95:5) gereinigt. Es werden 34 mg (53% d. Th.) der Titelverbindung erhalten.

LC/MS (Methode 1): Rₜ = 3.04 min.; MS (ES1pos): m/z = 573 [M+H]⁺.

### Beispiel 4

### (1-{2-[7-Chlor-5-(2-chlorphenyl)-1-isobutyl-2-oxo-1,2,4,5-tetrahydrobenzo[d]azepin-3-yl]-acetyl}-piperidin-4-yl)-essigsäure

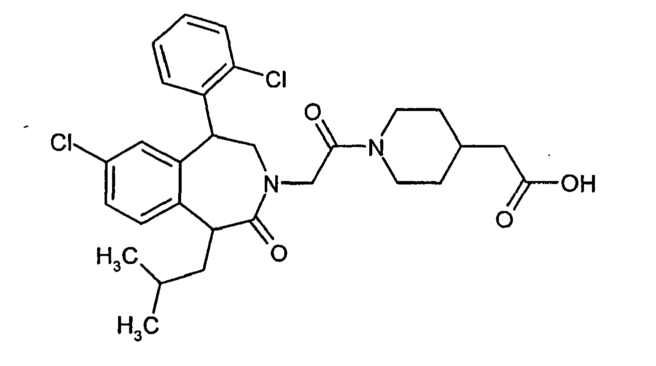

30 mg der Verbindung aus Beispiel 3 (0.05 mmol) werden in 1 ml Dioxan/Wasser (1:1) gelöst. Es werden 80 µl 1 M Natronlauge zugegeben und die Reaktionsmischung bei Raumtemperatur über Nacht gerührt. Es wird mit 1 M Salzsäure auf pH 1 angesäuert und dreimal mit Dichlormethan extrahiert. Die vereinigten organischen Phasen werden über Natriumsulfat getrocknet und das Lösungsmittel am Rotationsverdampfer entfernt. Es werden 25 mg (100% d. Th.) der Titelverbindung erhalten.

LC/MS (Methode 1): Rₜ = 2.76 min.; MS (ESIpos): m/z = 545 [M+H]⁺.

Durch präparative HPLC an chiraler Phase werden die Enantiomeren getrennt (Daicel Chiralpak AD-H 5 µm, Säule 250 x 20 mm; Eluent: *iso-*Hexan/Ethanol 65:35; Fluss: 20 ml/min; Detektion: UV 220 nm):

### Enantiomer 4-1:

Rₜ = 10.2 min.

### Enantiomer 4-2:

Rₜ = 24.0 min.

### Beispiel 5

### [5-(2-Chlorphenyl)-1-isobutyl-7-methyl-2-oxo-1,2,4,5-tetrahydrobenzo[d]azepin-3-yl]-essigsäure

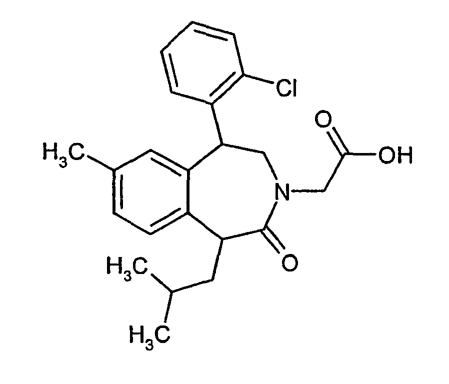

385 mg der Verbindung aus Beispiel 13A (0.90 mmol) werden in 12 ml Dioxan/Wasser (1:1) gelöst und mit 3 ml 1 N Natronlauge versetzt. Die Reaktionsmischung wird 3 h bei Raumtemperatur gerührt. Es wird mit Wasser verdünnt, mit 2 N Salzsäure angesäuert und die Mischung dreimal mit Dichlormethan extrahiert. Die vereinigten organischen Phasen werden über Natriumsulfat getrocknet und am Rotationsverdampfer eingeengt. Es werden 355 mg (99% d. Th.) der Titelverbindung erhalten.

¹H-NMR (400 MHz, CDCl₃): δ = 1.00 (d, *J* = 6.2, 3H), 1.01 (d, *J* = 6.1, 3H), 1.70-1.83 (m, 2H), 2.15-2.24 (m, 1H), 2.19 (s, 3H), 3.96-4.14 (m, 3H), 4.34-4.41 (m, 1H), 4.98-5.01 (m, 1H), 6.71-6.77 (m, 2H), 7.01-7.04 (m, 1H), 7.11-7.21 (m, 3H), 7.40-7.43 (m, 1 H).

HPLC (Methode 6): Rₜ = 5.11 min.

### Beispiel 6

### [5-(2-Bromphenyl)-7-chlor-1-isobutyl-2-oxo-1,2,4,5-tetrahydrobenzo[d]azepin-3-yl]-essigsäure

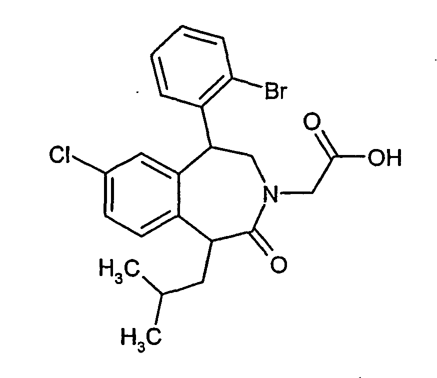

185 mg der Verbindung aus Beispiel 14A (0.38 mmol) werden in 6 ml THF/Methanol (1:1) gelöst und mit 1 ml 1 N Natronlauge versetzt. Die Reaktionsmischung wird 16 h bei Raumtemperatur gerührt. Es wird mit Wasser verdünnt, mit 2 N Salzsäure angesäuert und die Mischung dreimal mit Dichlormethan extrahiert. Die vereinigten organischen Phasen werden über Natriumsulfat getrocknet und am Rotationsverdampfer eingeengt. Es werden 172 mg (99% d. Th.) der Titelverbindung erhalten.

¹H-NMR (400 MHz, CDCl₃): δ = 1.00 (d, *J* = 6.4, 3H), 1.01 (d, *J* = 6.4, 3H), 1.67-1.82 (m, 2H), 2.19-2.26 (m, 1H), 3.95-4.09 (m, 2H), 4.18 (d, *J* = 17.4, 1H), 4.38-4.46 (m, 1H), 5.02 (t, *J* = 7.1, 1H), 6.73-6.79 (m, 1H), 6.91 (s, 1H), 7.10-7.23 (m, 4H), 7.61 (dd, *J* = 7.9, J= 1.1, 1H).

HPLC (Methode 6): Rₜ = 5.21 min.; MS (CI): m/z = 464 [M+H]⁺.

### Beispiel 7

### (1-{2-[5-(2-Chlorphenyl)-1-isobutyl-7-methyl-2-oxo-1,2,4,5-tetrahydrobenzo[d]azepin-3-yl]-acetyl}-piperidin-4-yl)-essigsäureethylester

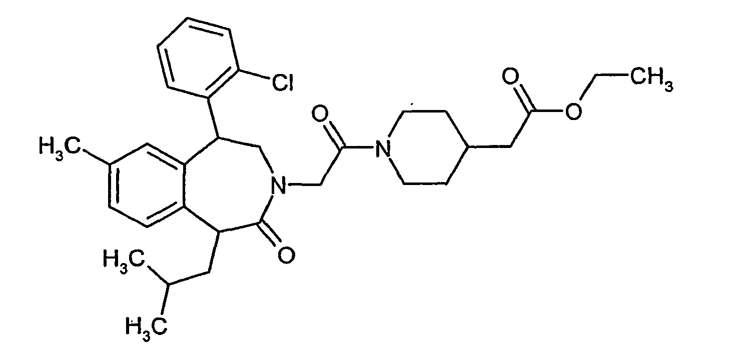

340 mg der Verbindung aus Beispiel 5 (0.85 mmol) werden in 5 ml Dimethylformamid gelöst und mit 388 mg *O*-(7-Azabenzotriazol-1-yl)-*N*,*N*,*N*',*N*'-tetramethyluronium-Hexafluorophosphat (HATU) (1.02 mmol) sowie 177 µl Diisopropylethylamin (132 mg, 1.02 mmol) versetzt. Nach 30 min. Rühren bei Raumtemperatur werden 212 mg 4-Piperidylessigsäureethylester-Hydrochlorid (1.02 mmol) und weitere 353 µl Diisopropylethylamin (264 mg, 2.04 mmol) hinzugegeben. Die Reaktionsmischung wird bei Raumtemperatur über Nacht gerührt und dann direkt mittels präparativer HPLC (Eluent: Acetonitril/Wasser mit 0.1 % Ameisensäure, Gradient 20:80 → 95:5) aufgereinigt. Es werden 300 mg (64% d. Th.) der Titelverbindung erhalten.

HPLC (Methode 6): Rₜ = 5.54 min.; MS (ESIpos): m/z = 553 [M+H]⁺.

### Beispiel 8

### (1-{2-[5-(2-Bromphenyl)-7-chlor-1-isobutyl-2-oxo-1,2,4,5-tetrahydrobenzo[d]azepin-3-yl]-acetyl}-piperidin-4-yl)-essigsäureethylester

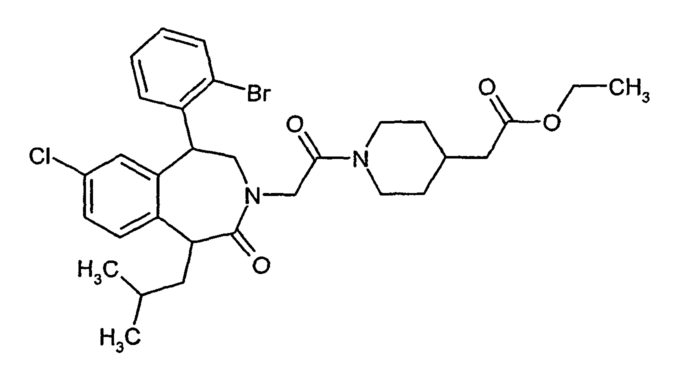

150 mg der Verbindung aus Beispiel 6 (0.32 mmol) werden in 2 ml Dimethylformamid gelöst und mit 147 mg *O*-(7-Azabenzotriazol-1-yl)-*N*,*N*,*N*',*N*'-tetramethyluronium-Hexafluorophosphat (HATU) (0.39 mmol) sowie 67 µl Diisopropylethylamin (50 mg, 0.39 mmol) versetzt. Nach 30 min. Rühren bei Raumtemperatur werden 80 mg 4-Piperidylessigsäureethylester-Hydrochlorid (0.39 mmol) und weitere 135 µl Diisopropylethylamin (100 mg, 0.77 mmol) hinzugegeben. Die Reaktionsmischung wird bei Raumtemperatur über Nacht gerührt und dann direkt mittels präparativer HPLC (Eluent: Acetonitril/Wasser mit 0.1% Ameisensäure, Gradient 20:80 → 95:5) aufgereinigt. Es werden 130 mg (65% d. Th.) der Titelverbindung erhalten.

LC/MS (Methode 3): Rₜ = 3.24 min.; MS (ESIpos): m/z = 618 [M+H]⁺.

### Beispiel 9

### (1-{2-[5-(2-Chlorphenyl)-1-isobutyl-7-methyl-2-oxo-1,2,4,5-tetrahydrobenzo[d]azepin-3-yl]-acetyl}-piperidin-4-yl)-essigsäure

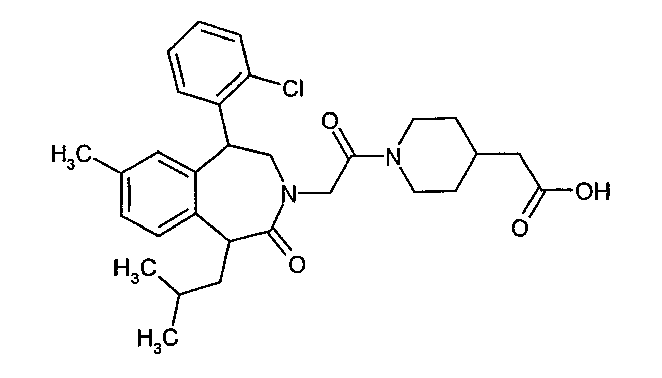

300 mg der Verbindung aus Beispiel 7 (0.54 mmol) werden in 6 ml Methanol gelöst. Es werden 1.5 ml 2 N Natronlauge zugegeben und die Reaktionsmischung 3 h bei Raumtemperatur gerührt. Es wird mit Wasser verdünnt, mit 1 N Salzsäure auf pH 1 angesäuert und dreimal mit Dichlormethan extrahiert. Die vereinigten organischen Phasen werden über Natriumsulfat getrocknet und das Lösungsmittel am Rotationsverdampfer entfernt. Der Rückstand wird mittels präparativer HPLC (Eluent: Acetonitril/Wasser mit 0.1% Ameisensäure, Gradient 20:80 → 95:5) aufgereinigt. Es werden 200 mg (70% d. Th.) der Titelverbindung erhalten.

HPLC (Methode 6): Rₜ = 5.03 min.; MS (ESIpos): m/z = 525 [M+H]⁺.

### Beispiel 10

### (1-{2-[5-(2-Bromphenyl)-7-chlor-1-isobutyl-2-oxo-1,2,4,5-tetrahydrobenzo[d] azepin-3-yl]-acetyl}-piperidin-4-yl)-essigsäure

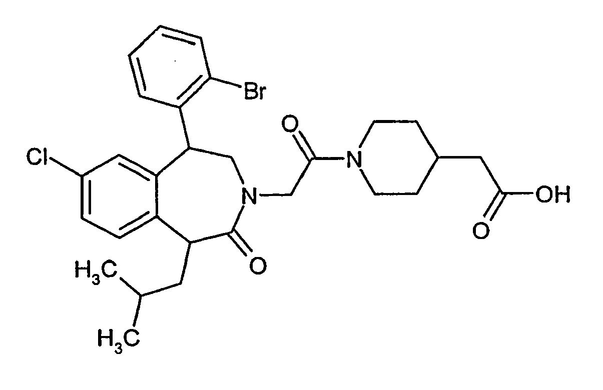

110 mg der Verbindung aus Beispiel 8 (0.18 mmol) werden in 6 ml Dioxan/Methanol (1:1) gelöst. Es werden 1.0 ml 1N Natronlauge zugegeben und die Reaktionsmischung 3 h bei Raumtemperatur gerührt. Es wird mit Wasser verdünnt, mit 2 N Salzsäure angesäuert und dreimal mit Dichlormethan extrahiert. Die vereinigten organischen Phasen werden über Natriumsulfat getrocknet und das Lösungsmittel am Rotationsverdampfer entfernt. Es werden 105 mg (100% d. Th.) der Titelverbindung erhalten.

HPLC (Methode 6): Rₜ = 5.17 min.

### Beispiel 11

### (1-{2-[5-(2-Chlorphenyl)-7-chlor-1-isobutyl-2-oxo-1,2,4,5-tetrahydrobenzo[d]azepin-3-yl]-acetyl}-piperidin-4-yl)-carbonsäareethylester

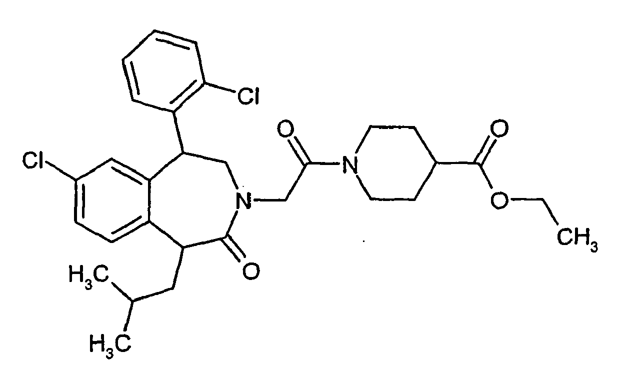

45 mg der Verbindung aus Beispiel 2 (0.11 mmol) und 18 mg Piperidin-4-carbonsäureethylester (0.11 mmol) werden in 2 ml Dimethylformamid gelöst und mit 21 mg Cyanophosphonsäurediethylester (0.12 mmol) sowie 22 µl Triethylamin (16 mg, 0.16 mmol) versetzt. Die Reaktionsmischung wird bei Raumtemperatur über Nacht gerührt, dann mit Essigsäureethylester versetzt und mit 5%-iger Kaliumhydrogensulfat-Lösung, gesättigter Natriumhydrogencarbonat-Lösung sowie gesättigter Natriumchlorid-Lösung gewaschen. Die organische Phase wird über Natriumsulfat getrocknet, das Lösungsmittel am Rotationsverdampfer entfernt und der Rückstand mittels präparativer HPLC (Eluent: Acetonitril/Wasser mit 0.1 % Ameisensäure, Gradient 20:80 → 95:5) aufgereinigt. Es werden 22 mg (36% d. Th.) der Titelverbindung erhalten.

¹H-NMR (400 MHz, CDCl₃): δ = 0.99 (d, *J* = 6.2, 3H), 1.02 (d, *J* = 6.4, 3H), 1.25 (t, *J* = 7.1, 3H), 1.46-1.91 (m, 6H), 2.20-2.27 (m, 1H), 2.41-2.51 (m, 1H), 2.79-3.06 (m, 2H), 3.56-3.68 (m, 1H), 3.87-4.01 (m, 1H), 4.06-4.17 (m, 1H), 4.14 (t, *J* = 7.1, 3H), 4.22-4.36 (m, 2H), 4.46-4.54 (m, 1H), 4.99-5.05 (m, 1H), 6.83-6.87 (m, 1H), 6.91-6.92 (m, 1H), 7.14-7.22 (m, 4H), 7.40-7.42 (m, 1H).

LC/MS (Methode 3): Rₜ = 3.15 min.; MS (ESIpos): m/z = 559 [M+H]⁺.

### Beispiel 12

### (1-{2-[5-(2-Chlorphenyl)-7-chlor-1-isobutyl-2-oxo-1,2,4,5-tetrahydrobenzo[d]azepin-3-yl]-acetyl}-piperidin-4-yl)-carbonsäure

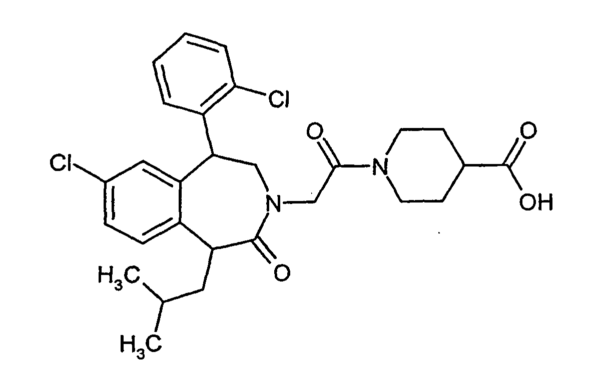

18 mg der Verbindung aus Beispiel 11 (0.11 mmol) werden in Dioxan/Wasser (2:1) gelöst, mit 50 µl 1 N Natronlauge versetzt und über Nacht bei Raumtemperatur gerührt. Die Reaktionslösung wird mit 1 N Salzsäure angesäuert (pH 2) und 20 min. gerührt, wobei das Produkt als Niederschlag ausfällt. Der Niederschlag wird abfiltriert und im Hochvakuum getrocknet. Es werden 11.7 mg (65% d. Th.) der Titelverbindung erhalten.

¹H-NMR (400 MHz, CDCl₃): δ = 0.99 (d, *J* = 6.1, 3H), 1.02 (d, *J* = 6.4, 3H), 1.54-1.97 (m, 6H), 2.20-2.27 (m, 1H), 2.48-2.56 (m, 1H), 2.80-2.89 (m, 1H), 2.93-3.05 (m, 1H), 3.58-3.70 (m, 1H), 3.85-4.53 (m, 6H), 5.00-5.05 (m, 1H), 6.82-6.89 (m, 1H), 6.92 (s, 1H), 7.15-7.22 (m, 4H), 7.39-7.42 (m, 1H).

LC/MS (Methode 2): Rₜ = 2.70 min.; MS (ESIpos): m/z = 531 [M+H]⁺.

### Beispiel 13

### 4-{2-[7-Chlor-5-(2-chlorphenyl)-1-isobutyl-2-oxo-1,2,4,5-tetrahydrobenzo[d]azepin-3-yl]-acetylamino}-hexansäuremethylester

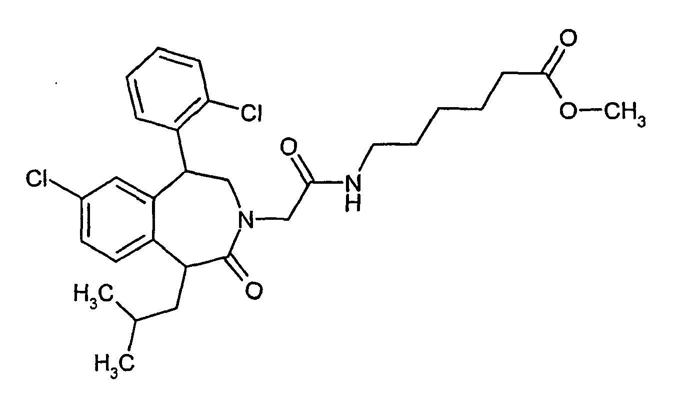

45 mg der Verbindung aus Beispiel 2 (0.11 mmol) und 20 mg 6-Aminohexansäuremethylester-Hydrochlorid (0.11 mmol) werden in 2 ml Dimethylformamid gelöst und mit 21 mg Cyanophosphonsäurediethylester (0.12 mmol) sowie 40 µl Triethylamin (27 mg, 0.27 mmol) versetzt. Die Reaktionsmischung wird bei Raumtemperatur über Nacht gerührt, dann mit Essigsäureethylester versetzt und mit 5%-iger Kaliumhydrogensulfat-Lösung, gesättigter Natriumhydrogencarbonat-Lösung sowie gesättigter Natriumchlorid-Lösung gewaschen. Die organische Phase wird über Natriumsulfat getrocknet, das Lösungsmittel am Rotationsverdampfer entfernt und der Rückstand mittels präparativer HPLC (Eluent: Acetonitril/Wasser mit 0.1% Ameisensäure, Gradient 20:80 → 95:5) aufgereinigt. Es werden 36 mg (62% d. Th.) der Titelverbindung erhalten.

¹H-NMR (400 MHz, CDCl₃): δ = 1.00 (d, *J* = 6.2, 3H), 1.03 (d, *J* = 6.2, 3H), 1.15-1.36 (m, 5H), 1.50-1.62 (m, 2H), 1.69-1.79 (m, 2H), 2.17-2.27 (m, 1H), 2.26 (t, *J* = 7.6, 2H), 3.01-3.20 (m, 2H), 3.65 (s, 3H), 3.89-3.99 (m, 1H), 4.14-4.23 (m, 1H), 4.22 (d, *J* = 15.1, 1H), 4.46-4.51 (m, 1H), 5.01 (dd, *J* = 10.2, *J* = 6.4, 1H), 5.92-5.98 (m, 1H), 6.81-6.85 (m, 1H), 6.93 (s, 1H), 7.14-7.24 (m, 4H), 7.42 (dd, *J* = 7.6, *J* = 1.9, 1H).

LC/MS (Methode 3): Rₜ = 3.04 min.; MS (ESIpos): m/z = 547 [M+H]⁺.

### Beispiel 14

### 4-{2-[7-Chlor-5-(2-chlorphenyl)-1-isobutyl-2-oxo-1,2,4,5-tetrahydrobenzo[d]azepin-3-yl]-acetylamino}-hexansäure

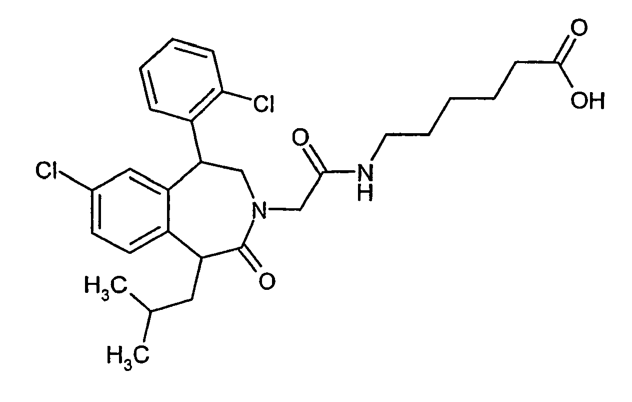

32 mg der Verbindung aus Beispiel 13 (0.06 mmol) werden in Dioxan/Wasser (2:1) gelöst, mit 90 µl 1 N Natronlauge versetzt und über Nacht bei Raumtemperatur gerührt. Die Reaktionslösung wird mit 1 N Salzsäure angesäuert (pH 2) und dreimal mit Dichlormethan extrahiert. Die vereinigten organischen Phasen werden über Natriumsulfat getrocknet und das Lösungsmittel am Rotationsverdampfer entfernt. Es werden 27 mg (88% d. Th.) der Titelverbindung erhalten.

¹H-NMR (400 MHz, CDCl₃): δ = 1.00 (d, *J* = 6.1, 3H), 1.02 (d, *J* = 6.1, 3H), 1.18-1.38 (m, 5H), 1.58 (sept, *J* = 7.3, 2H), 1.68-1.78 (m, 2H), 2.20-2.24 (m, 1H), 2.23 (t, *J* = 7.3, 2H), 3.04-3.20 (m, 2H), 3.91-4.02 (m, 1H), 4.15-4.24 (m, 1H), 4.24 (d, *J* = 14.9, 1H), 4.47-4.53 (m, 1H), 4.99-5.04 (m, 1H), 6.01-6.05 (m, 1H), 6.81-6.86 (m, 1H), 6.93 (s, 1H), 7.16-7.23 (m, 4H), 7.42 (dd, *J* = 7.6, *J* = 1.5, 1H).

LC/MS (Methode 3): Rₜ = 2.68 min.; MS (ESIpos): m/z = 533 [M+H]⁺.

### Beispiel 15

### [7-Chlor-1-isobutyl-5-(2-methoxyphenyl)-2-oxo-1,2,4,5-tetrahydro-3H-3-benzazepin-3-yl]essigsäure

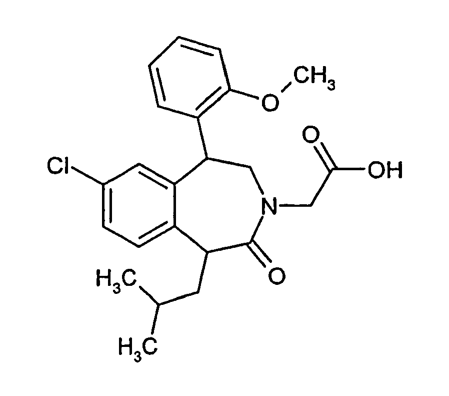

85 mg der Verbindung aus Beispiel 23A (0.19 mmol) werden in 3.5 ml Dioxan/Wasser (1:1) gelöst und mit 0.29 ml 1 N Natronlauge (0.29 mmol) versetzt. Die Reaktionsmischung wird 3 h bei Raumtemperatur gerührt. Es wird mit 5 ml 1 N Salzsäure angesäuert und die Mischung dreimal mit Dichlormethan extrahiert. Die vereinigten organischen Phasen werden über Natriumsulfat getrocknet und am Rotationsverdampfer eingeengt. Es werden 80 mg (100% d. Th.) der Titelverbindung erhalten.

¹H-NMR (400 MHz, CDCl₃): δ = 0.99-1.02 (m, 6H), 1.64-1.80 (m, 2H), 2.20-2.27 (m, 1H), 3.81-3.89 (m, 1H), 3.83 (s, 3H), 4.08-4.23 (m, 3H), 4.48-4.53 (m, 1H), 4.80 (dd, *J* = 9.5, *J* = 6.2, 1H), 6.75-6.79 (m, 1H), 6.86-9.94 (m, 3H), 7.12-7.17 (m, 2H), 7.23-7.27 (m, 1H).

LC/MS (Methode 1): Rₜ = 2.49 min.; MS (ESIpos): m/z = 416 [M+H]⁺.

### Beispiel 16

### Ethyl 1-{[7-chlor-1-isobutyl-5-(2-methoxyphenyl)-2-oxo-1,2,4,5-tetrahydro-3H-3-benzazepin-3-yl]acetyl}piperidin-4-carboxylat

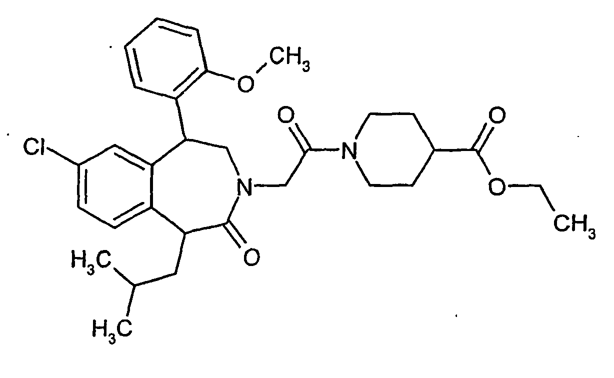

53 mg der Verbindung aus Beispiel 15 (0.13 mmol) sowie 21 mg Piperidin-4-carbonsäureethylester (0.13 mmol) werden in 1,9 ml Dimethylformamid gelöst und mit 25 mg Cyanophosphonsäurediethylester (0.14 mmol) sowie 30 µl Triethylamin (19 mg, 0.19 mmol) versetzt. Die Reaktionsmischung wird bei Raumtemperatur über Nacht gerührt, mit Essigsäureethylester versetzt und mit 5%-iger Kaliumhydrogensulfat-Lösung, gesättigter Natriumhydrogencarbonat-Lösung sowie gesättigter Natriumchlorid-Lösung gewaschen. Die organische Phase wird über Natriumsulfat getrocknet, das Lösungsmittel am Rotationsverdampfer entfernt und der Rückstand mittels präparativer HPLC (Eluent: Acetonitril/Wasser, Gradient 20:80 → 95:5) gereinigt. Es werden 52 mg (74% d. Th.) der Titelverbindung erhalten.

¹H-NMR (400 MHz, DMSO-d₆): δ = 0.95-0.99 (m, 6H), 1.17 (t, *J* = 7.3, 3H), 1.29-1.67 (m, 4H), 1.77-1.81 (m, 2H), 2.10-2.17 (m, 1H), 2.54-2.59 (m, 1H), 2.67-2.75 (m, 1H), 2.99-3.06 (m, 1H), 3.41-3.47 (m, 1H), 3.65-3.71 (m, 1H), 3.68 (s, 3H), 4.02-4.39 (m, 4H), 4.06 (t, *J* = 7.3, 2H), 4.69-4.77 (m, 2H), 6.89 (s, 1H), 6.93 (t, *J* = 7.4, 1H), 7.01 (d, *J* = 8.2, 1 H), 7.11-7.19 (m, 3H), 7.25-7.29 (m, 1H).

LC/MS (Methode 3): Rₜ = 3.04 min.; MS (ESIpos): m/z = 555 [M+H]⁺.

### Beispiel 17

### 7-Chlor-1-isobutyl-5-(2-methoxyphenyl)-3-(2-oxo-2-piperidin-1-ylethyl)-1,3,4,5-tetrahydro-2H-3-benzazepin-2-on

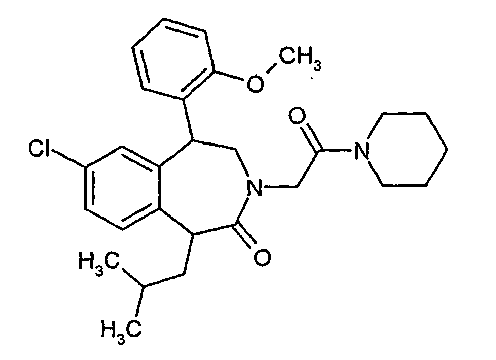

25 mg der Verbindung aus Beispiel 15 (0.06 mmol) und 6 mg Piperidin (0.06 mmol) werden in 1.0 ml Dimethylformamid gelöst und mit 12 mg Cyanophosphonsäurediethylester (0.07 mmol) sowie 13 µl Triethylamin (9 mg, 0.09 mmol) versetzt. Die Reaktionsmischung wird bei Raumtemperatur über Nacht gerührt, dann mit Essigsäureethylester versetzt und mit 5%-iger Kaliumhydrogensulfat-Lösung, gesättigter Natriumhydrogencarbonat-Lösung sowie gesättigter Natriumchlorid-Lösung gewaschen. Die organische Phase wird über Natriumsulfat getrocknet, das Lösungsmittel am Rotationsverdampfer entfernt und der Rückstand mittels präparativer HPLC (Eluent: Acetonitril/Wasser, Gradient 20:80 → 95:5) gereinigt. Es werden 16 mg (54% d. Th.) der Titelverbindung erhalten.

¹H-NMR (400 MHz, CDCl₃): δ = 0.99 (d, *J* = 6.4, 3H), 1.03 (d, *J* = 6.5, 3H), 1.41-1.62 (m, 6H), 1.63-1.80 (m, 2H), 2.24-2.31 (m, 1H), 3.20-3.25 (m, 2H), 3.45-3.52 (m, 2H), 3.78 (s, 3H), 3.79-4.34 (m, 4H), 4.64 (br. s, 1H), 4.76 (dd, *J* = 10.6, *J* = 7.0, 1H), 6.86-6.95 (m, 4H), 7.10-7.25 (m, 3H).

LC/MS (Methode 2): Rₜ = 3.04 min.; MS (ESIpos): m/z = 483 [M+H]⁺.

### Beispiel 18

### 1-{[7-Chlor-1-isobutyl-5-(2-rnethoxyphenyl)-2-oxo-1,2,4,5-tetrahydro-3H-3-benzazepin-3-yl]-acetyl}piperidin-4-carbonsäure

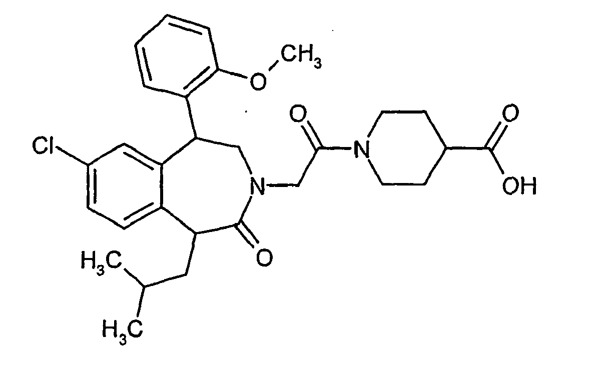

46 mg der Verbindung aus Beispiel 16 (0.08 mmol) werden in 2.5 ml Dioxan/Wasser (2:1) gelöst, mit 120 µl 1 N Natronlauge versetzt und über Nacht bei Raumtemperatur gerührt. Die Reaktionslösung wird mit 1 N Salzsäure angesäuert (pH 2) und dreimal mit Dichlormethan extrahiert. Die vereinigten organischen Phasen werden über Natriumsulfat getrocknet, am Rotationsverdampfer eingeengt und der Rückstand mittels präparativer HPLC (Eluent: AcetonitriWVasser mit 0.1% Ameisensäure, Gradient 20:80 → 95:5) gereinigt. Es werden 30 mg (69% d. Th.) der Titelverbindung erhalten.

¹H-NMR (400 MHz, CDCl₃): δ = 0.99 (d, *J* = 6.4, 3H), 1.02 (d, *J* = 6.4, 3H), 1.51-1.83 (m, 5H), 1.89-1.96 (m, 1H), 2.23-2.30 (m, 1H), 2.47-2.52 (m, 1H), 2.79-3.03 (m, 2H), 3.62-4.43 (m, 6H), 3.78 (s, 3H), 4.60-4.64 (m, 1H), 4.73-4.77 (m, 1H), 6.86-6.95 (m, 4H), 7.09-7.25 (m, 3H).

LC/MS (Methode 2): Rₜ = 2.61 min.; MS (ESIpos): m/z = 527 [M+H]⁺.

### Beispiel 19

### [7-Chlor-5-(2,3-dimethoxyphenyl)-1-isobutyl-2-oxo-1,2,4,5-tetrahydro-3H-3-benzazepin-3-yl]-essigsäure

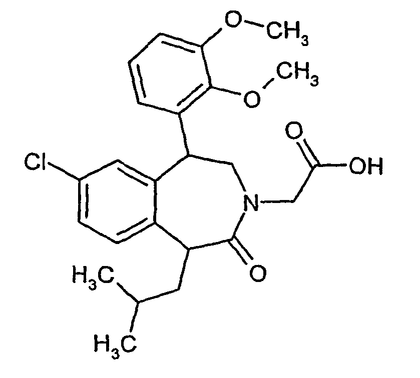

73 mg der Verbindung aus Beispiel 30A (0.15 mmol) werden in 3.5 ml Dioxan/Wasser (1:1) gelöst und mit 0.23 ml 1 N Natronlauge (0.23 mmol) versetzt. Die Reaktionsmischung wird 3 h bei Raumtemperatur gerührt. Es wird mit 5 ml 1 N Salzsäure angesäuert und die Mischung dreimal mit Dichlormethan extrahiert. Die vereinigten organischen Phasen werden über Natriumsulfat getrocknet und am Rotationsverdampfer eingeengt. Es werden 69 mg (100% d. Th.) der Titelverbindung erhalten.

¹H-NMR (400 MHz, DMSO-d₆): δ = 0.96 (d, *J* = 6.2, 3H), 0.98 (d, *J* = 6.2, 3H), 1.54-1.68 (m, 2H), 2.08-2.16 (m, 1H), 3.25 (s, 3H), 3.42-3.56 (m, 1H), 3.79 (s, 3H), 4.01-4.14 (m, 2H), 4.33-4.42 (m, 1H), 4.69 (dd, *J* = 12.0, *J* = 7.1, 1H), 4.74-4.79 (m, 1H), 6.81-6.84 (m, 1H), 6.92 (s, 1H), 6.98-7.09 (m, 2H), 7.20-7.23 (m, 2H), 12.55 (br. s, 1H).

LC/MS (Methode 2): Rₜ = 2.67 min.; MS (ESIpos): m/z = 446 [M+H]⁺.

### Beispiel 20

### Ethyl 1-{[7-chlor-5-(2,3-dinlethoxyphenyl)-1-isobutyl-2-oxo-1,2,4,5-tetrahydro-3H-3-benzazepin-3-yl]acetyl}piperidine-4-carboxylat

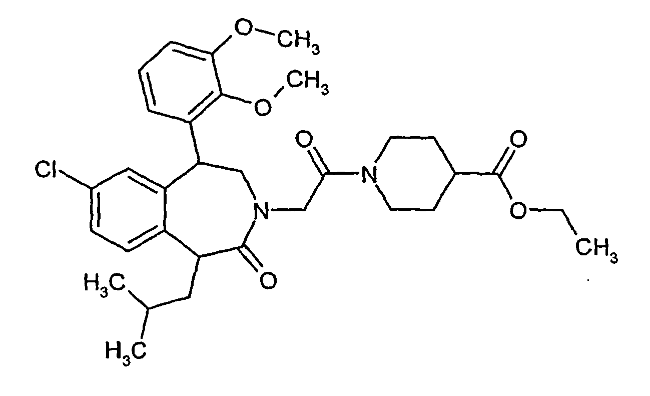

69 mg der Verbindung aus Beispiel 19 (0.15 mmol) und 26 mg Piperidin-4-carbonsäureethylester (0.16 mmol) werden in 2.5 ml Dimethylformamid gelöst und mit 30 mg Cyanophosphonsäurediethylester (0.17 mmol) sowie 32 µl Triethylamin (23 mg, 0.23 mmol) versetzt. Die Reaktionsmischung wird bei Raumtemperatur über Nacht gerührt, dann mit Essigsäureethylester versetzt und mit 5%-iger Kaliumhydrogensulfat-Lösung, gesättigter Natriumhydrogencarbonat-Lösung sowie gesättigter Natriumchlorid-Lösung gewaschen. Die organische Phase wird über Natriumsulfat getrocknet, das Lösungsmittel am Rotationsverdampfer entfernt und der Rückstand mittels präparativer HPLC (Eluent: Acetonitril/Wasser, Gradient 20:80 → 95:5) gereinigt. Es werden 62 mg (68% d. Th.) der Titelverbindung erhalten.

¹H-NMR (400 MHz, CDCl₃): δ = 1.00 (d, *J* = 6.2,3H), 1.04 (d, *J* = 6.2, 3H), 1.24 (t, J = 7.1,3H), 1.29-1.94 (m, 6H), 2.23-2.35 (m, 1H), 2.39-2.49 (m, 1H), 2.76-3.07 (m, 2H), 3.43 (s, 3H), 3.53-4.55 (m, 6H), 3.86 (m, 3H), 4.13 (q, *J* = 7.1, 2H), 4.57-4.66 (m, 1H), 4.73-4.83 (m, 1H), 6.67-6.71 (m, 1H), 6.84-6.87 (m, 1H), 6.96-7.20 (m, 4H).

LC/MS (Methode 3): Rₜ = 3.04 min.; MS (ESIpos): m/z = 585 [M+M]⁺

### Beispiel 21

### 1-{[7-Chlor-5-(2,3-dimethoxyphenyl)-1-isobutyl-2-oxo-1,2,4,5-tetrahydro-3H-3-benzazepin-3-yl]-acetyl} piperidin-4-carbonsäure

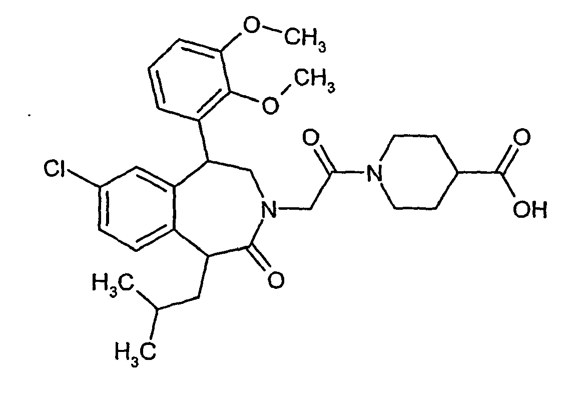

64 mg der Verbindung aus Beispiel 20 (0.11 mmol) werden in 3.3 ml Dioxan/Wasser (2:1) gelöst, mit 165 µl 1 N Natronlauge versetzt und über Nacht bei Raumtemperatur gerührt. Die Reaktionslösung wird mit 1 N Salzsäure angesäuert (pH 2) und dreimal mit Dichlormethan extrahiert. Die vereinigten organischen Phasen werden über Natriumsulfat getrocknet, am Rotationsverdampfer eingeengt und der Rückstand mittels präparativer HPLC (Eluent: Acetonitril/Wasser mit 0.1% Ameisensäure, Gradient 20:80 → 95:5) gereinigt. Es werden 61 mg (100% d. Th.) der Titelverbindung erhalten.

¹H-NMR (400 MHz, CDCl₃): δ = 1.00 (d, *J* = 6.5, 3H), 1.04 (d, *J* = 6.4, 3H), 1.48-1.97 (m, 6H), 2.26-2.32 (m, 1H), 2.46-2.52 (m, 1H), 2.77-3.04 (m, 2H), 3.43 (s, 3H), 3.54-4.63 (m, 7H), 3.86 (s, 3H), 4.76-4.81 (m, 1H), 6.67-7.20 (m, 6H).

LC/MS (Methode 2): Rₜ = 2.61 min.; MS (ESIpos): m/z = 557 [M+H]⁺.

### Beispiel 22

### Ethyl 1-{[5-(2-bromphenyl)-7-chlor-1-isobutyl-2-oxo-1,2,4,5-tetrahydro-3H-3-benzazepin-3-yl]-acetyl}piperidin-4-carboxylat

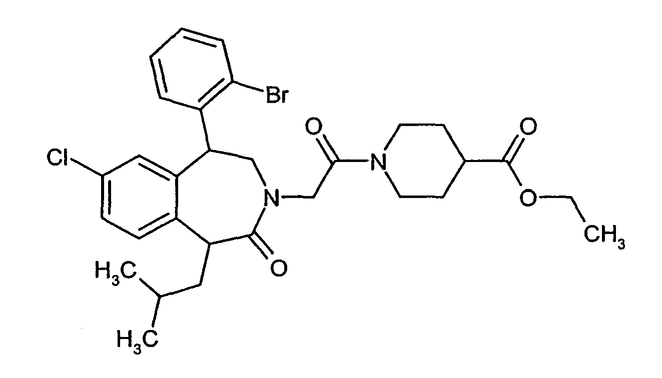

190 mg der Verbindung aus Beispiel 6 (0.39 mmol) und 65 mg Piperidin-4-carbonsäureethylester (0.41 mmol) werden in 5.0 ml Dimethylformamid gelöst und mit 75 mg Cyanophosphonsäurediethylester (0.43 mmol) sowie 81 µl Triethylamin (59 mg, 0.58 mmol) versetzt. Die Reaktionsmischung wird bei Raumtemperatur 2.5 h lang gerührt, dann mit Essigsäureethylester versetzt und mit 5%-iger Kaliumhydrogensulfat-Lösung, gesättigter Natriumhydrogencarbonat-Lösung sowie gesättigter Natriumchlorid-Lösung gewaschen. Die organische Phase wird über Natriumsulfat getrocknet, das Lösungsmittel am Rotationsverdampfer entfernt und der Rückstand mittels präparativer HPLC (Eluent: Acetonitril/Wasser, Gradient 20:80 → 95:5) gereinigt. Es werden 204 mg (87% d. Th.) der Titelverbindung erhalten.

¹H-NMR (400 MHz, CDCl₃): δ = 1.00 (d, *J* = 6.2, 3H), 1.03 (d, *J* = 6.2, 3H), 1.25 (t, *J* = 7.1, 3H), 1.46-1.93 (m, 6H), 2.20-2.28 (m, 1H), 2.44-2.51 (m, 1H), 2.80-3.07 (m, 2H), 3.55-4.33 (m, 6H), 4.14 (t, *J* = 7.1, 2H), 4.47-4.54 (m, 1H), 4.99-5.05 (m, 1H), 6.82-6.86 (m, 1H), 6.92 (br. s, 1H), 7.09-7.24 (m, 4H), 7.59-7.61 (m, 1H).

LC/MS (Methode 3): Rₜ = 3.16 min.; MS (ESIpos): m/z = 603 [M+H]⁺.

### Beispiel 23

### 1-{[5-(2-Bromphenyl)-7-chlor-1-isobutyl-2-oxo-1,2,4,5-tetrahydro-3H-3-benzazepin-3-yl]acetyl}-piperidin-4-carbonsäure

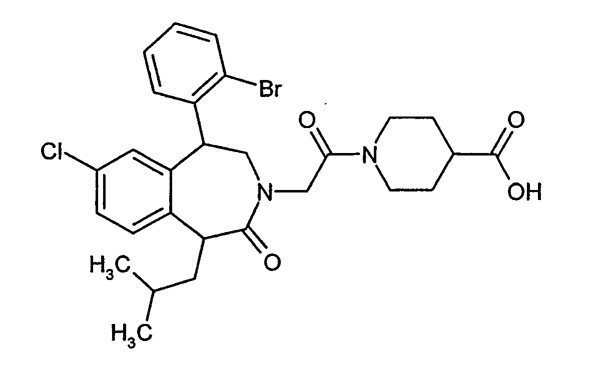

24 mg der Verbindung aus Beispiel 22 (0.04 mmol) werden in 2.0 ml THF/Methanol (1:1) gelöst. Es werden 0.1 ml 1 N Natronlauge zugegeben und die Reaktionsmischung bei Raumtemperatur 16 h lang gerührt. Es wird mit Wasser verdünnt, mit 1 N Salzsäure angesäuert und die Mischung dreimal mit Dichlormethan extrahiert. Die vereinigten organischen Phasen werden über Natriumsulfat getrocknet und das Lösungsmittel am Rotationsverdampfer entfernt. Es werden 23 mg (100% d. Th.) der Titelverbindung erhalten.

¹H-NMR (400 MHz, CDCl₃): δ = 1.00 (d, *J* = 6.2, 3H), 1.02 (d, *J* = 6.2, 3H), 1.41-1.98 (m, 6H), 2.19-2.27 (m, 1H), 2.49-2.56 (m, 1H), 2.81-3.06 (m, 2H), 3.56-4.43 (m, 6H), 4.47-4.54 (m, 1H), 4.98-5.04 (m, 1H), 6.79-6.85 (m, 1H), 6.91 (br. s, 1H), 7.09-7.25 (m, 4H), 7.60 (dd, *J* = 7.9, *J* = 1.1, 1H).

LC/MS (Methode 3): Rₜ = 2.75 min.; MS (ESIpos): m/z = 575 [M+H]⁺.

### Beispiel 24

### 1-{[7-Chlor-5-(2-ethynylphenyl)-1-isobutyl-2-oxo-1,2,4,5-tetrahydro-3H-3-benzazepin-3-yl]-acetyl}piperidin-4-carbonsäure

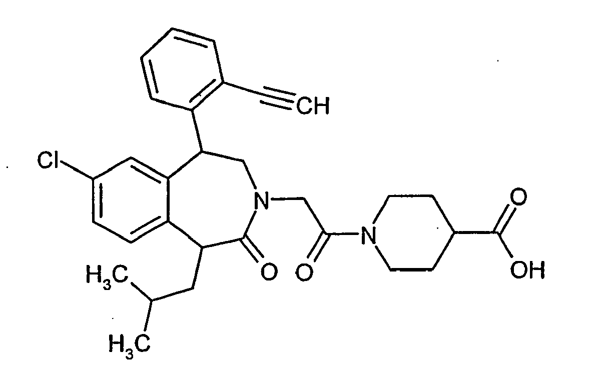

12 mg der Verbindung aus Beispiel 31A (0.019 mmol) werden in 2.0 ml THE/Methanol (1:1) gelöst, mit 100 µl N Natronlauge versetzt und bei Raumtemperatur für 16 h gerührt. Die Reaktionslösung wird mit 1 N Salzsäure angesäuert, mit Wasser verdünnt und dreimal mit Dichlormethan extrahiert. Die vereinigten organischen Phasen werden über Natriumsulfat getrocknet und der Rückstand mittels präparativer HPLC (Eluent: Acetonitril/Wasser mit 0.1% Ameisensäure, Gradient 20:80 → 95:5) gereinigt. Es werden 6 mg (58% d. Th.) der Titelverbindung erhalten.

LC/MS (Methode 1): Rₜ = 2.47 min.; MS (ESIpos): m/z = 521 [M+H]⁺.

Die folgenden Verbindungen werden analog zu den zuvor beschriebenen Beispielen aus den entsprechenden Ausgangsverbindungen hergestellt:

### Beispiel 25

### (1-{2-[7-Chlor-1-isobutyl-5-(2-methoxyphenyl)-2-oxo-1,2,4,5-tetrahydrobenzo[d]azepin-3-yl]-acetyl}-piperidin-4-yl)-essigsäure

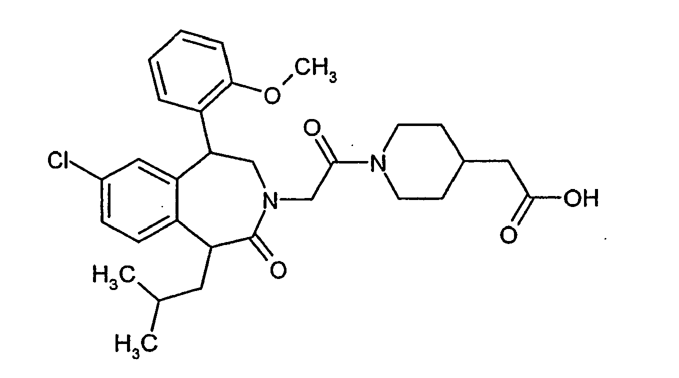

### Beispiel 26

### (1-{2-[7-Chlor-5-(2,4-dimethylphenyl)-1-isobutyl-2-oxo-1,2,4,5-tetrahydrobenzo[d]azepin-3yl]-acetyl}-piperidin-4-yl)-essigsäure

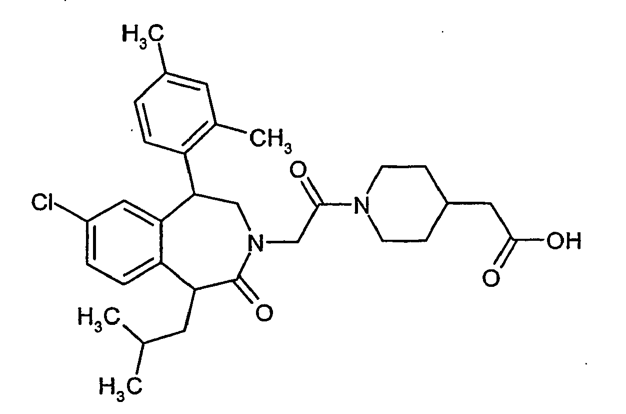

### Beispiel 27

### (1-{2-[7-Chlor-5-(2,3-dimethylphenyl)-1-isobutyl-2-oxo-1,2,4,5-tetrahydrobenzo[d]azepin-3-yl]-acetyl}-piperidin-4-yl)-essigsäure

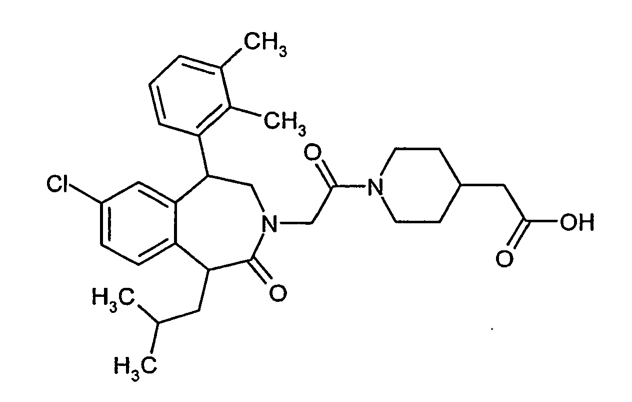

### Beispiel 28

### (1-{2-[7-Chlor-S-(2,3-dimethoxyphenyl)-1-isobutyl-2-oxo-1,2,4,5-tetrahydrobenzo[d]azepin-3-yl]-acetyl}-piperidin-4-yl)-essigsäure

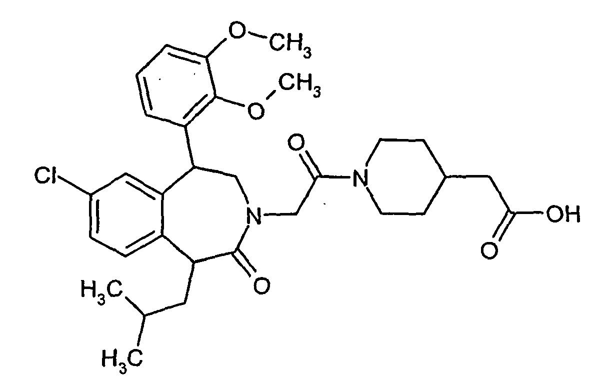

### Beispiel 29

### (1-{2-[7-Chlor-5-(2,3-dihydrobenzo[1,4]dioxin-5-yl)-1-isobutyl-2-oxo-1,2,4,5-tetrahydrobenzo[d]-azepin-3-yl]-acetyl}-piperidin-4-yl)-essigsäure

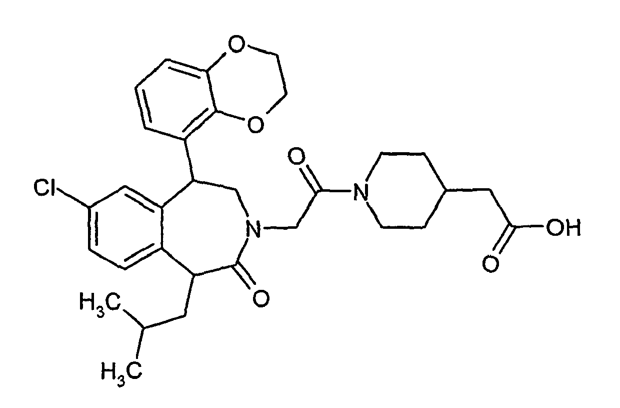

### Beispiel 30

### (1-{2-[7-Chlor-1-isobutyl-5-(naphthalin-l-yl)-2-oxo-1,2,4,5-tetrahydrobenzo[d]azepin-3-yl]-acetyl}-piperidin-4-yl)-essigsäure

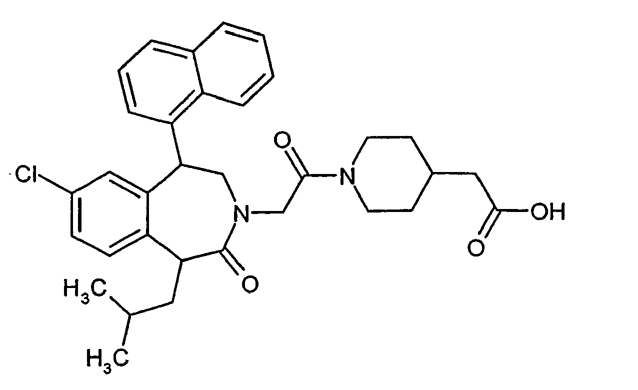

### Beispiel 31

### (1-{2-[5-(2-Chlorphenyl)-1-isobutyl-7-methyl-2-oxo-1,2,4,5-tetrahydrobenzo[d]azepin-3-yl]-acetyl}-piperidin-4-yl)-essigsäure

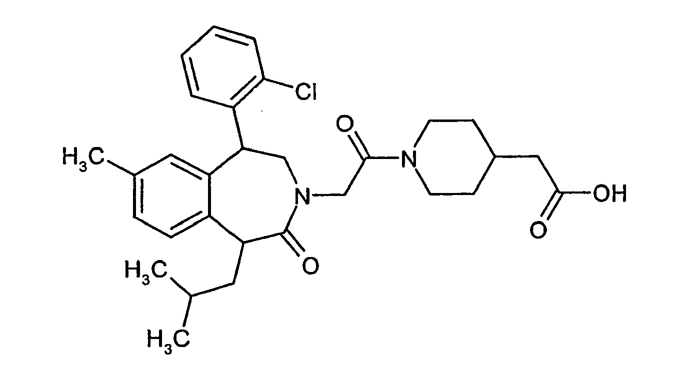

### Beispiel 32

### (1-{2-[5-(2-Chlorphenyl)-1-isobutyl-2-oxo-7-trifluormethyl-1,2,4,5-tetrahydrobenzo[d]azepin-3-yl]-acetyl}-piperidin-4-yl)-essigsäure

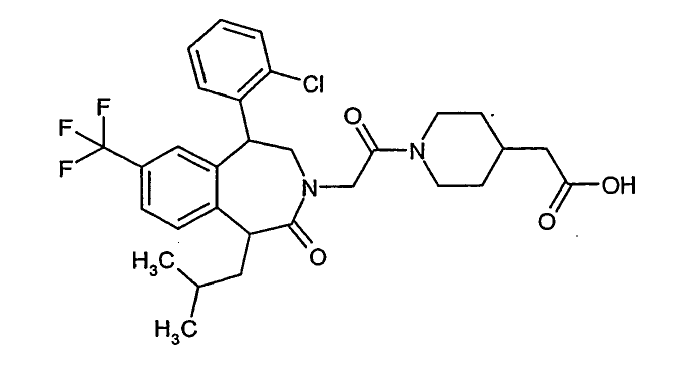

### Beispiel 33

### (1-{2-[5-(2-Chlorphenyl)-1-(2,2-dimethylpropyl)-2-oxo-7-trifluormethyl-1,2,4,5-tetrahydrobenzo-[d]azepin-3-yl]-acetyl}-piperidin-4-yl)-essigsäure

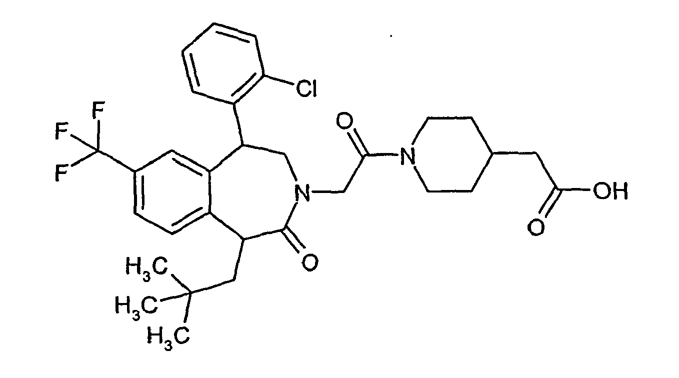

### Beispiel 34

### (1-{2-[1-Isobutyl-5-(2-methoxyphenyl)-2-oxo-7-trifluormethyl-1,2,4,5-tetrahydrobenzo[d]azepin-3-yl]-acetyl}-piperidin-4-yl)-essigsäure

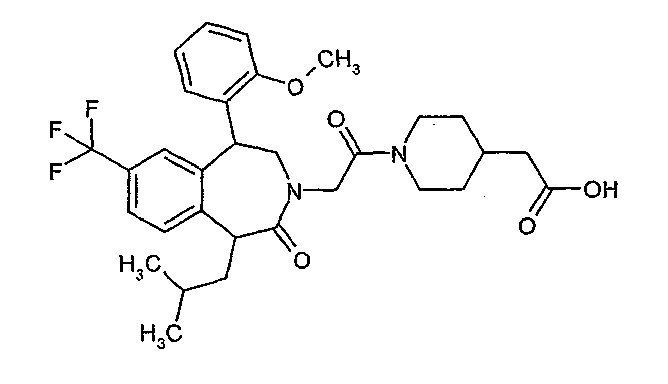

### Beispiel 35

### (1-{2-[1-(2,2-Dimethylpropyl)-5-(2-methoxyphenyl)-2-oxo-7-trifluormethyl-1,2,4,5-tetrahydrobenzo[d]azepin-3-yl]-acetyl}-piperidin-4-yl)-essigsäure

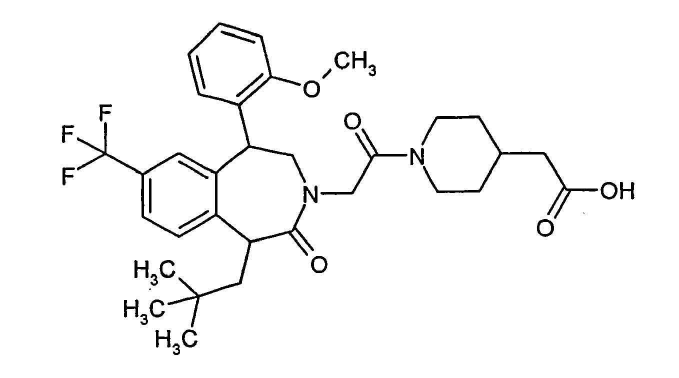

### Beispiel 36

### (1-{2-[5-(2,3-Dimethoxyphenyl)-1-isobutyl-2-oxo-7-trifluormethyl-1,2,4,5-tetrahydrobenzo[d]-azepin-3-yl]-acetyl}-piperidin-4-yl)-essigsäure

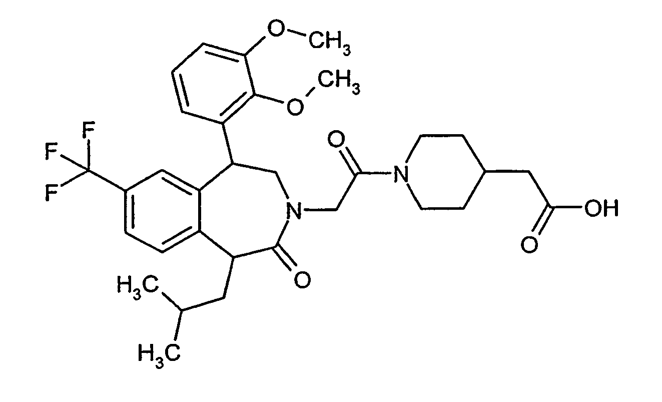

### Beispiel 37

### (1-{2-[5-(2,3-Dimethoxyphenyl)-1-(2,2-dimethylpropyl)-2-oxo-7-trifluormethyl-1,2,4,5-tetrahydrobenzo[d]azepin-3-yl]-acetyl}-piperidin-4-yl)-essigsäure

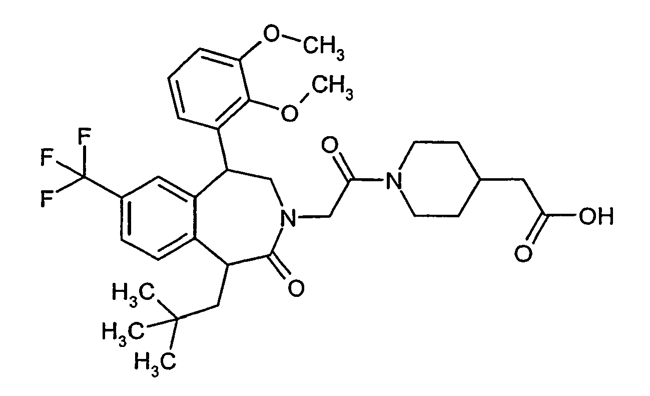

### Beispiel 38

### {2-[7-Chlor-5-(2-chlorphenyl)-1-isobutyl-2-oxo-1,2,4,5-tetrahydrobenzo[d]azepin-3-yl]-acetylamino}-essigsäure

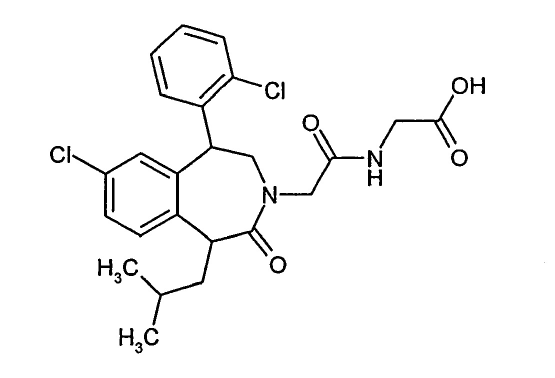

### Beispiel 39

### 4-{2-[7-Chlor-5-(2-chlorphenyl)-1-isobutyl-2-oxo-1,2,4,5-tetraliydrobenzo[d]azepin-3-yl]-acetylamino}-buttersäure

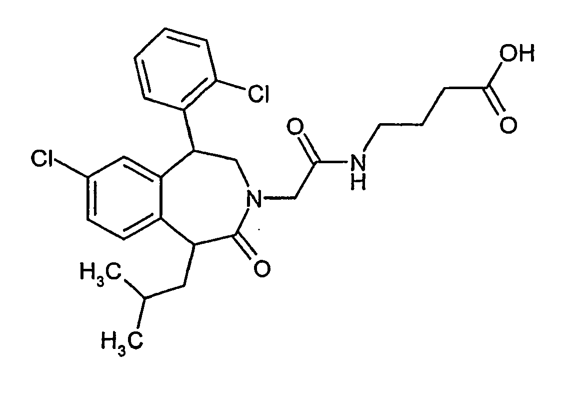

### Beispiel 40

### (1-{2-[7-Chlor-5-(2-chlorphenyl)-1-(2,2-dimethylpropyl)-2-oxo-1,2,4,5-tetrahydrobenzo[d]azepin-3-yl]-acetyl}-piperidin-4-yl)-carbonsäure

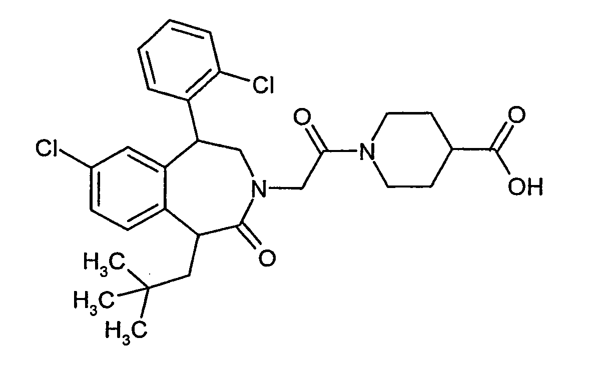

### Beispiel 41

### (1-{2-[7-Chlor-1-(2,2-dimethylpropyl)-5-(2-methoxyphenyl)-2-oxo-1,2,4,5-tetrahydrobenzo[d]-azepin-3-yl]-acetyl}-piperidin-4-yl)-carbonsäure

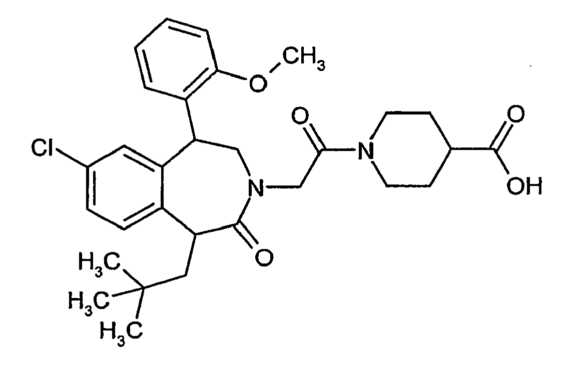

### Beispiel 42

### (1-{2-[7-Chlor-5-(2,4-dimethylphenyl)-1-isobutyl-2-oxo-1,2,4,5-tetrahydrobenzo[d]azepin-3-yl]-acetyl}-piperidin-4-yl)-carbonsäure

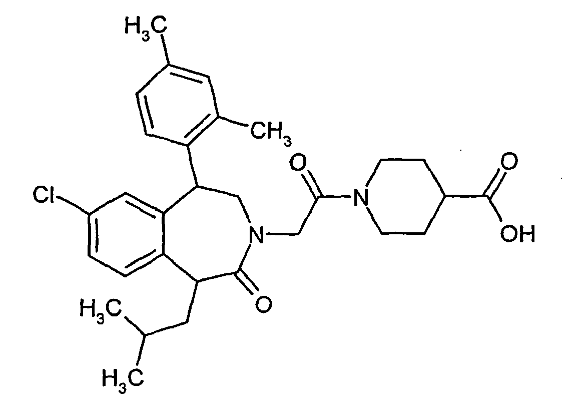

### Beispiel 43

### (1-{2-[7-Chlor-5-(2,3-dimethylphenyl)-1-isobutyl-2-oxo-1,2,4,5-tetrahydrobenzo[d]azepin-3-yl]-acetyl}-piperidin-4-yl)-carbonsäure

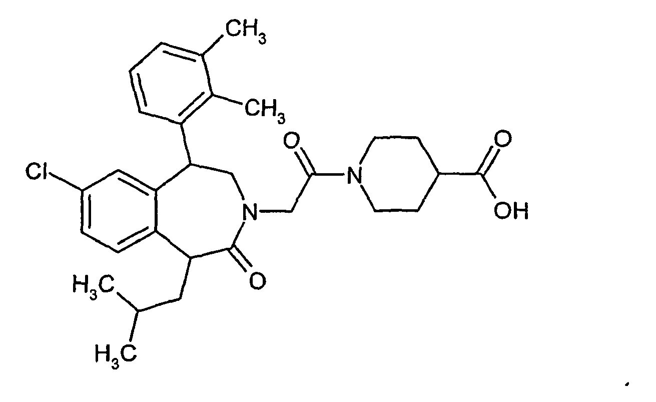

### Beispiel 44

### (1-{2-[7-Chlor-5-(2,3-dimethoxyphenyl)-1-(2,2-dimethylpropyl)-2-oxo-1,2,4,5-tetrahydrobenzo[d]-azepin-3-yl]-acetyl}-piperidin-4-yl)-carbonsäure

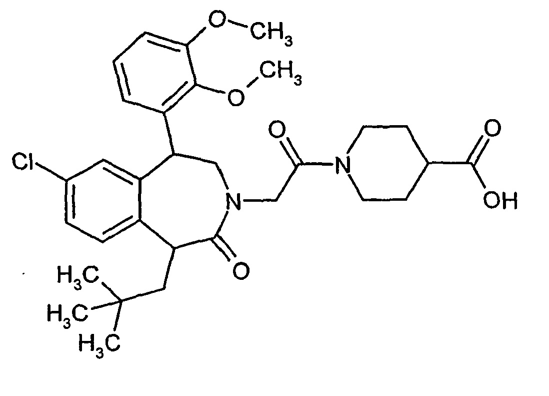

### Beispiel 45

### (1-{2-[7-Chlor-5-(2,3-dihydrobenzo[l,4]dioxin-5-yl)-1-isobutyt-2-oxo-1,2,4,5-tetrahydrobenzo[d]-azepin-3-yl]-acetyl}-piperidin-4-yl)-carbonsäure

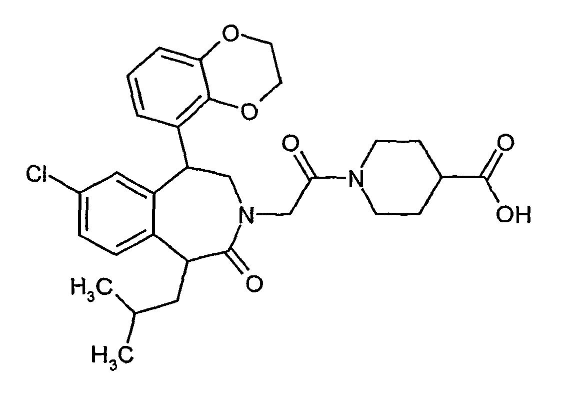

### Beispiel 46

### (1-{2-[7-Chlor-1-isobutyl-5-(naphthalin-1-yl)-2-oxo-1,2,4,5-tetrahydrobenzo[d]azepin-3-yl]-acetyl}-piperidin-4-yl)-carbonsäure

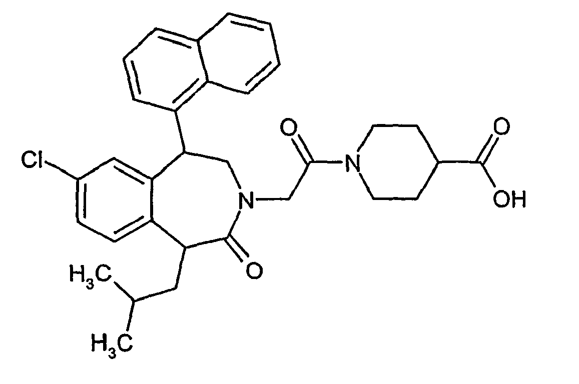

### Beispiel 47

### (1-{2-[5-(2-Chlorphenyl)-1-isobutyl-7-methyl-2-oxo-1,2,4,5-tetrahydrobenzo[d]azepin-3-yl]-acetyl}-piperidin-4-yl)-carbonsäure

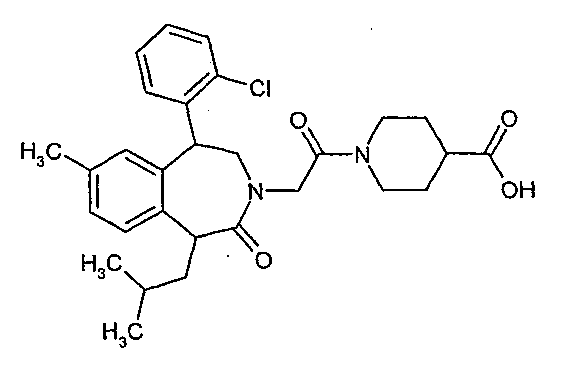

### Beispiel 48

### (1-{2-[5-(2-Chlorphenyl)-1-isobutyl-2-oxa-7-trifluormethyl-1,2,4,5-tetrahydrobenzo[d]azepin-3-yl]-acetyl}-piperidin-4-yl)-carbonsäure

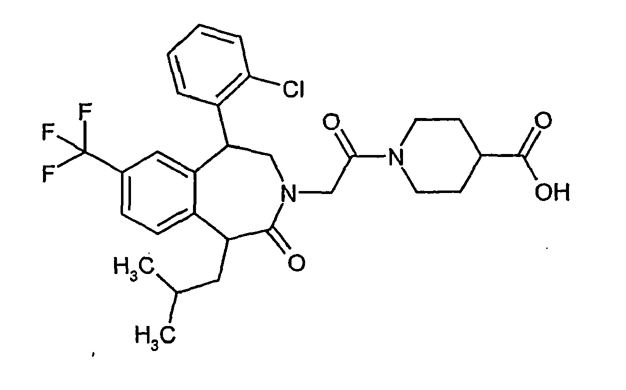

### Beispiel 49

### (1-{2-[5-(2-Chlorphenyl)-1-(2,2-dimethylpropyl)-2-oxo-7-trifluormethyl-1,2,4,5-tetrahydrobenzo-[d]azepin-3-yl]-acetyl}-piperidin-4-yl)-carbonsäure

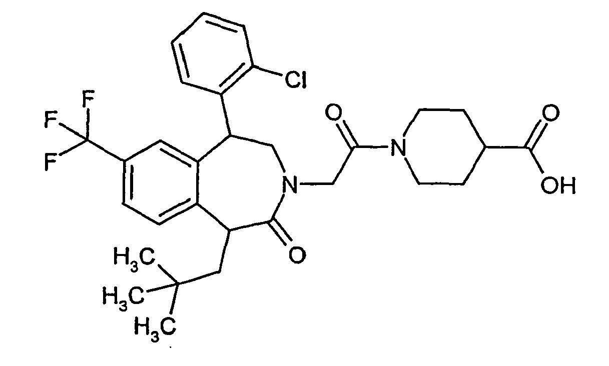

### Beispiel 50

### (1-{2-[1-Isobutyl-5-(2-methoxyphenyl)-2-oxo-7-trifluonnethyl-1,2,4,5-tetrahydrobenzo[d]azepin-3-yl]-acetyl}-piperidin-4-yl)-carbonsäure

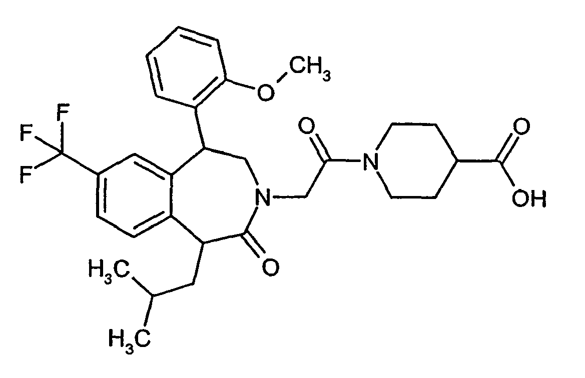

### Beispiel 51

### (1-{2-[1-(2,2-Dimethylpropyl)-5-(2-methoxyphenyl)-2-oxo-7-trifluormethyl-1,2,4,5-tetrahydrobenzo[d]azepin-3-yl]-acetyl}-piperidin-4-yl)-carbonsäure

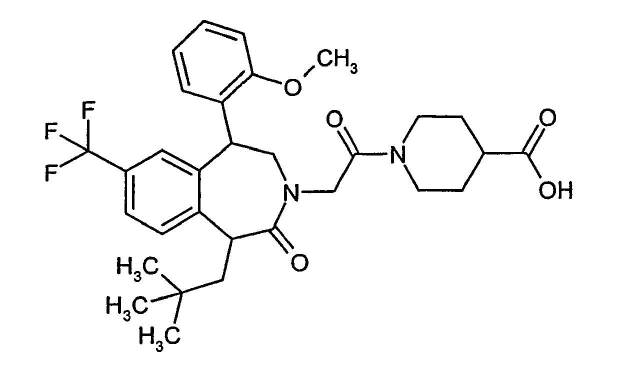

### Beispiel 52

### (1-{2-[5-(2,3-Dimethoxyphenyl)-1-isobutyl-2-oxo-7-trifluormethyl-1,2,4,5-tetrahydrobenzo[d]-azepin-3-yl]-acetyl}-piperidin-4-yl)-carbonsäure

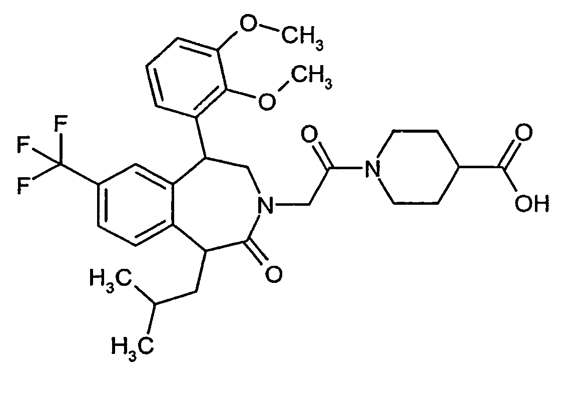

### Beispiel 53

### (1-{2-[5-(2,3-Dimethoxyphenyl)-1-(2,2-dimethylpropyl)-2-oxo-7-trifluormethyl-1,2,4,5-tetrahydrobenzo[d]azepin-3-yl]-acetyl}-piperidin-4-yl)-carbonsäure

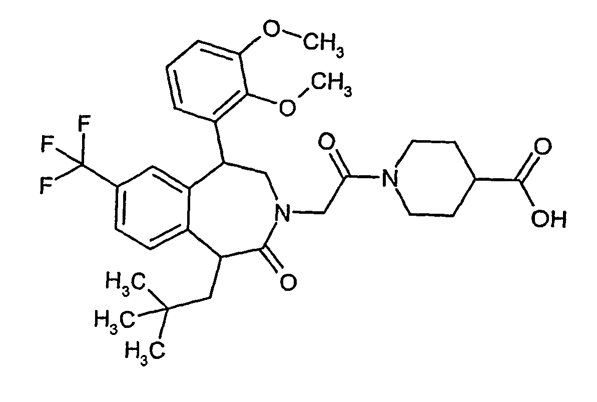

### B. Bewertung der pharmakologischen Wirksamkeit

Die pharmakologische Wirkung der erfindungsgemäßen Verbindungen kann in folgenden Assays gezeigt werden:

### 1. Squalen-Synthase-Inhibitionsassay

### a) Gewinnung von Mikrosomen:

Als Quelle für Squalen-Synthase für den Aktivitäts-Assay werden Mikrosomen aus Rattenlebern präpariert. Die Rattenlebern werden in doppeltem Volumen Homogenisierungs-Puffer [100 mM Tris/HCl, 0.2 M Sucrose, 30 mM Nicotinamid, 14 mM Natriumfluorid, 5 mM Dithiothreitol, 5 mM MgCl₂, Protease-Inhibitor-Cocktail (Fa. Sigma, Taufkirchen), pH 7.5] zerkleinert und homogenisiert (Dounce Homogenisator). Der Überstand einer 10.000 g - Zentrifugation wird anschließend bei 100.500 g zentrifugiert. Die pelletierten Mikrosomen werden in Homogenisierungspuffer aufgenommen, auf 10 mg/ml Protein verdünnt und bei -80°C gelagert.

### b) Aktivitäts-Assay der Squalen-Synthase:

Die Umsetzung von trans; trans-[1-³H]-Farnesylpyrophosphat zu [³H]-Squalen durch die mikrosomale Squalen-Synthase erfolgt unter folgenden Reaktionsbedingungen: Rattenleber-Mikrosomen (Proteingehalt 65 µg/ml), 1 mM NADPH, 6 mM Glutathion, 10% PBS, 10 mM Natriumfluorid, 5 mM MgCl₂, pH 7.5. Die jeweils zu testende Verbindung wird in DMSO gelöst und dem Assay in definierter Konzentration zugesetzt. Die Reaktion wird durch Zugabe von Farnesylpyrophosphat (Endkonzentration 5 uM) und 20 kBq/ml trans, trans-[1-³H]-Farnesylpyrophosphat gestartet und für 10 min. bei 37°C inkubiert. Anschließend werden 100 µl der Reaktionslösung mit 200 µl Chloroform, 200 µl Methanol und 60 µl 5 N Natronlauge versetzt und auf 2 mM Squalen eingestellt. Nach intensivem Mischen und anschließender Phasentrennung wird ein Aliquot der organischen Phase in Szintillationsflüssigkeit (Packard Ultima Gold LSC Cocktail) überführt und die organisch extrahierbaren radioaktiven Verbindungen quantifiziert (LS 6500, Fa. Beckman). Die Reduktion des radioaktiven Signals ist direkt proportional zur Inhibition der Squalen-Synthase durch die jeweils eingesetzte Verbindung.

Die Ausführungsbeispiele zeigen in diesem Test IC₅₀-Werte von < 20 µM.

### 2. Hemmung der Squalen- und Cholesterinsynthese in der Leber von Mäusen

Männliche NMRI-Mäuse werden auf normaler Nagerdiät (NAFAG 3883) in Stoffwechselkäfigen gehalten. Der LichtlDunkel-Zyklus beträgt 12 Stunden, von 6 Uhr bis 18 Uhr und von 18 Uhr bis 6 Uhr. Die Tiere werden mit einem Körpergewicht zwischen 25 g und 40 g in Gruppen von 8-10 Tieren in die Versuche eingesetzt. Futter und Trinkwasser stehen den Tieren ad libitum zur Verfügung.

Die Substanzen werden entsprechend ihrer Löslichkeit in wässriger Traganth-Suspension (0.5%) oder in Solutol HS15/Kochsalz-Lösung (20:80) mit der Schlundsonde in einem Volumen von 10 ml/kg Körpergewicht oral verabreicht oder auch in Solutol HS15/Kochsalz-Lösung (20:80) oder DMSO/Kochsalz-Lösung (20:80) subkutan injiziert. Die entsprechenden Kontrollgruppen erhalten nur das entsprechende Formulierungsmittel ohne Wirkstoff. Eine oder zwei Stunden nach Substanzapplikation wird den Tieren radioaktiv markiertes ¹⁴C-Mevalonolacton intraperitoneal injiziert. Eine oder zwei Stunden nach der Injektion von ¹⁴C-Mevalonolacton, bzw. 2-4 Stunden nach der Substanzapplikation, werden die Tiere getötet, der Bauchraum geöffnet und Lebergewebe entnommen. Sofort nach der Entnahme wird das Gewebe oberflächlich abgetrocknet, gewogen und in Isopropanol homogenisiert. Die weitere Aufarbeitung und Extraktion des synthetisierten Squalens und seiner Folgeprodukte erfolgt nach einer Methode von I. Duncan et al. (*J. Chromatogr.* 1979, 162), modifiziert nach H. Bischoff et al. (*Atherosclerosis* 1997, 135).

Die extrahierte Lipidfraktion wird in 1 ml Isopropanol aufgenommen, in Szintillationsröhrchen überführt, mit 15 ml Ultima Gold^{®}-Szintillationsflüssigkeit (Packard) aufgefüllt und in einem Flüssigszintillationszähler (Beckmann Coulter LS 6500) gezählt.

Nach Berechnung der spezifischen ¹⁴C-Aktivität der Lipidfraktion (dpm/g Lebergewebe) wird die Syntheserate des radioaktiv markierten ¹⁴C-Squalens und der ¹⁴C-Folgemetabolite der mit Wirkstoff behandelten Tiere verglichen mit der Syntheserate des radioaktiv markierten ¹⁴C-Squalens und der ¹⁴C-Folgemetabolite der nur mit Formulierungsmittel behandelten Kontrolltiere. Eine Herabsetzung der Syntheserate um ≥ 30% verglichen mit der Syntheserate der Kontrolltiere (= 100%) wird als pharmakologisch wirksam angesehen, wenn die statistische Beurteilung mit Student's t-test einen p-Wert < 0.05 ergibt.

### 3. Hemmung der Squalen- und Cholesterinsynthese in der Leber von Ratten

Männliche Wistar-Ratten werden auf normaler Nagerdiät (NAFAG 3883) in Makrolon^{®}-Typ III-Käfigen gehalten. Der Licht/Dunkel-Zyklus beträgt 12 Stunden, von 6 Uhr bis 18 Uhr und von 18 Uhr bis 6 Uhr. Die Tiere werden mit einem Körpergewicht zwischen 150 g und 200 g in Gruppen von 6-8 Tieren in die Versuche eingesetzt. Das Futter wird den Tieren 18-22 Stunden vor Versuchsbeginn entzogen, Trinkwasser steht ad libitum bis Versuchsende zur Verfügung.

Die Substanzen werden entsprechend ihrer Löslichkeit in wässriger Traganth-Suspension (0.5%) oder in Solutol HS15/Kochsalz-Lösung (20:80) mit der Schlundsonde in einem Volumen von 10 ml/kg Körpergewicht oral verabreicht oder auch in Solutol HS15/Kochsalz-Lösung (20:80) oder DMSO/Kochsalz-Lösung (20:80) subkutan injiziert. Die entsprechenden Kontrollgruppen erhalten nur das entsprechende Formulierungsmittel ohne Wirkstoff. Eine oder zwei Stunden nach Substanzapplikation wird den Tieren radioaktiv markiertes ¹⁴C-Mevalonolacton intraperitoneal injiziert. Eine oder zwei Stunden nach der Injektion von ¹⁴C-Mevalonolacton, bzw. 2-4 Stunden nach der Substanzapplikation, werden die Tiere getötet, der Bauchraum geöffnet und Lebergewebe entnommen. Sofort nach der Entnahme wird das Gewebe oberflächlich abgetrocknet, gewogen und in Isopropanol homogenisiert. Die weitere Aufarbeitung und Extraktion des synthetisierten Squalens und seiner Folgeprodukte erfolgt nach einer Methode von I. Duncan et al. (J. Chromatogr. 1979, 162), modifiziert nach H. Bischoff et al. (Atherosclerosis 1997, 135).

Die extrahierte Lipidfraktion wird in 1 ml Isopropanol aufgenommen, in Szintillationsröhrchen überführt, mit 15 ml Ultima Gold^{®}-Szintillationsflüssigkeit (Packard) aufgefüllt und in einem Flüssigszintillationszähler (Beckmann Coulter LS 6500) gezählt.

Nach Berechnung der spezifischen ¹⁴C-Aktivität der Lipidfraktion (dpm/g Lebergewebe) wird die Syntheserate des radioaktiv markierten ¹⁴C-Squalens und der ¹⁴C-Folgemetabolite der mit Wirkstoff behandelten Tiere verglichen mit der Syntheserate des radioaktiv markierten ¹⁴C-Squalens und der ¹⁴C-Folgemetabolite der nur mit Formulierungsmittel behandelten Kontrolltiere. Eine Herabsetzung der Syntheserate um ≥ 30% verglichen mit der Syntheserate der Kontrolltiere (= 100%) wird als pharmakologisch wirksam angesehen, wenn die statistische Beurteilung mit Student's t-test einen p-Wert < 0.05 ergibt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 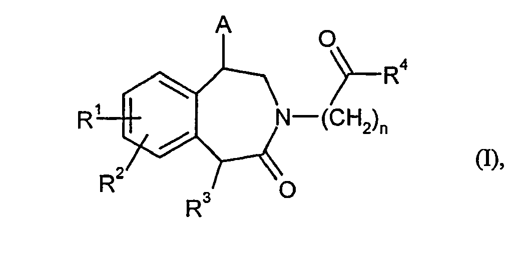 in welcher
A für (C₆-C₁₀)-Aryl oder 5- bis 10-gliedriges Heteroaryl, welche jeweils bis zu dreifach, gleich oder verschieden, durch Substituenten ausgewählt aus der Gruppe Halogen, Cyano, Nitro, Trifluormethyl, Trifluormethoxy, (C₁-C₆)-Alkyl, (C₂-C₆)-Alkinyl und (C₁-C₆)-Alkoxy substituiert sein können,
oder
für eine Gruppe der Formel 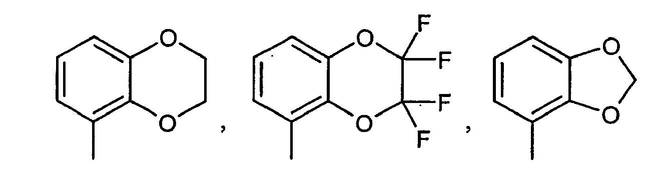 oder 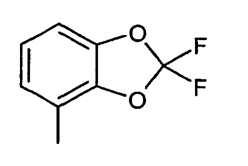 steht,
n für die Zahl 1, 2 oder 3 steht,
R¹ und R² gleich oder verschieden sind und unabhängig voneinander für Wasserstoff, Halogen, Cyano, Nitro, Trifluormethyl, Trifluormethoxy, (C₁-C₆)-Akl oder (C₁-C₆)-Alkoxy stehen,
R³ für (C₁-C₈)-Alkyl, (C₂-C₈)-Alkenyl oder (C₂-C₈)-Alkinyl, welche jeweils durch Phenyl, (C₃-C₈)-Cycloalkyl, Hydroxy, (C₁-C₆)-Alkoxy, (C₁-C₆)-Acyloxy oder Amino substituiert sein können, steht,
und
R⁴ für eine Gruppe der Formel -OR⁷ oder NR⁸R⁹ steht, worin
R⁷ Wasserstoff oder (C₁-C₆)-Alkyl bedeutet,
R⁸ und R⁹ gleich oder verschieden sind und unabhängig voneinander Wasserstoff, (C₁-C₆)-Alkyl oder (C₃-C₈)-Cycloalkyl, die durch Substituenten ausgewählt aus der Gruppe Carboxyl, (C₁C₆)-Alkoxycarbonyl, Aminocarbonyl, Mono- und Di-(C₁-C₆)-alkylaminocarbonyl substituiert sein können, bedeuten
oder
R⁸ und R⁹ gemeinsam mit dem Stickstoffatom, an das sie gebunden sind, einen 4-bis 8-gliedrigen Heterocyclus, der ein weiteres Ring-Heteroatom aus der Reihe N-R¹⁰, O, S, SO oder SO₂ enthalten und durch Substituenten ausgewählt aus der Gruppe Hydroxy, Oxo, Amino, (C₁-C₆)-Alkyl, Carboxyl, (C₁-C₆)-Alkoxycarbonyl, Aminocarbonyl, Mono- und Di-(C₁-C₆)-alkylaminocarbonyl substituiert sein kann, bilden, worin
(C₁-C₆)-Alkyl seinerseits durch Substituenten ausgewählt aus der Gruppe Hydroxy, Amino, Carboxyl, (C₁-C₆)-Alkoxycarbonyl, Aminocarbonyl, Mono- und Di-(C₁-C₆)-alkylaminocarbonyl substituiert sein kann
und
R¹⁰ Wasserstoff, (C₁-C₄)-Alkyl, (C₁-C₄)-Acyl oder (C₁-C₄)-Alkoxycarbonyl bedeutet,
sowie ihre Salze, Solvate und Solvate der Salze.

2. Verbindung der Formel (I) nach Anspruch 1, in welcher
A für Phenyl, Naphthyl oder Pyridyl, welche jeweils bis zu zweifach, gleich oder verschieden, durch Substituenten ausgewählt aus der Gruppe Fluor, Chlor, Brom, Cyano, Trifluormethyl, Trifluormethoxy, (C₁-C₄)-Alkyl, (C₂-C₄)-Alkinyl und (C₁-C₄)-Alkoxy substituiert sein können,
oder
für eine Gruppe der Formel 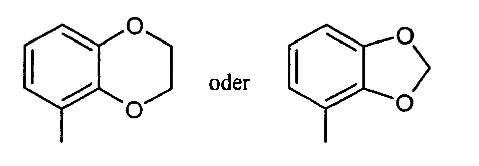 steht,
n für die Zahl 1, 2 oder 3 steht,
R¹ für Wasserstoff, Fluor, Chlor, Cyano, Trifluormethyl, Trifluormethoxy, (C₁-C₄)-Alkyl oder (C₁-C₄)-Alkoxy steht,
R² für Wasserstoff steht,
R³ für (C₁-C₆)-Alkyl oder (C₂-C₆)-Alkenyl, welche jeweils durch Phenyl, (C₃-C₆)-Cycloalkyl oder Hydroxy substituiert sein können, steht,
und
R⁴ für eine Gruppe der Formel -OR⁷ oder NR⁸R⁹ steht, worin
R⁷ Wasserstoff bedeutet,
R⁸ und R⁹ gleich oder verschieden sind und unabhängig voneinander Wasserstoff, (C₁-C₆)-Alkyl oder (C₃-C₆)-Cycloalkyl, die durch Substituenten ausgewählt aus der Gruppe Carboxyl, (C₁-C₄)-Alkoxycarbonyl, Aminocarbonyl, Mono- und Di-(C₁-C₄)-alkylaminocarbonyl substituiert sein können, bedeuten
oder
R⁸ und R⁹ gemeinsam mit dem Stickstoffatom, an das sie gebunden sind, einen 5-bis 7-gliedrigen Heterocyclus, der ein weiteres Ring-Heteroatom aus der Reihe N-R¹⁰ und O enthalten und durch Substituenten ausgewählt aus der Gruppe Hydroxy, Oxo, Amino, (C₁-C₄)-Alkyl, Carboxyl, (C₁-C₄)-Alkoxycarbonyl, Aminocarbonyl, Mono- und Di-(C₁-C₄)-alkylaminocarbonyl substituiert sein kann, bilden, worin
(C₁-C₄)-Alkyl seinerseits durch Substituenten ausgewählt aus der Gruppe Hydroxy, Amino, Carboxyl, (C₁-C₄)-Alkoxycarbonyl, Aminocarbonyl, Mono- und Di-(C₁-C₄)-alkylaminocarbonyl substituiert sein kann
und
R¹⁰ Wasserstoff, (C₁-C₄)-Alkyl, (C₁-C₄)-Acyl oder (C₁-C₄)-Alkoxycarbonyl bedeutet,
sowie ihre Salze, Solvate und Solvate der Salze.

3. Verbindung der Formel (I) nach Anspruch 1 oder 2, in welcher
A für Phenyl, welches ein- oder zweifach, gleich oder verschieden, durch Fluor, Chlor, Brom, Methyl, Ethyl, Ethinyl oder Methoxy substituiert sein kann, für Naphthyl oder für eine Gruppe der Formel 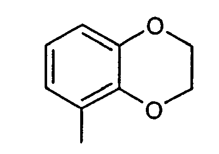 steht,
n für die Zahl 1 steht,
R¹ für Wasserstoff, Chlor, Methyl oder Trifluormethyl steht,
R² für Wasserstoff steht,
R³ für (C₁-C₆)-Alkyl, (C₂-C₆)-Alkenyl oder für Benzyl steht,
und
R⁴ für eine Gruppe der Formel -OR⁷ oder NR⁸R⁹ steht, worin
R⁷ Wasserstoff bedeutet,
R⁸ und R⁹ gleich oder verschieden sind und unabhängig voneinander Wasserstoff oder (C₁-C₆)- Alkyl, welches durch Carboxyl oder (C₁-C₄)-Alkoxycarbonyl substituiert sein kann, bedeuten
oder
R⁸ und R⁹ gemeinsam mit dem Stickstoffatom, an das sie gebunden sind, einen 5-bis 6-gliedrigen Heterocyclus, der ein weiteres Ring-Heteroatom aus der Reihe N-R¹⁰ und O enthalten und durch Substituenten ausgewählt aus der Gruppe Hydroxy, Oxo, Amino, (C₁-C₄)-Alkyl, Carboxyl, (C₁-C₄)-Alkoxycarbonyl, Aminocarbonyl, Mono- und Di-(C₁-C₄)-alkylaminocarbonyl substituiert sein kann, bilden, worin
(C₁-C₄)-Alkyl seinerseits durch Substituenten ausgewählt aus der Gruppe Hydroxy, Amino, Carboxyl, (C₁-C₄)-Alkoxycarbonyl, Aminocarbonyl, Mono- und Di-(C₁-C₄)-alkylaminocarbonyl substituiert sein kann
und
R¹⁰ Wasserstoff, (C₁-C₄)-Alkyl oder (C₁-C₄)-Acyl bedeutet,
sowie ihre Salze, Solvate und Solvate der Salze.

4. Verbindung der Formel (I-A) wie in Anspruch 1 definiert 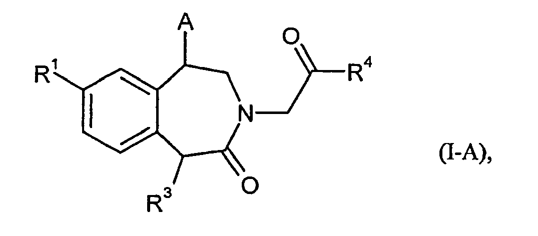 in welcher
A für Phenyl, welches ein- oder zweifach, gleich oder verschieden, durch Fluor, Chlor, Brom, Methyl, Ethinyl oder Methoxy substituiert ist, oder für eine Gruppe der Formel 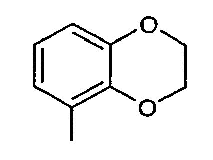 steht,
R¹ für Chlor, Methyl oder Trifluormethyl steht,
R³ für (C₁-C₆)-Alkyl oder (C₂-C₆)-Alkenyl steht,
und
R⁴ für eine Gruppe der Formel -OR⁷ oder -NR⁸R⁹ steht, worin
R⁷ Wasserstoff bedeutet,
R⁸ und R⁹ gleich oder verschieden sind und unabhängig voneinander Wasserstoff oder (C₁-C₆)-Alkyl, welches durch Carboxyl oder (C₁-C₄)-Alkoxycarbonyl substituiert sein kann, bedeuten
oder
R⁸ und R⁹ gemeinsam mit dem Stickstoffatom, an das sie gebunden sind, einen 5-bis 6-gliedrigen Heterocyclus, der ein weiteres Ring-Heteroatom aus der Reihe N-R¹⁰ und O enthalten und durch Substituenten ausgewählt aus der Gruppe Hydroxy, Oxo, Amino, (C₁-C₄)-Alkyl, Carboxyl, (C₁-C₄)-Alkoxycarbonyl, Aminocarbonyl, Mono- und Di-(C₁-C₄)-alkylaminocarbonyl substituiert sein kann, bilden, worin
(C₁-C₄)-Alkyl seinerseits durch Substituenten ausgewählt aus der Gruppe Hydroxy, Amino, Carboxyl, (C₁-C₄)-Alkoxycarbonyl, Aminocarbonyl, Mono- und Di-(C₁-C₄)-alkylaminocarbonyl substituiert sein kann
und
R¹⁰ Wasserstoff, (C₁-C₄)-Alkyl oder (C₁-C₄)-Acyl bedeutet,
sowie ihre Salze, Solvate und Solvate der Salze.

5. Verfahren zur Herstellung einer Verbindung der Formel (I) bzw. (I-A), wie in den Ansprüchen 1 bis 4 definiert, **dadurch gekennzeichnet, dass** man Verbindungen der Formel (II) 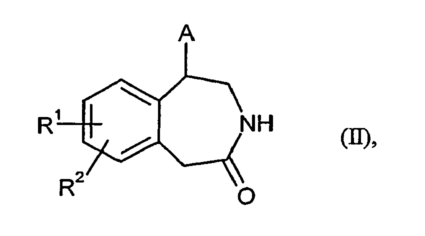 in welcher R¹, R² und A jeweils die in den Ansprüchen 1 bis 4 angegebenen Bedeutungen haben,
zunächst in einem inerten Lösungsmittel in Gegenwart einer Base mit einer Verbindung der Formel (III) 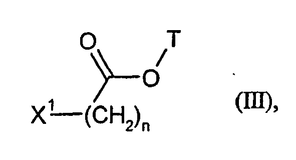 in welcher n die in den Ansprüchen 1 bis 4 angegebenen Bedeutungen hat,
T für (C₁-C₄)-Alkyl oder Benzyl
und
X¹ für eine geeignete Fluchtgruppe wie beispielsweise Halogen, Mesylat oder Tosylat steht,
zu Verbindungen der Formel (IV) 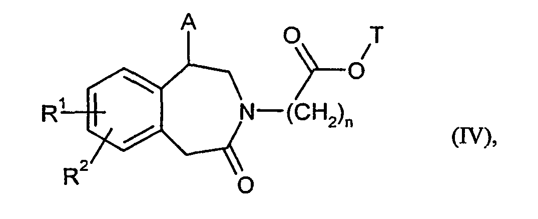 in welcher R¹, R², A, T und n jeweils die oben angegebenen Bedeutungen haben,
umsetzt, anschließend in einem inerten Lösungsmittel in Gegenwart einer geeigneten Base, vorzugsweise einer Phosphazen-Base, mit einer Verbindung der Formel (V)
R³-X² (V),
in welcher R³ die in den Ansprüchen 1 bis 4 angegebenen Bedeutungen hat und
x² für eine geeignete Fluchtgruppe wie beispielsweise Halogen, Mesylat oder Tosylat steht,
in Verbindungen der Formel (VI) 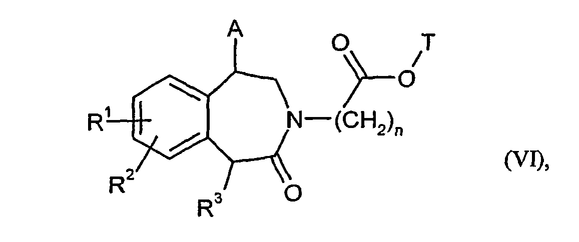 in welcher R¹, R², R³, A, T und n jeweils die oben angegebenen Bedeutungen haben,
überführt, diese durch basische oder saure Hydrolyse oder im Falle, dass T für Benzyl steht, auch hydrogenolytisch zu Carbonsäuren der Formel (VII) 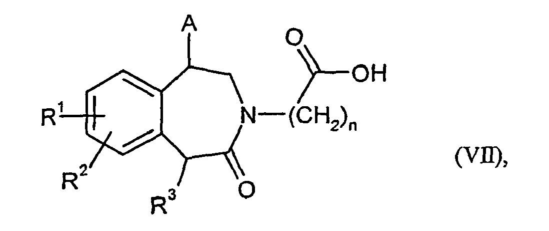 in welcher R¹, R², R³, A und n jeweils die oben angegebenen Bedeutungen haben,
umsetzt und dann nach literaturbekannten Methoden zur Veresterung bzw. Amidierung von Carbonsäuren in die Verbindungen der Formel (I) bzw. (I-A) überführt
und die Verbindungen der Formel (I) bzw. (I-A) gegebenenfalls mit den entsprechenden (i) Lösungsmitteln und/oder (ii) Basen oder Säuren zu ihren Solvaten, Salzen und/oder Solvaten der Salze umsetzt.

6. Verbindung, wie in einem der Ansprüche 1 bis 4 definiert, zur Behandlung und/oder Prophylaxe von Krankheiten.

7. Verwendung einer Verbindung, wie in einem der Ansprüche 1 bis 4 definiert, zur Herstellung eines Arzneimittels zur Behandlung und/oder Prävention von Dyslipidämien, Arteriosklerose, Restenose und Ischämien.

8. Arzneimittel enthaltend eine Verbindung, wie in einem der Ansprüche 1 bis 4 definiert, in Kombination mit einem weiteren Wirkstoff ausgewählt aus der Gruppe bestehend aus Cholesterin-senkende Statine, Cholesterin-Absorptionshemmer, HDL-erhöhende, Triglycerid-senkende und/oder Apolipoprotein B-senkende Substanzen, Oxidationshemmer und anti-entzündlich wirkende Verbindungen.

9. Arzneimittel enthaltend eine Verbindung, wie in einem der Ansprüche 1 bis 4 definiert, in Kombination mit einem inerten, nichttoxischen, pharmazeutisch geeigneten Hilfsstoff.

10. Arzneimittel nach Anspruch 8 oder 9 zur Behandlung und/oder Prävention von Dyslipidämien, Arteriosklerose, Restenose und Ischämien.

11. Verfahren zur Herstellung eines Arzneimittels zur Behandlung und/oder Prävention von Dyslipidämien, Arteriosklerose, Restenose und Ischämien in Menschen und Tieren durch Verabreichung einer wirksamen Menge mindestens einer Verbindung, wie in einem der Ansprüche 1 bis 4 definiert, oder eines Arzneimittels, wie in einem der Ansprüche 8 bis 10 definiert.

## Claims

1. Compound of the formula (I) 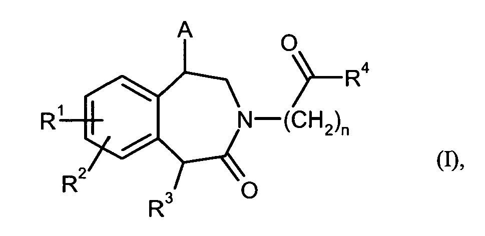 in which
A is (C₆-C₁₀)-aryl or 5- to 10-membered heteroaryl, each of which may be substituted up to three times, identically or differently, by substituents selected from the group of halogen, cyano, nitro, trifluoromethyl, trifluoromethoxy, (C₁-C₆)-alkyl, (C₂-C₆)-alkynyl and (C₁-C₆)-alkoxy,
or
is a group of the formula 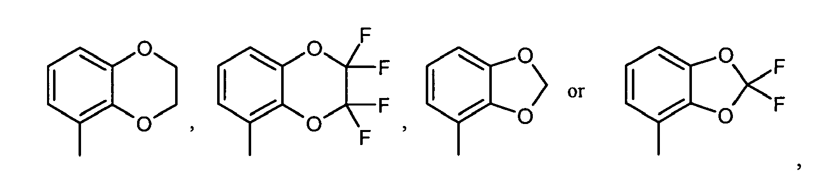
n is the number 1, 2 or 3,
R¹ and R² are identical or different and are independently of one another hydrogen, halogen, cyano, nitro, trifluoromethyl, trifluoromethoxy, (C₁-C₆)-alkyl or (C₁-C₆)-alkoxy,
R³ is (C₁-C₈)-alkyl, (C₂-C₈)-alkenyl or (C₂-C₈)-alkynyl, each of which may be substituted by phenyl, (C₃-C₈)-cycloalkyl, hydroxy, (C₁-C₆)-alkoxy, (C₁-C₆)-acyloxy or amino,
and
R⁴ is a group of the formula -OR⁷ or -NR⁸R⁹, in which
R⁷ is hydrogen or (C₁-C₆)-alkyl,
R⁸ and R⁹ are identical or different and are independently of one another hydrogen, (C₁-C₆)-alkyl or (C₃-C₈)-cycloalkyl, each of which may be substituted by substituents selected from the group of carboxyl, (C₁-C₆)-alkoxycarbonyl, aminocarbonyl, mono- and di-(C₁-C₆)-alkylaminocarbonyl,
or
R⁸ and R⁹ form together with the nitrogen atom to which they are bonded a 4- to 8-membered heterocycle which may comprise a further ring heteroatom from the series N-R¹⁰, O, S, SO or SO₂ and may be substituted by substituents selected from the group of hydroxy, oxo, amino, (C₁-C₆)-alkyl, carboxyl, (C₁-C₆)-alkoxycarbonyl, aminocarbonyl, mono- and dl-(C₁-C₆)-alkylaminocarbonyl, in which
(C₁-C₆)-alkyl in turn may be substituted by substituents selected from the group of hydroxy, amino, carboxyl, (C₁-C₆)-alkoxycarbonyl, aminocarbonyl, mono- and di-(C₁-C₆)-alkylaminocarbonyl,
and
R¹⁰ is hydrogen, (C₁-C₄)-alkyl, (C₁-C₄)-acyl or (C₁-C₄)-alkoxycarbonyl,
and the salts, solvates and solvates of the salts thereof.

2. Compound of the formula (I) according to Claim 1, in which
A is phenyl, naphthyl or pyridyl, each of which may be substituted up to twice, identically or differently, by substituents selected from the group of fluorine, chlorine, bromine, cyano, trifluoromethyl, trifluoromethoxy, (C₁-C₄)-alkyl, (C₂-C₄)-alkynyl and (C₁-C₄)-alkoxy,
or
is a group of the formula 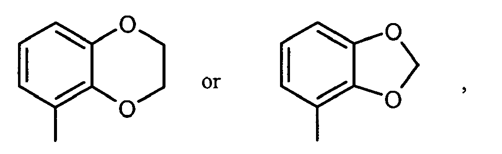
n is the number 1, 2 or 3,
R¹ is hydrogen, fluorine, chlorine, cyano, trifluoromethyl, trifluoromethoxy, (C₁-C₄)-alkyl or (C₁-C₄)-alkoxy,
R² is hydrogen,
R³ is (C₁-C₆)-alkyl or (C₂-C₆)-alkenyl, each of which may be substituted by phenyl, (C₃-C₆)-cycloalkyl or hydroxy,
and
R⁴ is a group of the formula -OR⁷ or -NR⁸R⁹ in which
R⁷ is hydrogen,
R⁸ and R⁹ are identical or different and are independently of one another hydrogen, (C₁-C₆)-alkyl or (C₃-C₆)-cycloalkyl, each of which may be substituted by substituents selected from the group of carboxyl, (C₁-C₄)-alkoxycarbonyl, aminocarbonyl, mono- and di-(C₁-C₄)-alkylaminocarbonyl,
or
R⁸ and R⁹ form together with the nitrogen atom to which they are bonded a 5- to 7-membered heterocycle which may comprise a further ring heteroatom from the series N-R¹⁰ and O and may be substituted by substituents selected from the group of hydroxy, oxo, amino, (C₁-C₄)-alkyl, carboxyl, (C₁-C₄)-alkoxycarbonyl, aminocarbonyl, mono- and di-(C₁-C₄)-alkylaminocarbonyl, in which
(C₁-C₄)-alkyl in turn may be substituted by substituents selected from the group of hydroxy, amino, carboxyl, (C₁-C₄)-alkoxycarbonyl, aminocarbonyl, mono- and di-(C₁-C₄)-alkylaminocarbonyl,
and
R¹⁰ is hydrogen, (C₁-C₄)-alkyl, (C₁-C₄)-acyl or (C₁-C₄)-alkoxycarbonyl,
and the salts, solvates and solvates of the salts thereof.

3. Compound of the formula (I) according to Claim 1 or 2, in which
A is phenyl which may be substituted once or twice, identically or differently, by fluorine, chlorine, bromine, methyl, ethyl, ethynyl or methoxy, is naphthyl or is a group of the formula 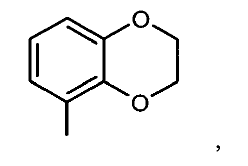
n is the number 1,
R¹ is hydrogen, chlorine, methyl or trifluoromethyl,
R² is hydrogen,
R³ is (C₁-C₆)-alkyl, (C₂-C₆)-alkenyl or is benzyl,
and
R⁴ is a group of the formula -OR⁷ or -NR⁸R⁹ in which
R⁷ is hydrogen,
R⁸ and R⁹ are identical or different and are independently of one another hydrogen or (C₁-C₆)-alkyl which may be substituted by carboxyl or (C₁-C₄)-alkoxycarbonyl,
or
R⁸ and R⁹ form together with the nitrogen atom to which they are bonded a 5- to 6-membered heterocycle which may comprise a further ring heteroatom from the series N-R¹⁰ and O and may be substituted by substituents selected from the group of hydroxy, oxo, amino, (C₁-C₄)-alkyl, carboxyl, (C₁-C₄)-alkoxycarbonyl, aminocarbonyl, mono- and di-(C₁-C₄)-alkylaminocarbonyl, in which
(C₁-C₄)-alkyl in turn may be substituted by substituents selected from the group of hydroxy, amino, carboxyl, (C₁-C₄)-alkoxycarbonyl, aminocarbonyl, mono- and di-(C₁-C₄)-alkylaminocarbonyl,
and
R¹⁰ is hydrogen, (C₁-C₄)-alkyl or (C₁-C₄)-acyl,
and the salts, solvates and solvates of the salts thereof.

4. Compound of the formula (I-A) as defined in Claim 1 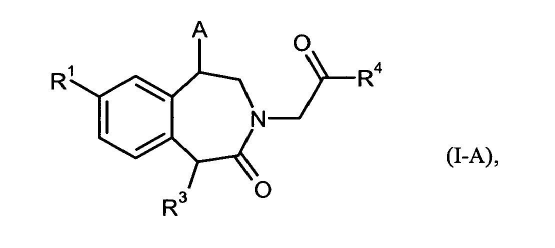 in which
A is phenyl which may be substituted once or twice, identically or differently, by fluorine, chlorine, bromine, methyl, ethynyl or methoxy, or is a group of the formula 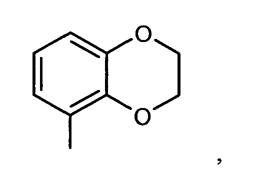
R¹ is chlorine, methyl or trifluoromethyl,
R³ is (C₁-C₆)-alkyl or (C₂-C₆)-alkenyl,
and
R⁴ is a group of the formula -OR⁷ or -NR⁸R⁹ in which
R⁷ is hydrogen,
R⁸ and R⁹ are identical or different and are independently of one another hydrogen or (C₁-C₆)-alkyl which may be substituted by carboxyl or (C₁-C₄)-alkoxycarbonyl,
or
R⁸ and R⁹ form together with the nitrogen atom to which they are bonded a 5- to 6-membered heterocycle which may comprise a further ring heteroatom from the series N-R¹⁰ and O and may be substituted by substituents selected from the group of hydroxy, oxo, amino, (C₁-C₄)-alkyl, carboxyl, (C₁-C₄)-alkoxycarbonyl, aminocarbonyl, mono- and di-(C₁-C₄)-alkylaminocarbonyl, in which
(C₁-C₄)-alkyl in turn may be substituted by substituents selected from the group of hydroxy, amino, carboxyl, (C₁-C₄)-alkoxycarbonyl, aminocarbonyl, mono- and di-(C₁-C₄)-alkylaminocarbonyl,
and
R¹⁰ is hydrogen, (C₁-C₄)-alkyl or (C₁-C₄)-acyl,
and the salts, solvates and solvates of the salts thereof.

5. Process for preparing a compound of the formula (I) or (I-A) as defined in Claims 1 to 4, **characterized in that** compounds of the formula (II) 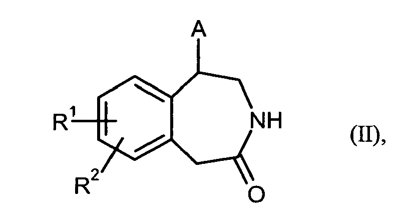 in which R¹, R² and A each have the meanings indicated in Claims 1 to 4,
are firstly reacted in an inert solvent in the presence of a base with a compound of the formula (III) 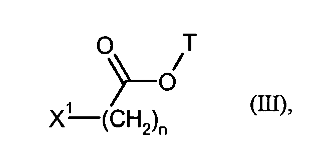 in which n has the meanings indicated in Claims 1 to 4,
T is (C₁-C₄)-alkyl or benzyl
and
X¹ is a suitable leaving group such as, for example, halogen, mesylate or tosylate,
to give compounds of the formula (IV) 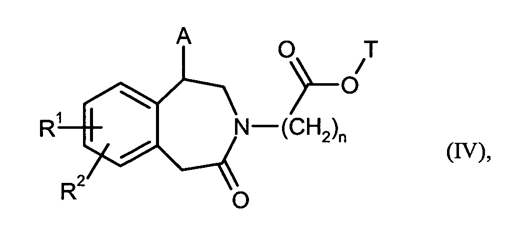 in which R¹, R², A, T and n each have the abovementioned meanings,
subsequently converted in an inert solvent in the presence of a suitable base, preferably a phosphazene base, with a compound of the formula (V)
R³-X² (V),
in which R³ has the meanings indicated in Claims 1 to 4, and
x² is a suitable leaving group such as, for example, halogen, mesylate or tosylate,
into compounds of the formula (VI) 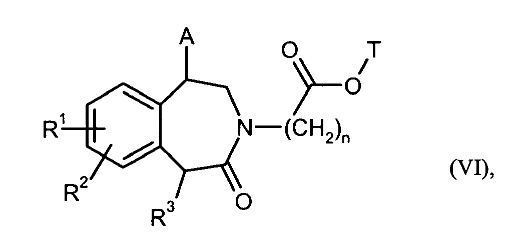 in which R¹, R², R³, A, T and n each have the abovementioned meanings,
the latter are converted by basic or acidic hydrolysis, or in the case where T is benzyl also by hydrogenolysis, into carboxylic acids of the formula (VII) 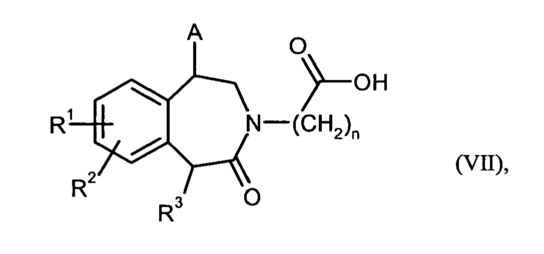 in which R¹, R², R³, A and n each have the abovementioned meanings,
and then converted by methods known from the literature for the esterification or amidation of carboxylic acids into the compounds of the formula (I) or (I-A),
and the compounds of the formula (I) or (I-A) are reacted where appropriate with the appropriate (i) solvents and/or (ii) bases or acids to give the solvates, salts and/or solvates of the salts thereof.

6. Compound as defined in any of Claims 1 to 4 for the treatment and/or prophylaxis of diseases.

7. Use of a compound as defined in any of Claims 1 to 4 for producing a medicament for the treatment and/or prevention of dyslipidaemias, arteriosclerosis, restenosis and ischaemias.

8. Medicament comprising a compound as defined in any of Claims 1 to 4 in combination with a further active ingredient selected from the group consisting of cholesterol-lowering statins, cholesterol absorption inhibitor, HDL-elevating or triglyceride-lowering and/or apolipoprotein B-lowering substances, oxidation inhibitor and compounds having antiinflammatory activity.

9. Medicament comprising a compound as defined in any of Claims 1 to 4 in combination with an inert, non-toxic, pharmaceutically suitable excipient.

10. Medicament according to Claim 8 or 9 for the treatment and/or prevention of dyslipidaemias, arteriosclerosis, restenosis and ischaemias.

11. Process for producing a medicament for the treatment and/or prevention of dyslipidaemias, arteriosclerosis, restenosis and ischaemias in humans and animals by administering an effective amount of at least one compound as defined in any of Claims 1 to 4, or of a medicament as defined in any of Claims 8 to 10.

## Revendications

1. Composé de formule (I) 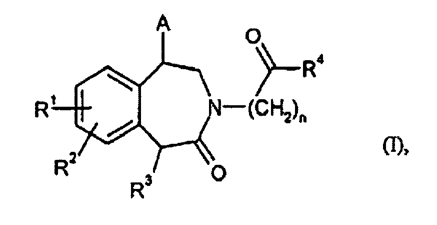 dans laquelle
A représente un reste aryle en C₆ à C₁₀ ou un reste hétéroaryle de 5 à 10 chaînons, ces restes pouvant être substitués dans chaque cas jusqu'à trois fois identiques ou différentes par des substituants choisis dans le groupe consistant en halogéno, cyano, nitro, trifluorométhyle, trifluorométhoxy, alkyle en C₁ à C₆, alcynyle en C₂ à C₆ et alkoxy en C₁ à C₆,
ou bien
un groupe de formule 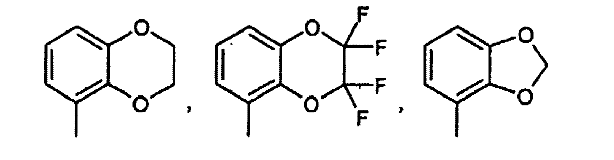 ou 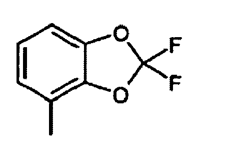
n représente le nombre 1, 2 ou 3,
R¹ et R² sont identiques ou différents et représentent, indépendamment l'un de l'autre, l'hydrogène, un halogène, un groupe cyano, nitro, un reste trifluorométhyle, trifluorométhoxy, alkyle en C₁ à C₆ ou alkoxy en C₁ à C₆,
R³ représente un reste alkyle en C₁ à C₈, alcényle en C₂ à C₈ ou alcynyle en C₂ à C₈, chacun pouvant être substitué par un radical phényle, cycloalkyle en C₃ à C₈, hydroxy, alkoxy en C₁ à C₆, alkoxy en C₁ à C₆ ou amino,
et
R⁴ représente un groupe de formule -OR⁷ ou -NR⁸R⁹, où R⁷ représente l'hydrogène ou un reste alkyle en C₁ à C₆,
R⁸ et R⁹ sont identiques ou différents et représentent, indépendamment l'un de l'autre, l'hydrogène, un reste alkyle en C₁ à C₆ ou cycloalkyle en C₃ à C₈, qui peuvent être substitués par des substituants choisis dans le groupe consistant en carboxyle, (alkoxy en C₁ à C₆)carbonyle, aminocarbonyle, mono- ou di-(alkyle en C₁ àC₆)aminocarbonyle,
ou bien
R⁸ et R⁹ forment, conjointement avec l'atome d'azote auquel ils sont liés, un hétérocycle de 4 à 8 chaînons, dont le noyau peut contenir un autre hétéroatome de la série N-R¹⁰, O, S, SO ou SO₂ et qui peut être substitué par des substituants choisis dans le groupe consistant en hydroxy, oxo, amino, alkyle en C₁ à C₆, carboxyle, (alkoxy en C₁ à C₆) carbonyle, aminocarbonyle, mono- et di-(alkyle en C₁ à C₆)aminocarbonyle, où
le substituant alkyle en C₁ à C₆ peut lui-même être substitué par des substituants choisis dans le groupe consistant en hydroxy, amino, carboxyle, (alkoxy en C₁ à C₆)carbonyle, aminocarbonyle, mono- et di-(alkyle en C₁ à C₆)aminocarbonyle
et
R¹⁰ représente l'hydrogène, un reste alkyle en C₁ à C₄, acyle en C₁ à C₄ ou (alkoxy en C₁ à C₄) carbonyle,
ainsi que ses sels, ses produits de solvation et les produits de solvatation des sels.

2. Composé suivant la revendication 1, de formule (I) dans laquelle
A représente un reste phényle, naphtyle ou pyridyle, chacun pouvant être substitué une ou plusieurs fois identiques ou différentes par des substituants choisis dans le groupe consistant en fluor, chlore, brome, cyano, trifluorométhyle, trifluorométhoxy, alkyle en C₁ à C₄, alcynyle en C₂ à C₄ ou alkoxy en C₁ à C₄,
ou bien
un groupe de formule 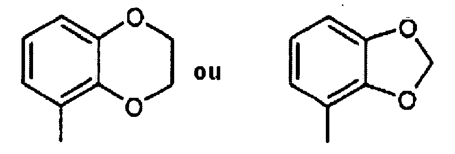
n représente le nombre 1, 2 ou 3,
R¹ représente l'hydrogène, le fluor, le chlore, un groupe cyano, un reste trifluorométhyle, trifluoro-méthoxy, alkyle en C₁ à C₄ ou alkoxy en C₁ à C₄,
R² représente l'hydrogène,
R³ représente un reste alkyle en C₁ à C₆ ou alcényle en C₂ à C₆, chacun pouvant être substitué par un radical phényle, cycloalkyle en C₃ à C₆ ou hydroxy,
et
R⁴ représente un groupe de formule -OR⁷ ou -NR⁸R⁹, où R⁷ représente l'hydrogène,
R⁸ et R⁹ sont identiques ou différents et représentent, indépendamment l'un de l'autre, l'hydrogène, un reste alkyle en C₁ à C₆ ou un reste cycloalkyle en C₃ à C₆, qui peuvent être substitués par des substituants choisis dans le groupe consistant en carboxyle, (alkoxy en C₁ à C₄)carbonyle, aminocarbonyle, mono- ou di-(alkyle en C₁ à C₄)amino-carbonyle,
ou bien
R⁸ et R⁹ forment, conjointement avec l'atome d'azote auquel ils sont liés, un hétérocycle de 5 à 7 chaînons dont le noyau peut contenir un autre hétéroatome de la série N-R¹⁰ et O et peut être substitué par des substituants choisis dans la groupe consistant en hydroxy, oxo, amino, alkyle en C₁ à C₄, carboxyle, (alkoxy en C₁ à C₄)carbonyle, aminocarbonyle, mono- et di-(alkyle en C₁ à C₄)aminocarbonyle, où
le reste alkyle en C₁ à C₄ peut lui-même être substitué par des substituants choisis dans le groupe consistant en hydroxy, amino, carboxyle, (alkoxy en C₁ à C₄)carbonyle, aminocarbonyle, mono- et di-(alkyle en C₁ à C₄)aminocarbonyle
et
R¹⁰ représente l'hydrogène, un reste alkyle en C₁ à C₄, acyle en C₁ à C₄ ou (alkoxy en C₁ à C₄)carbonyle,
ainsi que ses sels, ses produits de solvatation et les produits de solvatation des sels.

3. Composé suivant la revendication 1 ou 2, de formule (I) dans laquelle
A représente un reste phényle qui peut être substitué une ou plusieurs fois identiques ou différentes par du fluor, du chlore, du brome, un reste méthyle, éthyle, éthynyle ou méthoxy, un reste naphtyle ou un groupe de formule 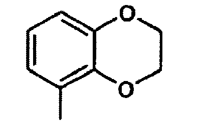
n représente le nombre 1,
R¹ représente l'hydrogène, le chlore, un reste méthyle ou trifluorométhyle,
R² représente l'hydrogène
R³ est un reste alkyle en C₁ à C₆, alcényle en C₂ à C₆ ou un reste benzyle,
et
R⁴ représente un groupe de formule -OR⁷ ou -NR⁸R^{9'}, où
R⁷ représente l'hydrogène,
R⁸ et R⁹ sont identiques ou différents et représentent, indépendamment l'un de l'autre, l'hydrogène ou un reste alkyle en C₁ à C₆, qui peut être substitué par un radical carboxyle ou (alkoxy en C₁ à C₄) carbonyle,
ou bien
R⁸ et R⁹ forment, conjointement avec l'atome d'azote auquel ils sont liés, un hétérocycle pentagonal ou hexagonal dont le noyau peut contenir un autre hétéroatome de la série N-R¹⁰ et O et peut être substitué par des substituants choisis dans le groupe consistant en hydroxy, oxo, amino, alkyle en C₁ à C₄, carboxyle, (alkoxy en C₁ à C₄)carbonyle, aminocarbonyle, mono- et di-(alkyle en C₁ à C₄)aminocarbonyle, où
le substituant alkyle en C₁ à C₄ peut lui-même être substitué par des substituants choisis dans le groupe consistant en hydroxy, amino, carboxyle, (alkoxy en C₁ à C₄)carbonyle, aminocarbonyle, mono- et di-(alkyle en C₁ à C₄)aminocarbonyle
et
R¹⁰ représente l'hydrogène, un reste alkyle en C₁ à C₄ ou acyle en C₁ à C₄,
ainsi que ses sels, ses produits de solvatation et les produits de solvatation des sels.

4. Composé de formule (I-A) telle que définie dans la revendication 1 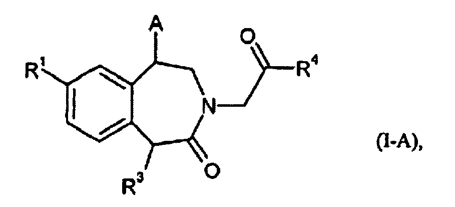 formule dans laquelle
A représente un reste phényle qui est substitué une ou deux fois identiques ou différentes par du fluor, du chlore, du brome, un radical méthyle, éthynyle ou méthoxy, ou représente un groupe de formule 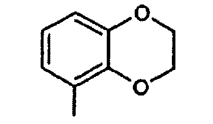
R¹ représente le chlore, un reste méthyle ou trifluorométhyle,
R³ est un reste alkyle en C₁ à C₆ ou alcényle en C₂ à C₆,
et
R⁴ représente un groupe de formule -OR⁷ ou -NR⁸R⁹, où R⁷ représente l'hydrogène,
R⁸ et R⁹ sont identiques ou différents et représentent, indépendamment l'un de l'autre, l'hydrogène ou un reste alkyle en C₁ à C₆, qui peut être substitué par un radical carboxyle ou (alkoxy en C₁ à C₄)carbonyle,
ou bien
R⁸ et R⁹ forment, conjointement avec l'atome d'azote auquel ils sont liés, un hétérocycle pentagonal ou hexagonal dont le noyau peut contenir un autre hétéroatome de la série N-R¹⁰ et O et peut être substitué par des substituants choisis dans le groupe consistant en hydroxy, oxo, amino, alkyle en C₁ à C₄, carboxyle, (alkoxy en C₁ à C₄)carbonyle, aminocarbonyle, mono- et di-(alkyle en C₁ à C₄)aminocarbonyle, où
le substituant alkyle en C₁ à C₄ peut lui-même être substitué par des substituants choisis dans le groupe consistant en hydroxy, amino, carboxyle, (alkoxy en C₁ à C₄)carbonyle, aminocarbonyle, mono- ou di-(alkyle en C₁ à C₄) aminocarbonyle
et
R¹⁰ représente l'hydrogène, un reste alkyle en C₁ à C₄ ou acyle en C₁ à C₄,
ainsi que ses sels, ses produits de solvatation et les produits de solvatation des sels.

5. Procédé de production d'un composé de formule (I) ou (I-A) comme défini dans les revendications 1 à 4, **caractérisé en ce qu'**on fait d'abord réagir des composés de formule (II) 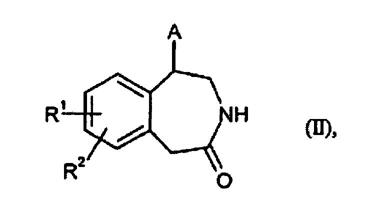 dans laquelle R¹, R² et A ont chacun les définitions indiquées dans les revendications 1 à 4,
dans un solvant inerte, en présence d'une base, avec un composé de formule (III) 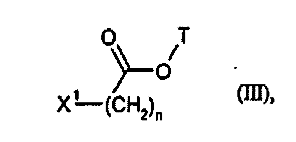 dans laquelle n a les définitions indiquées dans les revendications 1 à 4,
T est un reste alkyle en C₁ à C₄ ou benzyle
et
X¹ représente un groupe partant convenable tel que, par exemple, halogène, mésylate ou tosylate,
pour former des composés de formule (IV) 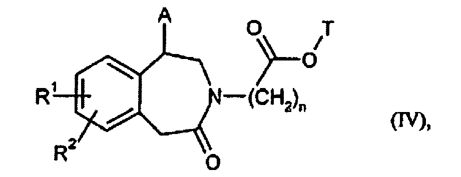 dans laquelle R¹, R², A, T et n ont chacun les définitions indiquées ci-dessus,
qu'on transforme ensuite, dans un solvant inerte, en présence d'une base convenable, avantageusement une base de type phosphazène, avec un composé de formule (V)
R³-X² (V)
dans laquelle R³ a les définitions indiquées dans les revendications 1 à 4 et X² représente un groupe partant convenable tel que, par exemple, halogène, mésylate ou tosylate,
en composés de formule (VI) 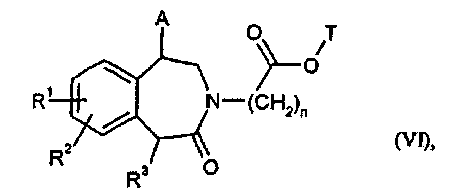 dans laquelle R¹, R², R³, A, T et n ont chacun les définitions indiquées ci-dessus,
on fait transformer ces composés par hydrolyse basique ou acide ou bien, au cas où T est un reste benzyle, par voie hydrogénolytique, en acides carboxyliques de formule (VII) 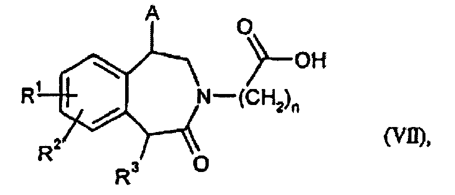 dans laquelle R¹, R², R³, A et n ont chacun les définitions indiquées ci-dessus,
que l'on transforme ensuite selon des techniques d'estérification ou d'amidation d'acides carboxyliques connues d'après la littérature en composés de formule (I) ou (I-A),
et on transforme éventuellement les composés de formule (I) ou (I-A) avec (i) les solvants correspondants et/ou (ii) les bases ou les acides correspondants en leurs produits de solvatation, leurs sels et/ou les produits de solvatation des sels.

6. Composé tel que défini dans l'une des revendications 1 à 4, destiné au traitement et/ou à la prophylaxie de maladies.

7. Utilisation d'un composé tel que défini dans l'une des revendications 1 à 4 pour la préparation d'un médicament destiné au traitement et/ou à la prévention de dyslipidémies, de l'artériosclérose, d'une resténose et d'ischémies.

8. Médicament contenant un composé tel que défini dans l'une des revendications 1 à 4, en combinaison avec une autre substance active choisie dans le groupe constitué des statines abaissant le taux de cholestérol, d'inhibiteurs d'absorption du cholestérol, de substances élevant le taux de lipoprotéines haute densité, abaissant le taux de triglycérides et/ou abaissant le taux d'apolipoprotéines B, d'inhibiteurs d'oxydation et de composés à action anti-inflammatoire.

9. Médicament contenant un composé tel que défini dans l'une des revendications 1 à 4, en combinaison avec une substance auxiliaire inerte non toxique pharmaceutiquement acceptable.

10. Médicament suivant la revendication 8 ou 9, destiné au traitement et/ou à la prévention de dyslipidémies, de l'artériosclérose, d'une resténose et d'ischémies.

11. Procédé de préparation d'un médicament destiné au traitement et/ou à la prévention de dyslipidémies, de l'artériosclérose, d'une resténose et d'ischémies chez l'homme et les animaux, par administration d'une quantité efficace d'au moins un composé tel que défini dans l'une des revendications 1 à 4, ou d'un médicament tel que défini dans l'une des revendications 8 à 10.
